# EUROPÄISCHE PATENTSCHRIFT

(11) **EP 0 233 267 B1**
(45) Veröffentlichungstag und Bekanntmachung des Hinweises auf die Patenterteilung: **03.08.1994**
(21) Anmeldenummer: 86905289.4
(22) Anmeldetag: 15.09.1986
(51) Int. Cl.: C09K 19/30, C09K 19/34, C09K 19/32, C07C 255/46, C07D 239/26, C07D 213/55

(54) **SMEKTISCHE FLÜSSIGKRISTALLINE PHASEN**
SMECTIC CRYSTAL PHASES
PHASES SMECTIQUES A CRISTAUX LIQUIDES

(30) Priorität: 18.09.1985 DE 3533333; 14.03.1986 DE 3608500
(43) Veröffentlichungstag der Anmeldung: 26.08.1987
(73) Patentinhaber: MERCK PATENT GmbH, D-64271 Darmstadt (DE)
(72) Erfinder: HOPF, Reinhard, D-6432 Heringen (DE); SCHEUBLE, Bernhard, Kanagawa 231 (JP); WÄCHTLER, Andreas, D-6103 Griesheim (DE); HITTICH, Reinhard, D-6101 Modautal 1 (DE); EIDENSCHINK, Rudolf, D-6109 Mühltal 1 (DE); GEELHAAR, Thomas, D-6500 Mainz (DE); KRAUSE, Joachim, D-6110 Dieburg (DE); REIFFENRATH, Volker, D-6100 Darmstadt (DE)
(86) Internationale Anmeldenummer: EP8600529
(87) Internationale Veröffentlichungsnummer: WO8701717

(56) Entgegenhaltungen:
- EP-A- 0 107 759
- EP-A- 0 149 208
- EP-A- 0 151 446
- EP-A- 0 182 054
- WO-A-86/06401
- WO-A-86/07055
- WO-A-86/07085
- DE-A- 3 437 935
- US-A- 4 664 840

## Beschreibung

Die Erfindung betrifft die Verwendung von Verbindungen der Formel I
R¹⁻Q¹⁻A-(Q²)q-R²

worin
R¹ und R² jeweils unabhängig voneinander eine Alkylgruppe oder eine Polyfluoralkylgruppe mit jeweils 1 bis 15 C-Atomen, worin auch eine oder mehrere CH₂-Gruppen bzw. CF₂-Gruppen durch eine Gruppierung ausgewählt aus der Gruppe -O-, -S-, -CO-, -CH-Halogen-, -O-CO-, -O-COO-, -CO-O- und -CH = CH- oder auch durch eine Kombination von zwei geeigneten Gruppierungen ersetzt sein können, wobei zwei Heteroatome nicht direkt miteinander verknüpft sind, einer der Reste R¹ und R² auch Halogen, 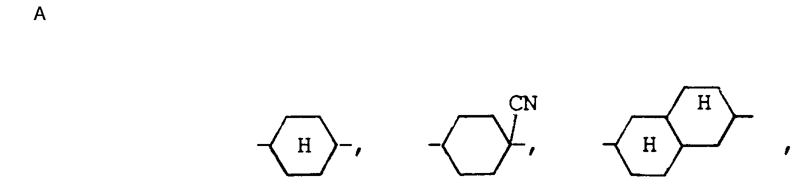 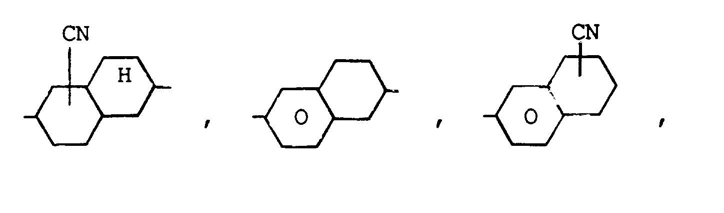 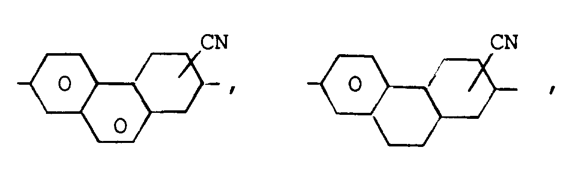 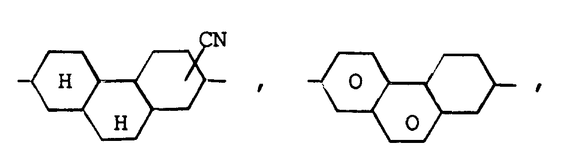 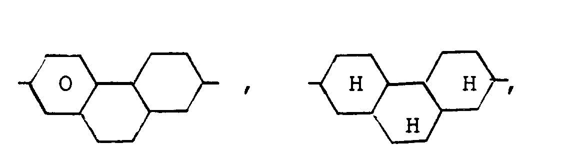 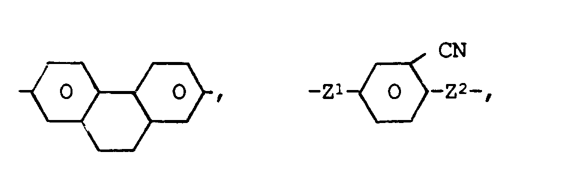 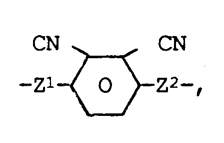 oder eine dieser Gruppen,
   worin eine oder mehrere nicht benachbarte CH₂-Gruppen durch O und/oder S oder aliphatische und/oder aromatische CH-Gruppen durch N ersetzt sind,
q 0 oder 1,
Q¹ und Q² jeweils unabhängig voneinander -(A ° -Z ° )p , wobei
A unsubstituiertes oder ein- oder mehrfach durch Halogenatome, CH₃- und/oder Nitril-Gruppen substituiertes 1,4-Cyclohexylen, worin auch eine oder zwei nicht benachbarte CH₂-Gruppen durch -O- und/oder -S- und/oder eine 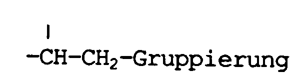 durch 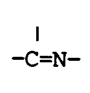 ersetzt sein können (Cy), oder unsubstituiertes oder ein- oder mehrfach durch Halogenatome, CH₃- und/oder Nitril-Gruppen substituiertes 1,4-Phenylen, worin auch eine oder mehrere CH-Gruppen durch N ersetzt sein können (Ph), bedeutet, einer der Reste A auch 2,6-Naphthylen (Na) oder Tetrahydro-2,6-naphthylen (4H-Na), gegebenenfalls durch Halogen oder CN substituiert,
Z°, Z¹ und Z² jeweils unabhängig voneinander -CO-O-, -O-CO-, -CH₂0-, -OCH₂-, -CH₂CH₂-, -CHCN-CH₂-, -CH₂-CHCN- oder eine Einfachbindung, und
p 1, 2 oder 3, oder im Falle A = Tetra- oder Octahydrophenanthren auch 0 bedeutet, wobei: im Falle A = 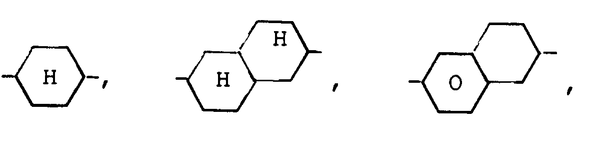 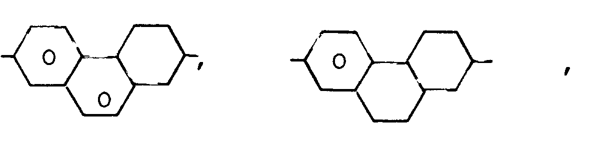 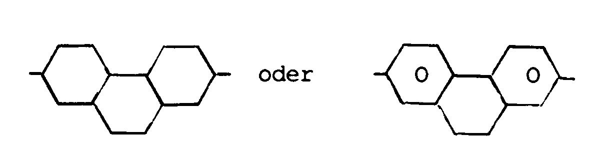 mindestens eine Gruppe Z ° -CHCNCH₂- oder -CH₂CHCN-bedeutet,
   als Komponenten chiraler getilteter smektischer Phasen mit negativen Werten der dielektrischen Anisotropie, wobei
   r-1-Cyan-1-(trans-4-pentylcyclohexyl)-trans-4-(trans-4-heptylcyclohexyl)-cyclohexan
   1-Cyan-1-(trans-4-pentylcyclohexyl)-2-(trans-4-propylcyclohexyl)-ethan
   1-Cyan-1-(trans-4-pentylcyclohexyl)-2-[trans-4-(trans-4-pentylcyclohexyl)-cyclohexyl]-ethan
   r-1-Cyan-cis-4-(trans-4-butylcyclohexyl)-1-butylcyclohexan

   sowie die Verbindungen der Formeln 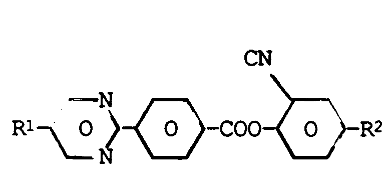 und 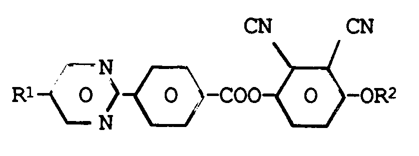 und worin
R¹ und R² unverzweigtes C₃₋₁₀-Alkyl bedeuten,

ausgenommen sind.

H.R. Brand et al., J. Physique 44, (lett.), L-771 (1983)). Solche Phasen können als Dielektrika für schnell schaltende Displays verwendet werden, die auf dem von Clark und Lagerwall beschriebenen Prinzip der SSFLC-Technologie (N.A. Clark und S.T. Lagerwall, Appl. Phys. Lett. 36, 899 (1980); USP 4,367,924) auf der Basis der ferroelektrischen Eigenschaften der chiralen getilteten Phase beruhen. In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtungen der Moleküle wird durch einen sehr geringen Abstand der Platten (ca. 1-2 um) unterdrückt. Dadurch werden die Längsachsen der Moleküle gezwungen, sich in einer Ebene parallel zu den Platten der Zelle anzuordnen, wodurch zwei ausgezeichnete Tiltorientierungen entstehen. Durch Anlegen eines geeigneten elektrischen Wechselfeldes kann in der eine spontane Polarisation aufweisenden flüssigkristalline Phase zwischen diesen beiden Zuständen hin- und hergeschaltet werden. Dieser Schaltvorgang ist wesentlich schneller als bei herkömmlichen verdrillten Zellen (TN-LCD's), die auf nematischen Flüssigkristallen basieren.

Ein großer Nachteil für viele Anwendungen der derzeit verfügbaren Materialien mit chiralen getilteten smektischen Phasen (wie z.B. Sc^{*}) ist, daß die dielektrische Anisotropie Werte größer Null oder, falls negativ, nur wenig von Null verschiedene Werte aufweist. Negative Werte der dielektrischen Anisotropie sind erforderlich, falls die erforderliche planare Orientierung durch Überlagerung des Ansteuerfeldes mit einem AC-Haltefeld mit kleiner Amplitude bewirkt wird (J.M. Geary, SID-Tagung, Orlando/Florida, April/Mai 1985, Vortrag 8.3). Der Einsatz von Materialien mit stark negativer dielektrischer Anisotropie führt meist zu einer starken Verminderung der Spontanpolarisation und/oder zu ungünstigen Werten für Pitch und/oder Tilt. Darüberhinaus wird meist der Temperaturbereich der ferroelektrischen Phasen in ungünstiger Weise eingeengt.

Es wurde nun gefunden, daß die Verwendung von Verbindungen der Formel I als Komponenten chiral getilteter smektischer Mischungen die erwähnten Nachteile wesentlich vermindern kann. Die Verbindungen der Formel 1 sind somit als Komponenten chiral getilteter smektischer flüssigkristalliner Phasen vorzüglich geeignet. Insbesondere sind mit ihrer Hilfe chemisch besonders stabile chiral getiltete smektische flüssigkristalline Phasen mit günstigen ferroelektrischen Phasenbereichen, insbesondere mit breiten Sc^{*} Phasenbereichen, negativer dielektrischer Anisotropie, günstiger Pitchhöhe und für derartige Phasen hohen Werten für die spontane Polarisation herstellbar. P ist die spontane Polarisation in nC/cm^{2.}

In der EP-A-0 107 759 werden flüssigkristalline Verbindungen beschrieben, die eine Gruppe der Formel 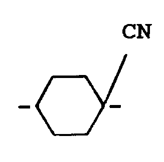 aufweisen.

Dieser Offenlegungsschrift kann der Fachmann jedoch keinen Hinweis entnehmen, daß diese Verbindungen auch als Komponenten smektischer flüssigkristalliner Phasen geeignet sind. Dagegen können diese Verbindungen nach der Lehre dieses Dokuments als Komponenten nematischer flüssigkristalliner Phasen eingesetzt werden.

In der EP-A-0 149 208 werden flüssigkristalline Verbindungen beschrieben, die eine endständige Cyanogruppe aufweisen. Diese Verbindungen führen zu flüssigkristallinen Phasen mit positiven Werten der dielektrischen Anisotropie und sind somit ungeeignet, die Anforderung der vorliegenden Erfindung nach negativen Werten der dielektrischen Anisotropie zu erfüllen.

In der EP-A-0 151 446 werden nematische flüssigkristalline Phasen beschrieben, die weder negative Werte der dielektrischen Anisotropie aufweisen noch geeignet sind für Displays, die auf dem Prinzip der SSFLC-Technologie beruhen.

In den Offenlegungsschriften WO 86/06401, WO 86/07055 und WO 86/07085 sind bereits folgende Verbindungen als Komponenten smektischer und chiraler getilteter smektischer Phasen beschrieben:
r-1-Cyan-1-(trans-4-pentylcyclohexyl)-trans-4-(trans-4-heptylcyclohexyl)-cyclohexan
1-Cyan-1-(trans-4-pentylcyclohexyl)-2-(trans-4-propylcyclohexyl)-ethan
1-Cyan-1-(trans-4-pentylcyclohexyl)-2-[trans-4-(trans-4-pentylcyclohexyl)-cyclohexyl]-ethan
r-1-Cyan-cis-4-(trans-4-butylcyclohexyl)-1-butylcyclohexan

sowie die Verbindungen der Formeln 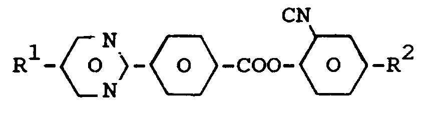 und 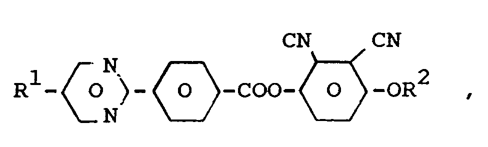 worin
R¹ und R² unverzweigtes C₃₋₁₀-Alkyl bedeuten.

Diese Dokumente fallen jedoch unter Art. 54 (3) EPÜ, so daß die Verbindungen per 'disclaimer' von der Formel I ausgenommen sind.

Die Verbindungen der Formel I besitzen einen breiten Anwendungsbereich. In Abhängigkeit von der Auswahl der Substituenten können diese Verbindungen als Basismaterialien dienen, aus denen flüssigkristalline smektische Phasen zum überwiegenden Teil zusammengesetzt sind; es können aber auch Verbindungen der Formel I flüssigkristalline Basismaterialien aus anderen Verbindungsklassen zugesetzt werden, um beispielsweise die dielektrische und/oder optische Anisotropie und/oder die Viskosität und/oder die spontane Polarisation und/oder den Phasenbereich und/oder den Tiltwinkel und/oder den Pitch eines solchen Dielektrikums zu variieren. Es können jedoch auch optisch aktive Verbindungen der Formel I als Dotierstoffe für Basis-Mischungen verwendet werden, wobei die Basismischungen aus Materialien aus anderen Verbindungsklassen oder ganz oder teilweise aus achiralen (Racemat oder Komponenten der Formel 1 ohne Asymmetriezentrum) oder weiteren chiralen Verbindungen der Formel I bestehen können.

Gegenstand der Erfindung ist somit die Verwendung der Verbindungen der Formel I als Komponenten (chiraler getilteter) smektischer flüssigkristalliner Phasen. Gegenstand der Erfindung sind ferner smektische flüssigkristalline Phasen, insbesondere chirale getiltete smektische Phasen, mit einem Gehalt an mindestens einer Verbindung der Formel I sowie Flüssigkristallanzeigeelemente, insbesondere elektrooptische Anzeigeelemente, die derartige Phasen enthalten.

Die Verbindungen der Formel I können geradkettige oder verzweigte Flügelgruppen R¹ und/oder R² haben. Verbindungen mit verzweigten Flügelgruppen können in Form des Racemates oder als optisch aktive Verbindungen eingesetzt werden. Achirale Basismischungen aus Verbindungen der Formel I und ggf. weiteren achiralen Komponenten können mit chiralen Verbindungen der Formel 1 oder auch mit anderen chiralen Verbindungen dotiert werden, um chirale getiltete smektische Phasen zu erhalten.

Die Verbindungen der Formel I umfassen insbesondere Verbindungen der Teilformeln la bis In: 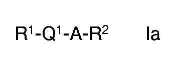 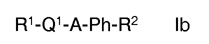 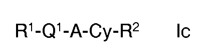 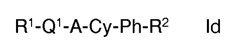 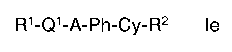 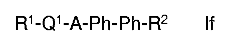 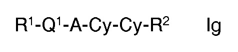 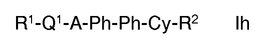 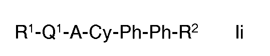 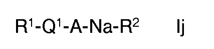 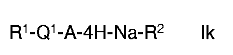 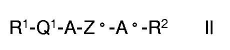 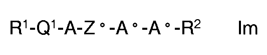 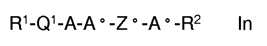 Darunter sind diejenigen der Teilformel la, Ib, Ic und II besonders bevorzugt.

Die bevorzugten Verbindungen der Teilformel la umfassen solche der Teilformel laa bis lan 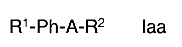 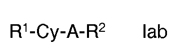 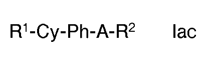 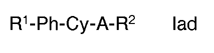 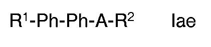 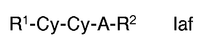 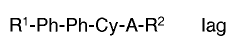 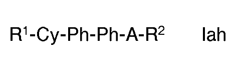 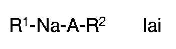 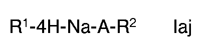 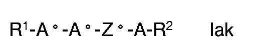 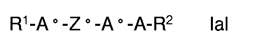 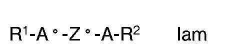 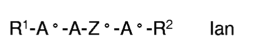 A und Z können gleich oder verschieden sein, falls p 2 oder 3 bedeutet.

Besonders bevorzugt sind Verbindungen der Formeln lah, lal, lam und lan, worin R¹ Alkyl, Alkoxy, Alkanoyloxy, Alkoxycarbonyl oder Alkoxycarbonyloxy bedeutet, A ° trans-1,4-Cyclohexylen oder 1,4-Phenylen (gegebenenfalls lateral durch ein Fluoratom substituiert) und Z° -CO-0-, -O-CO-oder -CH₂CH₂-, oder, falls A trans-1,4-Cyclohexylen ist, auch -CH₂-CHCN- oder -CHCN-CH₂- bedeutet.

Darunter sind diejenigen der Teilformel laa, lac, lad, lae, laf, lah, lak, lal, lam und lan bevorzugt. Besonders bevorzugt sind diejenigen der Teilformeln laa, lac, lae, lah, lak und lal, insbesondere lae, worin R¹ und R² verzweigte oder geradkettige Alkylgruppen oder Alkyloxygruppen mit 3 bis 12 C-Atomen bedeuten und laa, worin R¹ verzweigtes oder geradkettiges Alkoxy, Alkanoyloxy, Alkoxycarbonyl oder Alkoxycarbonyloxy bedeutet.

Die bevorzugten Verbindungen der Teilformel lak umfassen solche der Teilformeln laka bis lakc, 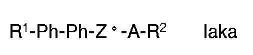 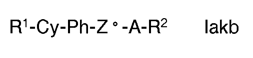 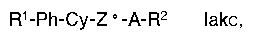 worin Z° vorzugsweise -CO-O-, -O-CO-, -CH₂CH₂-, -CH₂0- oder -OCH₂, insbesondere bevorzugt -CO-O-, -O-CO- oder -CH₂CH₂-, oder im Falle A = 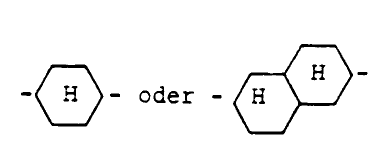 auch -CHCN-CH₂- oder -CH₂-CHCN- bedeutet.

In Verbindungen der Formel I, worin A 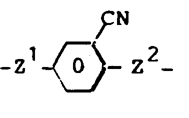 oder 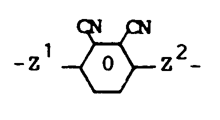 bedeutet, ist q vorzugsweise 1 und die anderen Ringe A sind vorzugsweise nicht lateral substituiert. Verbindungen dieses Typs enthalten vorzugsweise eine, insbesondere bevorzugt zwei trans-1,4-Cyclohexylen-Gruppen.

Die bevorzugten Verbindungen der Teilformel lal umfassen solche der Teilformeln lala bis lalc: 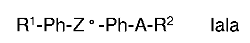 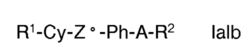 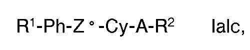 worin Z° vorzugsweise -CO-O-, -O-CO-, -CH₂CH₂-, -CH₂0- oder -OCH₂, insbesondere bevorzugt -CO-O-, -O-CO- oder -CH₂CH₂-, oder im Falle A = 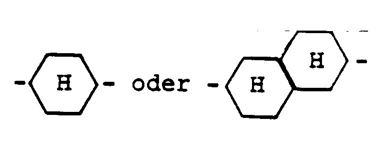 auch -CHCN-CH₂- oder -CH₂-CHCN- bedeutet.

Die bevorzugten Verbindungen der Teilformel Ib umfassen solche der Teilformeln Iba und Ibb: 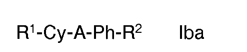 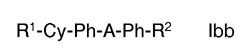 Darunter sind diejenigen der Teilformel Iba besonders bevorzugt.

Die bevorzugten Verbindungen der Teilformel Ic umfassen solche der Teilformeln Ica bis Ice: 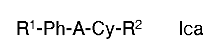 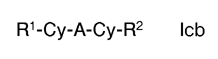 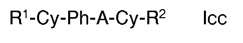 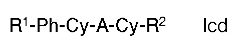 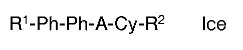 Darunter sind diejenigen der Teilformel Icb besonders bevorzugt.

Besonders bevorzugte Gruppen Q¹ und Q² sind diejenigen der Formel 1 bis 41: 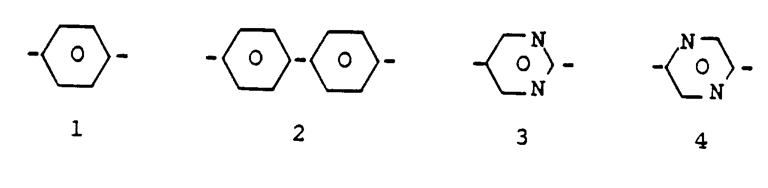 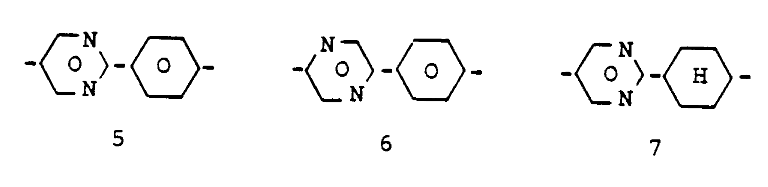 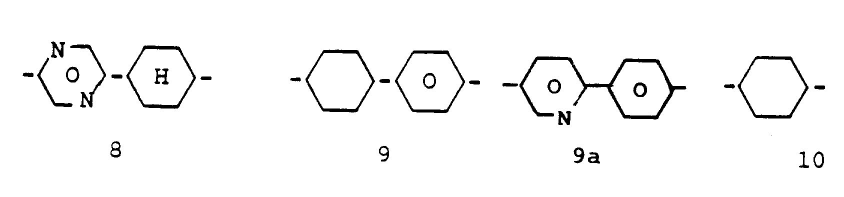 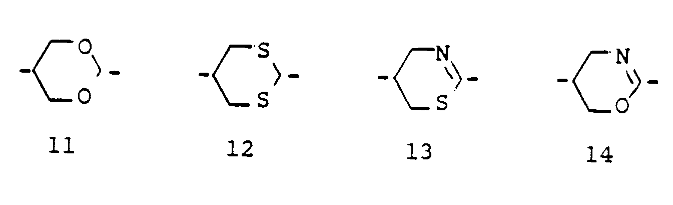 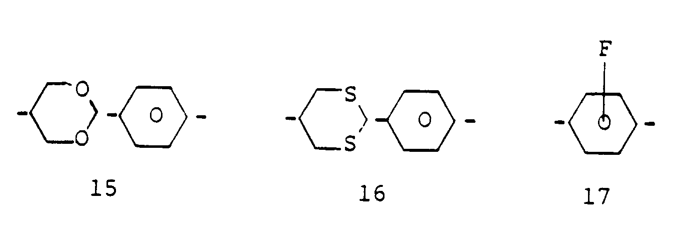 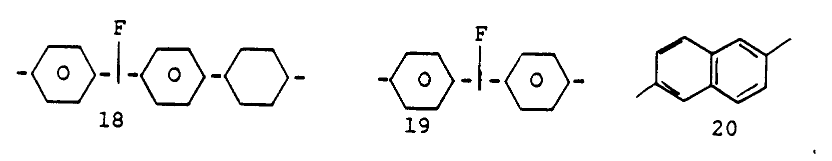 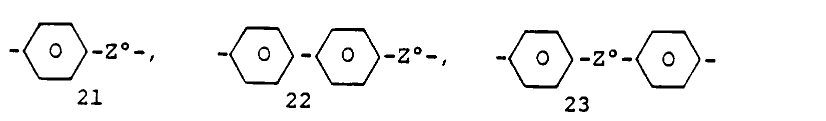 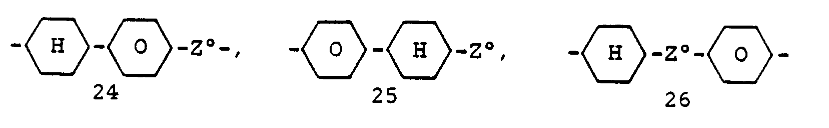 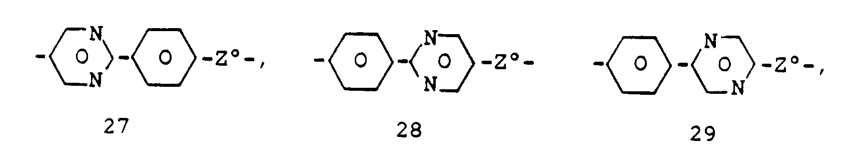 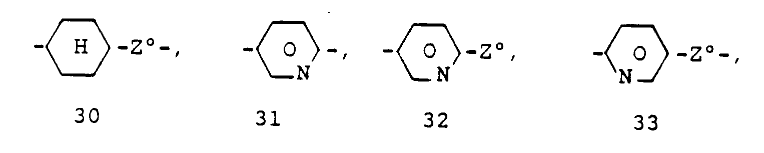 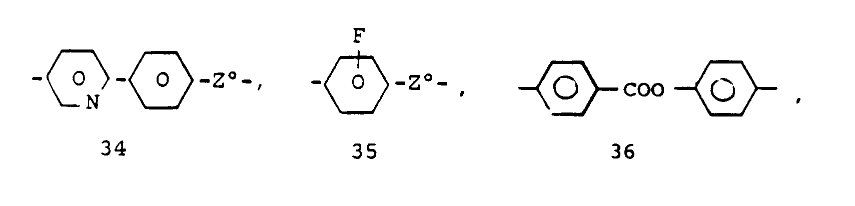 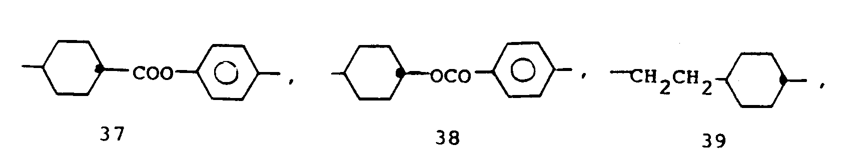 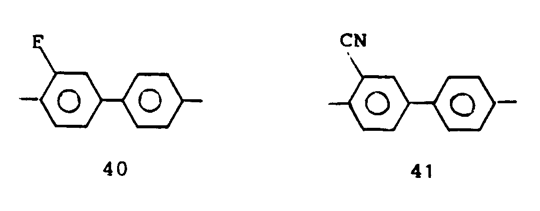 sowie deren drei Ringe nicht überschreitende Kombinationen. Auch die Spiegelbilder der unsymmetrischen vorstehenden Formeln kommen in Betracht.

Bevorzugt sind die Gruppen der Formeln 1, 2, 3, 5, 6, 7, 9, 10, 18, 19, 21, 22, 23, 24, 25, 26, 27, 29, 30, 34 und 36 bis 41.

Vorzugsweise ist einer der Reste Q¹ und Q² Cy, Cy-Z oder eine Einfachbindung; insbesondere bevorzugt eine Einfachbindung und der andere Rest Q¹ oder Q² eine Gruppe der Formel 2, 5, 6, 7, 8, 9, 18 oder 19.

A ist vorzugsweise eine Gruppe der Formel (A) 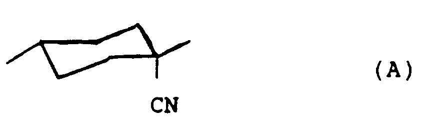 oder deren Spiegelbild in der angegebenen Konfiguration mit axialer Nitrilgruppe und trans-Stellung der Substituenten Q¹ und Q² oder trans-1,4-Cyclohexylen. Weiterhin bevorzugt sind Verbindungen der Formel I, worin A trans-1,4-Cyclohexylen bedeutet und/oder eine Gruppe Z ° -CHCNCH₂- oder -CH₂ CHCN- bedeutet.

In den Verbindungen der vor- und nachstehenden Formeln bedeuten R¹ und R² vorzugsweise R-, R-O-, R-O-CO-, R-O-COO- oder R-CO-O-. R ist vorzugsweise eine geradkettige Alkylgruppe mit vorzugsweise 5 bis 12 C-Atomen, worin auch eine oder zwei nicht endständige CH₂-Gruppen durch -O-, -O-CO-, -CHCH₃-, -CHCN-, -CH-Halogen -CHCH₃-O- und/oder -CH=CH- ersetzt sein können. R bedeutet beispielsweise bevorzugt Pentyl, Hexyl, Heptyl, Octyl, Nonyl, Decyl, Undecyl oder Dodecyl, ferner Methyl, Ethyl, Propyl, Butyl, 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6-oder 7- Oxaoctyl, 2-, 3-, 4-, 5-, 6-, 7- oder 8-Oxanonyl, 2-, 3-, 4-, 5-, 6-, 7-, 8- oder 9-Oxadecyl 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6-, oder 4,6-Dioxaheptyl, 1,4-Dioxaoctyl, 1,4,7-Trioxaoctyl, 1,4-Dioxanonyl, 1,4-Dioxadecyl.

Verbindungen der Formel I sowie der vor- und nachstehenden Teilformeln mit verzweigten Flügelgruppen R¹ bzw. R² können gelegentlich wegen einer besseren Löslichkeit in den üblichen flüssigkristallinen Basismaterialien von Bedeutung sein, insbesondere aber als chirale Dotierstoffe für chirale getiltete smektische Phasen, wenn sie optisch aktiv sind. Solche Verbindungen eignen sich jedoch auch als Komponenten nematischer flüssigkristalliner Phasen, insbesondere zur Vermeidung von reverse twist. Verzweigte Gruppen dieser Art enthalten in der Regel eine oder zwei Kettenverzweigungen. Vorzugsweise ist das asymmetrische Kohlenstoffatom mit zwei unterschiedlich substituierten C-Atomen, einem H-Atom und einem Substituenten ausgewählt aus der Gruppe Halogen (insbesondere F, CI oder Br), Alkyl oder Alkoxy mit jeweils 1 bis 5 C-Atomen und CN verknüpft. Der optisch aktive organische Rest R¹ bzw. R² hat vorzugsweise die Formel, 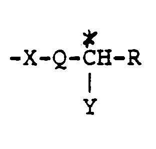 worin
X -CO-O-, -O-CO-, -O-CO-O-, -CO-, -O-, -S-, -CH = CH-, -CH = CH-COO- oder eine Einfachbindung,
Q Alkylen mit 1 bis 5 C-Atomen, worin auch eine nicht mit X verknüpfte CH₂-Gruppe durch -O-, -CO-, -O-CO-, -CO-O- oder -CH = CH- ersetzt sein kann, oder eine Einfachbindung,
Y CN, Halogen, Methyl oder Methoxy, und
R eine von Y verschiedene Alkylgruppe mit 1 bis 18 C-Atomen, worin auch eine oder zwei nicht benachbarte CH₂-Gruppen durch -O-, -CO-, -O-CO-, -CO-O- und/oder -CH=CH- ersetzt sein können,

bedeutet.
X ist vorzugsweise -CO-O-, -O-CO-, -CH = CH-COO- (trans) oder eine Einfachbindung. Besonders bevorzugt sind -CO-O- und -O-CO-.
Q ist vorzugsweise -CH₂-, -CH₂CH₂- oder eine Einfachbindung, insbesondere bevorzugt eine Einfachbindung.
Y ist vorzugsweise CH₃, -CN oder Cl, insbesondere bevorzugt -CN.
R ist vorzugsweise geradkettiges Alkyl mit 1 bis 10, insbesondere mit 1 bis 7, C-Atomen.

Bevorzugte verzweigte Reste sind Isopropyl, 2-Butyl (= 1-Methylpropyl), Isobutyl (= 2-Methylpropyl, Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 2-Octyloxy, 2-Chlorpropionyloxy, 2-Chlor-3-methylbutyryloxy, 2-Chlor-4-methylvaleryloxy, 2-Chlor-3-methylvaleryloxy, 2-Methyl-3-oxa-pentyl, 2-Methyl-3-oxa-hexyl.

Verbindungen der Formel I, worin einer der Reste R¹ und R² die Formel -X-Q-CHCN-R (mit den oben angegebenen bevorzugten Bedeutungen) hat, können z.B. nach D.A. Evans und J.M. Takacs, Tetrahedron Lett. 21, 4233 (1980) hergestellt werden.

Polyfluoralkylgruppen, worin auch eine oder mehrere CF₂-Gruppen durch eine Gruppierung ausgewählt aus der Gruppe -O-, -S-, -CO-, -CH-Halogen-, -O-CO-, -O-COO-, -CO-O- und -CH = CH- oder auch durch eine Kombination von zwei geeigneten Gruppierungen ersetzt sein können, wobei zwei Heteroatome nicht direkt miteinander verknüpft sind, bedeuten vorzugsweise Perfluoralkylgruppen mit 1 bis 15 C-Atomen, worin auch 1 bis 3 CF₂-Gruppen durch eine Gruppierung ausgewählt aus der Gruppe -O-, -CHHalogen-(insbesondere -CHF-), -O-CO-, -CO-O- und -O-COO- oder auch durch eine Kombination von zwei geeigneten Gruppierungen ersetzt sein können, wobei zwei Heteroatome nicht direkt miteinander verknüpft sind.

Besonders bevorzugte Gruppen sind diejenigen der Formeln R_{F}, R_{F}CH₂, R_{F}CH₂CH₂, R_{F}CH₂0 und R_{F}COO.

R_{F} ist vorzugsweise eine geradkettige Perfluoralkylgruppe mit vorzugsweise 2 bis 12 C-Atomen, worin auch ein oder mehrere Fluoratome (vorzugsweise 1 oder 2 Fluoratome, vorzugsweise in w- oder (",-1)-Position) durch H ersetzt sein können.

Bevorzugte Verbindungen der Formel I, worin mindestens einer der Reste R¹ und R² eine Polyfluoralkylgruppe ist, führen zu erfindungsgemäßen Phasen mit niedriger optischer Anisotropie und ausgeprägter S_{A}-Phase bei höheren Temperaturen.

Unter den Verbindungen der Formel I sowie der vor- und nachstehenden Teilformeln sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln 11 bis 118: 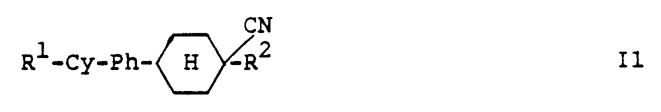 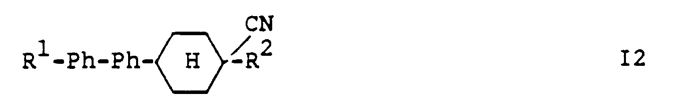 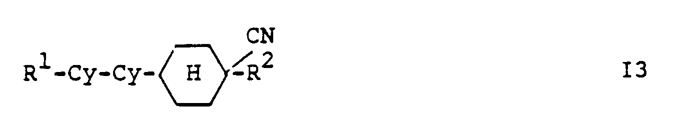 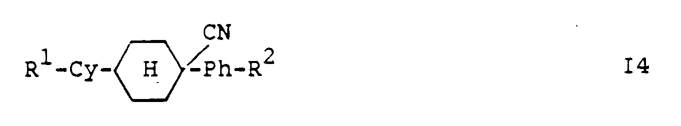 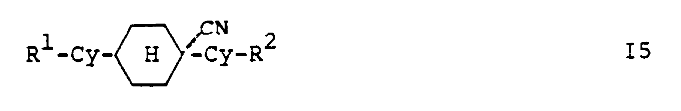 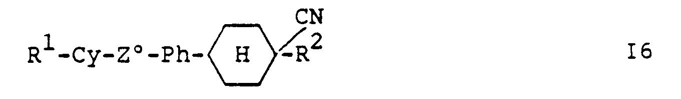 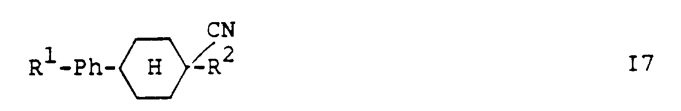 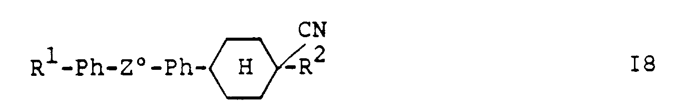 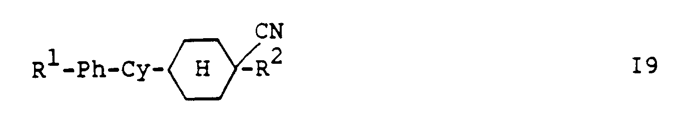 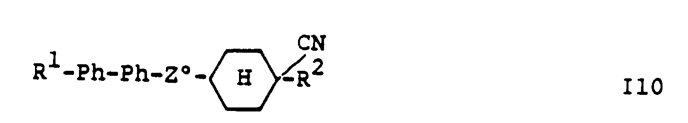 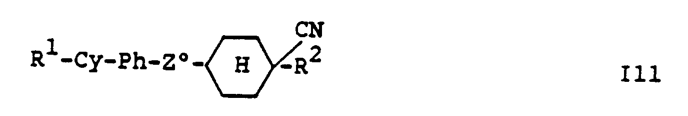 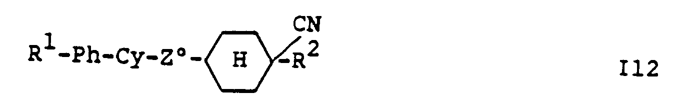 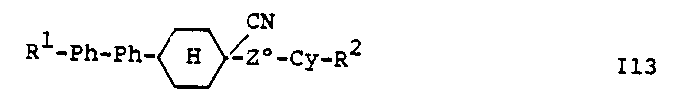 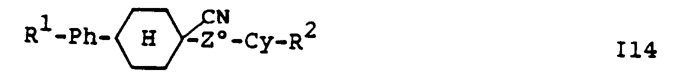 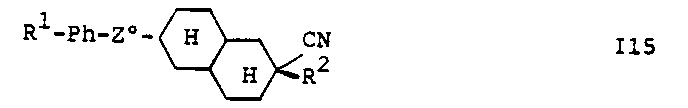 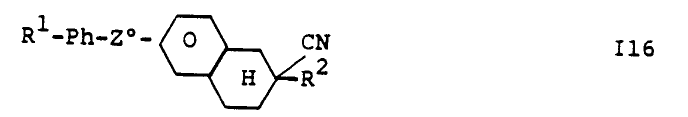 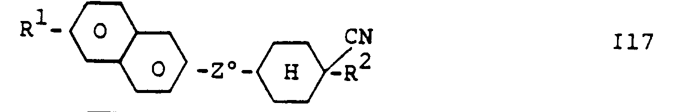 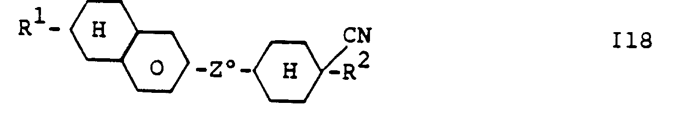 Eine weitere besonders bevorzugte kleinere Gruppe von Verbindungen sind diejenigen der Formeln 119 bis 122: 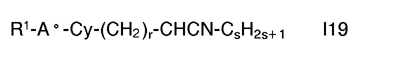 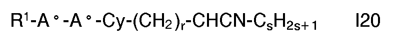 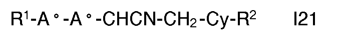 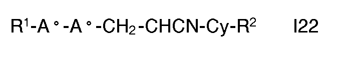 worin r 0, 1, 2 oder 3 bedeutet und (r+s)1 bis 14 ist.

Eine weitere besonders bevorzugte kleinere Gruppe von Verbindungen sind diejenigen der Formeln 123 bis 126: 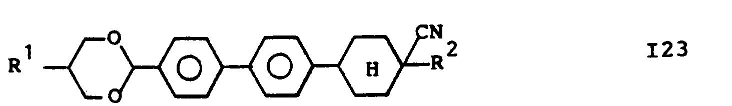 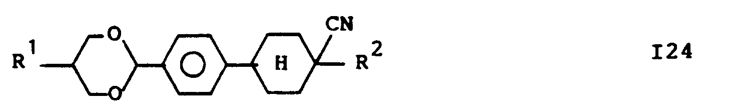 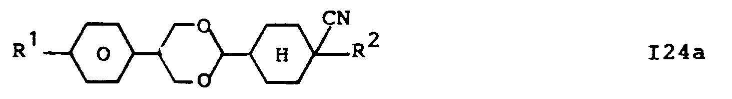 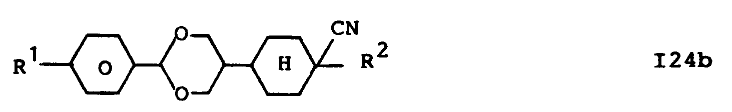 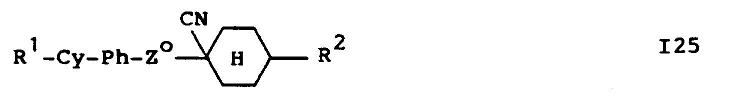 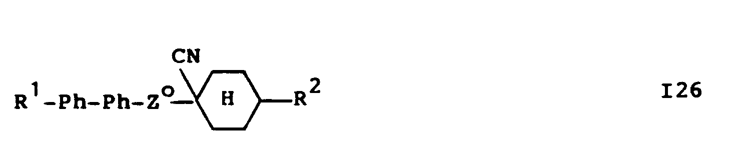 In den besonders bevorzugten Verbindungen der Formeln 12, 110 und 126 bedeutet -Ph-Ph- vorzugsweise eine Gruppe der Formel 2, 5, 9a oder 19 (wobei das Fluor in 2,3,2' oder 3'-Position sein kann). Besonders bevorzugt sind 2 und 40.

Verbindungen der Formel I, die keine S_{c-}Phasen aufweisen, eignen sich ebenfalls als Komponenten erfindungsgemäßer smektischer Phasen.

Besonders bevorzugte Verbindungen der Formel I sind im folgenden angegeben:
4-(4-Cyano-4-butylcyclohexyl)-4'-heptylbiphenyl, F. 56°, K. 122
4-(4-Cyano-4-butylcyclohexyl)-4'-octylbiphenyl, F. 42°, K. 118°
4-(4-Cyano-4-butylcyclohexyl)-4'-nonylbiphenyl
4-(4-Cyano-4-butylcyclohexyl)-4'-butoxybiphenyl
4-(4-Cyano-4-butylcyclohexyl)-4'-pentoxybiphenyl
4-(4-Cyano-4-butylcyclohexyl)-4'-hexoxybiphenyl
4-(4-Cyano-4-butylcyclohexyl)-4'-heptoxybiphenyl, F. 93°, K. 156
4-(4-Cyano-4-butylcyclohexyl)-4'-octoxybiphenyl, F. 80°, K. 154
4-(4-Cyano-4-butylcyclohexyl)-4'-nonoxybiphenyl, F. 89°, K. 150_{°}
4-(4-Cyano-4-pentylcyclohexyl)-4'-ethylbiphenyl
4-(4-Cyano-4-pentylcyclohexyl)-4'-propylbiphenyl
4-(4-Cyano-4-pentylcyclohexyl)-4'-butylbiphenyl, F. 75°, K. 128_{°}
4-(4-Cyano-4-pentylcyclohexyl)-4'-pentylbiphenyl
4-(4-Cyano-4-pentylcyclohexyl)-4'-hexylbiphenyl
4-(4-Cyano-4-pentylcyclohexyl)-4'-heptylbiphenyl, F. 54°, K. 127
4-(4-Cyano-4-pentylcyclohexyl)-4'-octylbiphenyl, F. 43°, K. 115°
4-(4-Cyano-4-pentylcyclohexyl)-4'-nonylbiphenyl
4-(4-Cyano-4-pentylcyclohexyl)-4'-butoxybiphenyl
4-(4-Cyano-4-pentylcyclohexyl)-4'-pentoxybiphenyl
4-(4-Cyano-4-pentylcyclohexyl)-4'-hexoxybiphenyl
4-(4-Cyano-4-pentylcyclohexyl)-4'-heptoxybiphenyl, F. 91 °, K. 161 °
4-(4-Cyano-4-pentylcyclohexyl)-4'-octoxybiphenyl, F. 93 ° , K. 160
4-(4-Cyano-4-pentylcyclohexyl)-4'-nonoxybiphenyl, F. 83 ° , K. 156°
4-(4-Cyano-4-hexylcyclohexyl)-4'-ethylbiphenyl
4-[4-Cyano-4-(2-methylbutylcyclohexyl)]-4'-ethylbiphenyl
4-[4-Cyano-4-(2-methylbutylcyclohexyl)]-4'-propylbiphenyl
4-[4-Cyano-4-(2-methylbutylcyclohexyl)]-4'-butylbiphenyl
4-[4-Cyano-4-(2-methylbutylcyclohexyl)]-4'-pentylbiphenyl
4-[4-Cyano-4-(2-methylbutylcyclohexyl)]-4'-hexylbiphenyl
4-[4-Cyano-4-(2-methylbutylcyclohexyl)]-4'-heptylbiphenyl
4-[4-Cyano-4-(2-methylbutylcyclohexyl)]-4'-octylbiphenyl
4-[4-Cyano-4-(2-methylbutylcyclohexyl)]-4'-nonylbiphenyl
4-[4-Cyano-4-(2-methylbutylcyclohexyl)]-4'-butoxybiphenyl
4-[4-Cyano-4-(2-methylbutylcyclohexyl)]-4'-pentoxybiphenyl
4-[4-Cyano-4-(2-methylbutylcyclohexyl)]-4'-hexoxybiphenyl
4-[4-Cyano-4-(2-methylbutylcyclohexyl)]-4'-heptoxybiphenyl, F. 66 ° , K. 131,4 ° , 131,0 Ch/Bp
4-[4-Cyano-4-(2-methylbutylcyclohexyl)]-4'-octoxybiphenyl
4-[4-Cyano-4-(2-methylbutylcyclohexyl)]-4'-nonoxybiphenyl
4-(4-Cyano-4-hexylcyclohexyl)-4'-propylbiphenyl
4-(4-Cyano-4-hexylcyclohexyl)-4'-butylbiphenyl
4-(4-Cyano-4-hexylcyclohexyl)-4'-pentylbiphenyl
4-(4-Cyano-4-hexylcyclohexyl)-4'-hexylbiphenyl
4-(4-Cyano-4-hexylcyclohexyl)-4'-heptylbiphenyl, F. 66 ° , K. 125
4-(4-Cyano-4-hexylcyclohexyl)-4'-octylbiphenyl
4-(4-Cyano-4-hexylcyclohexyl)-4'-nonylbiphenyl
4-(4-Cyano-4-hexylcyclohexyl)-4'-butoxybiphenyl
4-(4-Cyano-4-hexylcyclohexyl)-4'-pentoxybiphenyl
4-(4-Cyano-4-hexylcyclohexyl)-4'-hexoxybiphenyl
4-(4-Cyano-4-hexylcyclohexyl)-4'-heptoxybiphenyl, F. 88 ° , K. 156
4-(4-Cyano-4-hexylcyclohexyl)-4'-octoxybiphenyl, F. 90 ° , K. 155
4-(4-Cyano-4-hexylcyclohexyl)-4'-nonoxybiphenyl, F. 87 ° , K. 152
4-(4-Cyano-4-heptylcyclohexyl)-4'-ethylbiphenyl
4-(4-Cyano-4-heptylcyclohexyl)-4'-propylbiphenyl
4-(4-Cyano-4-heptylcyclohexyl)-4'-butylbiphenyl
4-(4-Cyano-4-heptylcyclohexyl)-4'-pentylbiphenyl
4-(4-Cyano-4-heptylcyclohexyl)-4'-hexylbiphenyl
4-(4-Cyano-4-heptylcyclohexyl)-4'-heptylbiphenyl, F. 61 ° , K. 124
4-(4-Cyano-4-heptylcyclohexyl)-4'-octylbiphenyl, F. 64 ° , K. 125
4-(4-Cyano-4-heptylcyclohexyl)-4'-nonylbiphenyl
4-(4-Cyano-4-heptylcyclohexyl)-4'-butoxybiphenyl
4-(4-Cyano-4-heptylcyclohexyl)-4'-pentoxybiphenyl
4-(4-Cyano-4-heptylcyclohexyl)-4'-hexoxybiphenyl
4-(4-Cyano-4-heptylcyclohexyl)-4'-heptoxybiphenyl, F. 87°, K. 155°
4-(4-Cyano-4-heptylcyclohexyl)-4'-octoxybiphenyl, F. 83 ° , K. 154°
4-(4-Cyano-4-heptylcyclohexyl)-4'-nonoxybiphenyl, F. 81 ° , K. 152°
4-(4-Cyano-4-octylcyclohexyl)-4'-ethylbiphenyl
4-(4-Cyano-4-octylcyclohexyl)-4'-propylbiphenyl
4-(4-Cyano-4-octylcyclohexyl)-4'-butylbiphenyl
4-(4-Cyano-4-octylcyclohexyl)-4'-pentylbiphenyl, F. 52°, K. 124°
4-(4-Cyano-4-octylcyclohexyl)-4'-hexylbiphenyl
4-(4-Cyano-4-octylcyclohexyl)-4'-heptylbiphenyl, F. 61 ° , K. 122°
4-(4-Cyano-4-octylcyclohexyl)-4'-octylbiphenyl, F. 65 ° , K. 125°
4-(4-Cyano-4-octylcyclohexyl)-4'-nonylbiphenyl
4-(4-Cyano-4-octylcyclohexyl)-4'-butoxybiphenyl
4-(4-Cyano-4-octylcyclohexyl)-4'-pentoxybiphenyl
4-(4-Cyano-4-octylcyclohexyl)-4'-hexoxybiphenyl
4-(4-Cyano-4-octylcyclohexyl)-4'-heptoxybiphenyl, F. 85°, K. 151 °
4-(4-Cyano-4-octylcyclohexyl)-4'-octoxybiphenyl, F. 81 ° , K. 150°
4-(4-Cyano-4-octylcyclohexyl)-4'-nonoxybiphenyl, F. 72 ° , K. 149°
4-(4-Cyano-4-nonylcyclohexyl)-4'-ethylbiphenyl
4-(4-Cyano-4-nonylcyclohexyl)-4'-propylbiphenyl
4-(4-Cyano-4-nonylcyclohexyl)-4'-butylbiphenyl
4-(4-Cyano-4-nonylcyclohexyl)-4'-pentylbiphenyl
4-(4-Cyano-4-nonylcyclohexyl)-4'-heptylbiphenyl
4-(4-Cyano-4-nonylcyclohexyl)-4'-octylbiphenyl
4-(4-Cyano-4-nonylcyclohexyl)-4'-nonylbiphenyl
4-(4-Cyano-4-nonylcyclohexyl)-4'-butoxybiphenyl
4-(4-Cyano-4-nonylcyclohexyl)-4'-pentoxybiphenyl
4-(4-Cyano-4-nonylcyclohexyl)-4'-hexoxybiphenyl
4-(4-Cyano-4-nonylcyclohexyl)-4'-heptoxybiphenyl
4-(4-Cyano-4-nonylcyclohexyl)-4'-octoxybiphenyl
4-(4-Cyano-4-nonylcyclohexyl)-4'-nonoxybiphenyl
4-Cyan-4-heptylcyclohexancarbonsäure-(4'-octyloxybiphenyl-4-ylester),F. 86 ° , K. 185
4-Cyan-4-heptylcyclohexancarbonsäure-p-(trans-4-propylcyclohexyl)-phenylester, F. 84°, K. 153°
1-(1-Cyan-4-heptylcyclohexyl)-2-(4'-octyloxybiphenyl-4-yl)-ethan , F. 85°, K. 141 °
1-Cyan-4-pentylcyclohexancarbonsäure-(4'-octyloxybiphenyl-4-ylester),F. 53 ° , K. 149°
4-Cyan-4-heptylcyclohexancarbonsäure-p-heptylphenylester, F. 38 ° , K. 56
4-Cyan-4-heptylcyclohexancarbonsäure-p-heptyloxyphenylester, F. 35 °, K. 84
1-Cyan-4-pentylcyclohexancarbonsäure-(4'-hexylbiphenyl-4-ylester), F. 46 ° , K. 121 °
1-Cyan-1-pentyl-4-[4'-(trans-4-pentylcyclohexyl)-biphenyl-4-yl]-cyclohexan, F. 96 ° , K. 285
1-Cyan-4-pentylcyclohexancarbonsäure-p-(trans-4-propylcyclohexyl)-phenylester, F. 79 ° , K. 114°
1-(trans-4-Pentylcyclohexyl)-2-[1-cyan-4-(4'-heptyloxybiphenyl-4-yl)-cyclohexyl]-ethan, F. 100°, K. 251 °
p-(4-Cyan-4-heptylcyclohexyl)-benzoesäure-p-heptylphenylester, F. 88 ° , K. 138°

Besonders bevorzugt sind optisch aktive Verbindungen der Formel I, worin eine der Gruppen R¹ und R² -O-C₂ H₄ -CHCH₃ -C₃ H₆ -CH(CH₃)₂ oder -COO-C₂ H₄ -CHCH₃ -C₃ H₆ -CH(CH₃)₂ bedeuten.

Diese Verbindungen sind entsprechend analogen Verbindungen erhältlich durch Veretherung bzw. Veresterung mit Dihydrocitronellol bzw. dessen reaktionsfähigen Derivaten. Besonders bevorzugt sind die optisch aktiven Verbindungen
r-1-Cyan-cis-4-[4'-(3,7-dimethyloctyloxy)-biphenyl-4-yl]-1-pentylcyclohexan
r-1-Cyan-cis-4-[4'-(3,7-dimethyloctyloxy)-biphenyl-4-yl]-1-hexylcyclohexan
r-1-Cyan-cis-4-[4'-(3,7-dimethyloctyloxy)-biphenyl-4-yl]-1-heptylcyclohexan
r-1-Cyan-cis-4-[4'-(3,7-dimethyloctyloxy)-biphenyl-4-yl]-1-octylcyclohexan, F. 51 °, K. 113°
r-1-Cyan-cis-4-[4'-(3,7-dimethyloctyloxy)-biphenyl-4-yl]-1-nonylcyclohexan
r-1-Cyan-cis-4-[4'-(3,7-dimethyloctyloxy)-biphenyl-4-yl]-1-decylcyclohexan
r-1-Cyan-cis-4-[4'-(3,7-dimethyloctyloxycarbonyl)-biphenyl-4-yl]-1-pentylcyclohexan
r-1-Cyan-cis-4-[4'-(3,7-dimethyloctyloxycarbonyl)-biphenyl-4-yl]-1-hexylcyclohexan
r-1-Cyan-cis-4-[4'-(3,7-dimethyloctyloxycarbonyl)-biphenyl-4-yl]-1-heptylcyclohexan
r-1-Cyan-cis-4-[4'-(3,7-dimethyloctyloxycarbonyl)-biphenyl-4-yl]-1-octylcyclohexan
r-1-Cyan-cis-4-[4'-(3,7-dimethyloctyloxycarbonyl)-biphenyl-4-yl]-1-nonylcyclohexan
r-1-Cyan-cis-4-[4'-(3,7-dimethyloctyloxycarbonyl)-biphenyl-4-yl]-1-decylcyclohexan

Alle Verbindungen der Formel I werden nach an sich bekannten Methoden hergestellt wie sie in der Literatur (z.B. in den Standardwerken wie Houben-Weyl, Methoden der Organischen Chemie, Georg-Thieme-Verlag, Stuttgart) oder in den nachfolgenden Beispielen beschrieben sind, und zwar unter Reaktionsbedingungen, die für die genannten Umsetzungen bekannt und geeignet sind.Dabei kann man auch von an sich bekannten, hier nicht näher erwähnten Varianten Gebrauch machen.

Formel I umfaßt zum überwiegenden Teil bekannte Verbindungen, wie beispielsweise die bevorzugten, in DOS 32 31 707, 33 19 781, 33 20 024, 34 07 013, 34 43 929, 33 32 690, 33 32 691, 33 32 692, 29 33 563, 28 53 728, 26 13 293, 34 01 320, 31 36 624, 30 40 632, 32 05 766, 22 40 864, 29 37 700, 34 10 734, 33 24 686, EP-OS 0 085 995, EP-OS 0 084 194, DD 116 732, FR 24 25 469, FR 24 19 966, USP 4 237 026, USP 3 953 491, USP 4,225,454 bzw. in H.J. Deutscher et al., J. prakt. Chemie, 321, 569 (1979) und J.C. Dubois et al. Mol. Cryst. Liq. Cryst. 47, 193 (1978) beschriebenen Verbindungen.

Bevorzugte erfindungsgemäße Phasen enthalten mindestens eine Verbindung der Formel II, 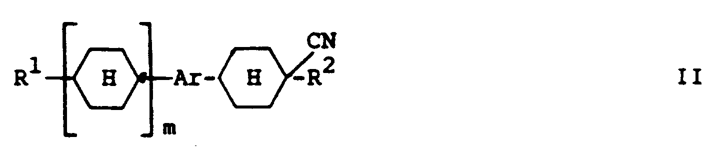 worin
R¹ und R² jeweils eine geradkettige Alkylgruppe mit 1 bis 15 C-Atomen, worin auch eine oder mehrere nicht benachbarte CH₂-Gruppen durch -O-, -CO-, -O-CO-, -O-COO-, -CO-O-, -CHCH₃-, -CH = CH-, -CH-Halogen und/oder -CHCN- ersetzt sein können,
m 0 oder 1, und
Ar 1,4-Phenylen, 4,4'-Bi-phenylyl oder 2,6-Naphthylen bedeutet, worin jeweils eine oder mehrere CH-Gruppen durch N ersetzt sind.

Die Verbindungen der Formel II besitzen eine hohe chemische Stabilität. Sie sind farblos und gut mischbar mit allen gebräuchlichen Flüssigkristallen. Ihr Einsatz in flüssigkristallinen Phasen führt zu breiteren Mesophasenbereichen und verbesserten Werten für die Spontanpolarisation in chiralen getilteten smektischen Phasen. Die erfindungsgemäßen Phasen eignen sich somit sehr gut für Displays, die auf dem Prinzip der SSFLC-Technolgie beruhen.

R¹ und R² haben vorzugsweise die bei Formel I angegebenen bevorzugten Bedeutungen.

Ar ist vorzugsweise eine Gruppe der Formel a bis j: 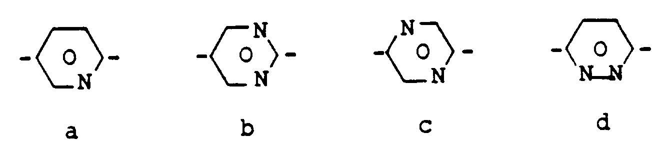 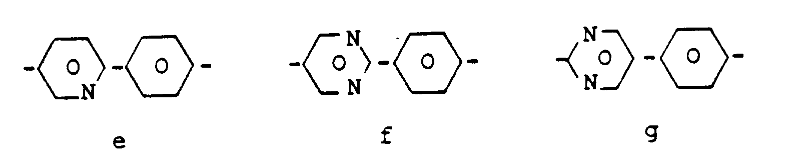 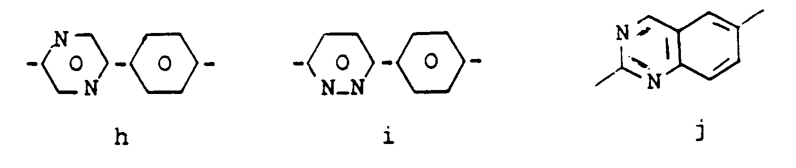 Gruppen der Formeln a, b, c, e, f, g und h sind bevorzugt. Besonders bevorzugt sind e und f.

Die Verbindungen der Formel II können beispielsweise durch Umsetzung von entsprechenden Cyclohexancarbonitrilen (Formel 11, R² = H) mit einem Halogenid der Formel R²⁻Halogen erhalten werden.

Das Nitril wird zweckmäßig zunächst mit einer starken Base wie NaH, NaNH₂, Lithiumdiisopropylamid, -piperidid oder -2,5-diisopropylpiperidid oder K-tert.Butylat in das entsprechende Carbanion übergeführt, vorzugsweise in einem inerten Lösungsmittel, beispielsweise einem Kohlenwasserstoff wie Toluol, einem Ether wie THF oder Dioxan, einem Amid wie DMF, einem Sulfoxid wie Dimethylsulfoxid oder einem Gemisch derartiger Lösungsmittel. Nach Zugabe von R²⁻Halogen hält man zweckmäßig 0,5 bis 16 Stunden bei Temperaturen zwischen 0 und 150°.

Die Halogenide sind beispielsweise aus den entsprechenden Alkoholen erhältlich. Halogen ist vorzugsweise Brom oder Jod.

Die bevorzugten Bedeutungen für Gruppen aus Formel I gelten analog auch für die Verbindungen der Formel II.

Die vorliegende Erfindung betrifft ebenfalls neue optisch aktive Verbindungen der Formel I-A
R¹-Q¹-A-(Q²)q-R² I-A

worin
R¹ und R² jeweils unabhängig voneinander eine Alkylgruppe oder eine Polyfluoralkylgruppe mit jeweils 1 bis 15 C-Atomen, worin auch eine oder mehrere CH₂-Gruppen bzw. CF₂-Gruppen durch eine Gruppierung ausgewählt aus der Gruppe -O-, -S-, -CO-, -CH-Halogen, -CHCN-, -O-CO-, -O-COO-, -CO-O- und -CH = CH- oder auch durch eine Kombination von zwei geeigneten Gruppierungen ersetzt sein können, wobei zwei Heteroatome nicht direkt miteinander verknüpft sind, einer der Reste R¹ und R² auch Halogen,
A 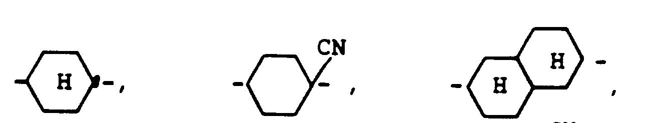 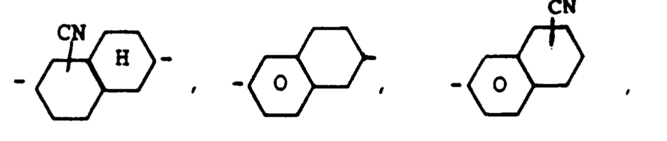 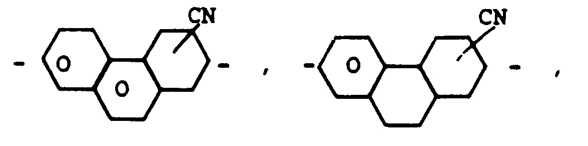 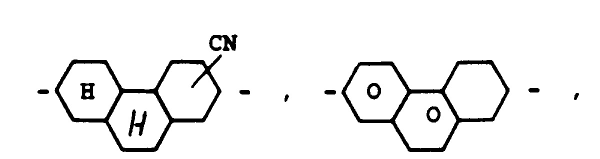 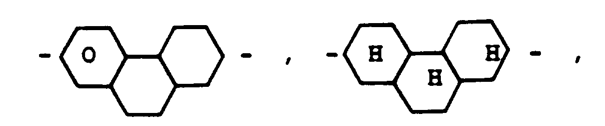 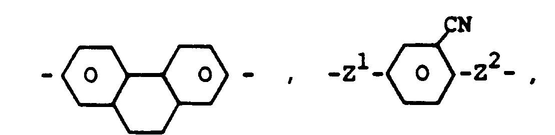 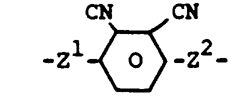 oder eine dieser Gruppen,
   worin eine oder mehrere CH₂-Gruppen durch 0 und/oder S oder aliphatische und/oder aromatische CH-Gruppen durch N ersetzt sind,
q 0 oder 1,
Q1 und Q2 jeweils unabhängig voneinander, -(A ° -Z ° )p ,wobei
A unsubstituiertes oder ein- oder mehrfach durch Halogenatome, CH₃- und/oder Nitril-Gruppen substituiertes 1,4-Cyclohexylen, worin auch eine oder zwei nicht benachbarte CH₂-Gruppen durch -O- und/oder -S- und/oder eine 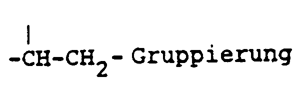 durch 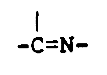 ersetzt sein können (Cy), oder unsubstituiertes oder ein- oder mehrfach durch Halogenatome, CH₃- und/oder Nitril-Gruppen substituiertes 1,4-Phenylen,
   worin auch eine oder mehrere CH-Gruppen durch N ersetzt sein können (Ph) bedeutet, einer der Reste A auch 2,6-Naphthylen (Na) oder Tetrahydro-2,6-naphthylen (4H-Na), gegebenenfalls durch Halogen oder CN substituiert,
Z°, Z¹ und Z² jeweils unabhängig voneinander -CO-O-, -O-CO-, -CH2O-,-OCH₂-, -CH₂CH₂-, -CHCN-CH₂-, -CH₂-CHCN- oder eine Einfachbindung, und
p 1, 2 oder 3, oder im Falle A = Tetra- oder Octahydrophenanthren auch O bedeutet, wobei im Falle A = 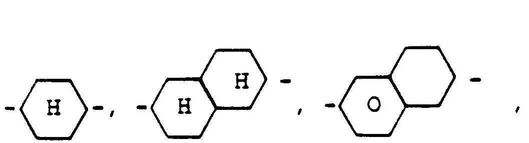 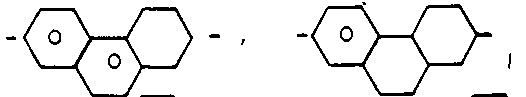 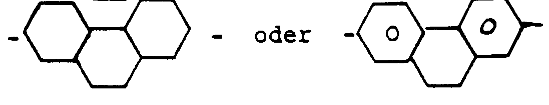 mindestens eine Gruppe Z° -CHCNCH₂- oder -CH₂CHCN- bedeutet mit der Maßgabe, daß mindestens eine der Gruppen R¹ und R² die Formel -O-Q °-O-Alkyl oder -CO-O-Q ° -O-Alkyl hat, worin Alkyl eine Alkylgruppe mit 1 bis 10 C-Atomen und Q ° - HCH₃-CO-, -CO-HCH₃-, -HCH₃-CH₂- oder -CH₂-HCH₃- bedeutet.

Die Verbindungen der Formel I-A besitzen eine hohe chemische Stabilität. Sie sind farblos und gut mischbar mit vielen gebräuchlichen Flüssigkristallen. Ihr Einsatz in flüssigkristallinen Phasen führt zu breiteren Mesophasenbereichen und/oder verbesserten Werten für die Spontanpolarisation in chiralen getilteten smektischen Phasen. Die erfindungsgemäßen Phasen mit Verbindungen der Formel I-A eignen sich somit sehr gut für Displays, die auf dem Prinzip der SSFLC-Technologie beruhen.

Die Verbindungen der Formel I-A sind nach bekannten Methoden herstellbar, wie sie in der Literatur (z.B. in den Standardwerken wie Houben-Weyl, Methoden der Organischen Chemie, Georg-Thieme-Verlag, Stuttgart; EP-OS O 175 591; WO 86/02938) beschrieben sind, und zwar unter Reaktionsbedingungen, die für die erforderlichen Umsetzungen (z.B. Veresterungen oder Veretherungen) bekannt und geeignet sind. Dabei kann man auch von an sich bekannten, hier nicht näher erwähnten Varianten Gebrauch machen.

Die bevorzugten Bedeutungen für Reste der Formel I gelten analog auch für die Verbindungen der Formel I-A. Vorzugsweise enthalten die Verbindungen der Formel I-A mindestens einen Rest der Formel -O-HCH₃-CO-O-Alkyl. Alkyl ist vorzugsweise eine geradkettige Alkylgruppe mit vorzugsweise 2 bis 6 C-Atomen.

Die Erfindung betrifft ferner neue Tetralincarbonitrile der Formel I' 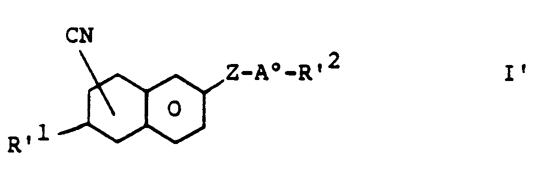 worin
R'¹ eine Alkylgruppe mit 1-15 C-Atomen, worin auch eine oder mehrere nicht benachbarte CH₂-Gruppen durch -O-, -O-CO-, -CO- und/oder -CH = CH-(trans) ersetzt sein können, oder trans-4-R'¹⁻Cyclohexyl,
Z -CH₂CH₂-, -CO-O-, -O-CO-, -O-CH₂-, -CH₂0- oder eine Einfachbindung,
A unsubstituiertes oder durch ein oder zwei Halogenatome, CH₃- und/oder CN-Gruppen substituiertes 1,4-Phenylen worin auch eine oder mehrere CH-Gruppen durch N ersetzt sein können, trans-1,4-Cyclohexylen, worin auch eine oder zwei nicht benachbarte CH₂-Gruppen durch -O- und/oder -S- ersetzt sein können, 1,4-Bicyclo(2.2.2)-octylen oder eine Einfachbindung,
R'² eine Alkylgruppe mit 1-15 C-Atomen, worin auch eine oder mehrere nicht benachbarte CH₂-Gruppen durch -O-, -O-CO-, -CO- und/oder -CH = CH-(trans) ersetzt sein können, F, Cl, Br, CN oder, falls mindestens eine der Gruppen Z und A_{°} nicht für eine Einfachbindung steht, auch H

bedeuten, wobei im aromatischen Ring auch eine oder mehrere CH-Gruppen durch N ersetzt sein können und/oder im hydrierten Ring eine oder zwei nicht benachbarte CH₂-Gruppen durch O und/oder S ersetzt sein können.

Der Einfachheit halber bedeuten im folgenden "Phe" eine 1,4-Phenylengruppe, "Cyc" eine 1,4-Cyclohexylengruppe, "Dio" eine 1,3-Dioxan-2,5-diylgruppe, "Bi" eine Bicyclo-(2.2.2)-octylengruppe, "Pyr" eine Pyrimidin-2,5-diylgruppe und Tet die Gruppe 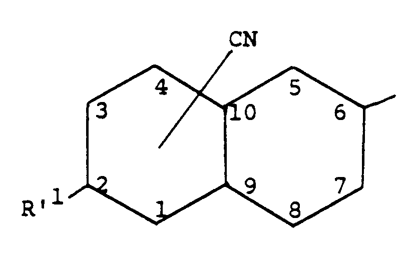 Ähnliche Verbindungen sind z.B. aus der DE-OS 33 19 781 bekannt. Die dort angegebenen Verbindungen enthalten jedoch im Gegensatz zu den vorliegenden ein perhydriertes Dekalin-System.

Die Verbindungen der Formel I' können wie ähnliche Verbindungen als Komponenten flüssigkristalliner Dielektrika verwendet werden, insbesondere für Displays, die auf dem Prinzip der verdrillten Zelle, dem Prinzip der SSFLC-Technologie, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Dielektrika geeignet sind. Diese Aufgabe wurde durch die Bereitstellung der Verbindungen der Formel I' gelöst.

Es wurde gefunden, daß die Verbindungen der Formel I' als Komponenten flüssigkristalliner Dielektrika vorzüglich geeignet sind. Insbesondere sind mit ihrer Hilfe stabile flüssigkristalline Phasen mit stark negativer dielektrischer Anisotropie und damit kleiner Schwellen- bzw. Steuerspannung elektrooptischer Effekte und vergleichsweise niedriger Viskosität herstellbar.

Mit der Bereitstellung der Verbindungen der Formel I' wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I' besitzen einen breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind; es können aber auch Verbindungen der Formel I' flüssigkristallinen Basismaterialien aus anderen Verbindungsklassen zugesetzt werden, um beispielsweise die dielektrische und/oder optische Anisotropie eines solchen Dielektrikums zu senken. Die Verbindungen der Formel I' eignen sich ferner als Zwischenprodukte zur Herstellung anderer Substanzen, die sich als Bestandteile flüssigkristalliner Dielektrika verwenden lassen.

Die Verbindungen der Formel I' sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I' sowie ein Verfahren zu ihrer Herstellung, dadurch gekennzeichnet, daß man eine Verbindung, die sonst der Formel I' entspricht, aber an stelle von H-Atomen eine oder mehrere reduzierbare Gruppen und/oder C-C-Bindungen enthält, mit einem reduzierenden Mittel behandelt,
oder daß man an eine Verbindung, die sonst der Formel I entspricht, aber im Tetralinrest keine CN-Gruppe, aber eine zusätzliche Doppelbindung enthält, HCN anlagert,
oder daß man zur Herstellung von Estern der Formel I' (worin Z -CO-O- oder -O-CO- oder worin R'² -O-CO-Alkyl oder -COO-Alkyl bedeutet) eine entsprechende Carbonsäure oder eines ihrer reaktionsfähigen Derivate mit einem entsprechenden Alkohol oder einem seiner reaktionsfähigen Derivate umsetzt,
oder daß man zur Herstellung von Dioxanderivaten der Formel I' (worin A ° 1,3-Dioxan-2,5-diyl bedeutet) einen entsprechenden Aldehyd mit einem entsprechenden Diol umsetzt,
oder daß man ein entsprechendes Carbonsäureamid dehydratisiert oder ein entsprechendes Carbonsäurehalogenid mit Sulfamid umsetzt oder eine entsprechende Chlor- oder Bromverbindung mit einem Cyanid umsetzt,
oder daß man zur Herstellung von Tetralin-2-carbonitrilen der Formel I' ein Nitril der Formel II' 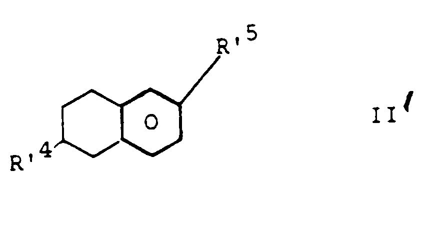 worin
R'⁴ CN und R'⁵ -Z-A°-R'²
bedeuten und
A ° , Z, und R_{'}²

die angegebenen Bedeutungen haben, mit einer Verbindung der Formel III'
Q-X' III

worin
Q R'¹ und
X' Cl, Br, J, OH oder eine reaktionsfähig veresterte OH-Gruppe

bedeuten und R'¹ die angegebenen Bedeutungen hat, umsetzt,
oder daß man eine Verbindung, die der Formel I' entspricht, aber an Stelle einer C-C-Bindung zwischen dem die CN-Gruppe tragenden C-Atom und einem diesem benachbarten C-Atom ein zusätzliches H-Atom (an dem die CN-Gruppe tragenden C-Atom) und eine zusätzliche Gruppe X' (an dem diesem benachbarten C-Atom, wobei X' die angegebene Bedeutung hat) enthält, unter Abspaltung von HX' cyclisiert,
oder daß man zur Herstellung von Ethern der Formel I' (worin R'¹ und/oder R'² Alkylketten bedeuten worin bis zu 2 CH₂-Gruppen durch O-Atome ersetzt sind und/oder Z eine -OCH₂- oder -CH₂0-Gruppe ist) eine entsprechende Hydroxyverbindung verethert.

Weiterhin ist Gegenstand der Erfindung die Verwendung der Verbindungen der Formel I' als Komponenten flüssigkristalliner Dielektrika. Gegenstand der Erfindung sind ferner flüssigkristalline Dielektrika mit einem Gehalt an mindestens einer Verbindung der Formel I' sowie elektrooptische Anzeigeelemente, die derartige Dielektrika enthalten.

Vor- und nachstehend haben A°, Q, R'¹, R'², R'⁴, R'⁵, X' und Z die angegebene Bedeutung, sofern nicht ausdrücklich etwas anderes vermerkt ist.

Die Verbindungen der Formel I' umfassen dementsprechend Verbindungen der Teilformeln I'a bis I'jj: 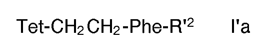 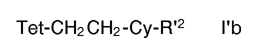 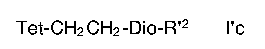 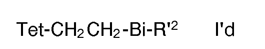 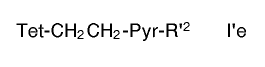 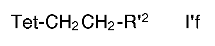 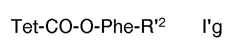 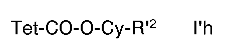 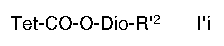 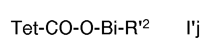 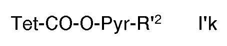 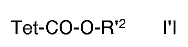 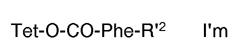 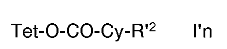 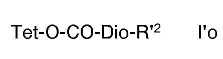 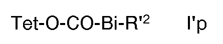 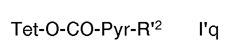 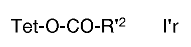 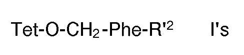 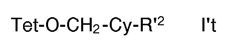 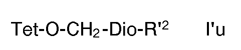 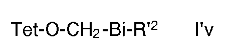 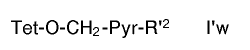 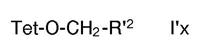 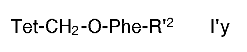 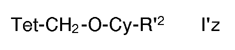 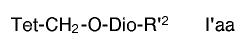 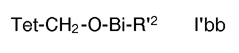 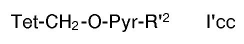 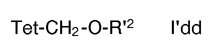 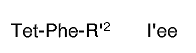 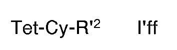 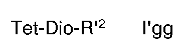 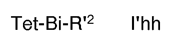 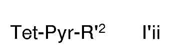 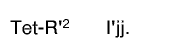 (In I'jj ist R'² ungleich H). Z ist bevorzugt eine Einfachbindung. A ist bevorzugt eine Einfachbindung oder Cy. Bevorzugt sind demnach Verbindungen der Formel I'ff und I'jj.

Die CN-Gruppe steht im Tetralin-System bevorzugt am tertiären C-Atom oder in der 1-Stellung; sie kann aber auch in 3- oder 4-Stellung stehen.

In den Verbindungen der vor- und nachstehenden Formeln bedeuten R'¹ und R'² vorzugsweise Alkyl, ferner Alkoxy (insbesondere, wenn diese Reste an einer Phe-Gruppe stehen) oder eine andere Oxaalkylgruppe.

X' ist vorzugsweise CI oder Br, aber auch J, OH oder reaktionsfähig verestertes OH wie Alkylsulfonyloxy mit insbesondere 1-6 C-Atomen (z.B. Methylsulfonyloxy) oder Arylsulfonyloxy mit insbesondere 6-10 C-Atomen (z.B. Phenyl-, p-Tolyl- oder Naphthylsulfonyloxy).

In den Verbindungen der vor- und nachstehenden Formeln können die Alkylreste, in denen auch eine ("Alkoxy" bzw. "Oxaalkyl") oder zwei ("Alkoxyalkoxy" bzw. "Dioxaalkyl") CH₂-Gruppen durch O-Atome ersetzt sein können, geradkettig oder verzweigt sein. Vorzugsweise sind sie geradkettig, haben 2, 3, 4, 5, 6, 7, 8, 9 oder 10 C-Atome und bedeuten demnach bevorzugt Ethyl, Propyl, Butyl, Pentyl, Hexyl, Heptyl, Octyl, Nonyl, Decyl, Ethoxy, Propoxy, Butoxy, Pentoxy, Hexoxy, Heptoxy, Octoxy, Nonoxy, Decoxy, 2-Oxapropyl (= Methoxymethyl), 2-(= = Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methyl, Methoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxyhexyl, 1,3-, 1,4-, 1,5-, 1,6-, 2,4-, 2,5-, 2,6-, 3,5-, 3,6- oder 4,6-Dioxaheptyl.

Verbindungen der Formeln I' sowie l'a bis I'jj mit verzweigten Flügelgruppen R'¹ bzw. R'² können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel eine Kettenverzweigung.
Bevorzugte verzweigte Reste R'¹ und R'² sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Unter den Verbindungen der Formeln I' sowie l'a bis I'z sind diejenigen bevorzugt, in denen mindestens einer der darin enthaltenen Reste eine der angegebenen bevorzugten Bedeutungen hat und/oder worin die CN-Gruppe in einer der angegebenen bevorzugten Stellungen steht. Besonders bevorzugte kleinere Gruppen von Verbindungen sind diejenigen der Formeln I'kk und I'll:

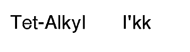

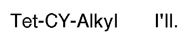

Dabei sind diejenigen Stereoisomeren des trans-Tetralins bevorzugt, in denen die Gruppen R'¹ und -Z-A°-R'² equatorial, die CN-Gruppe axial steht.

Diejenigen der vorstehend genannten Formeln, die eine der Gruppen Dio oder Pyr enthalten, umschließen jeweils die beiden möglichen 2,5- bzw. 1,4-Stellungsisomeren. So umschließt beispielsweise die Teilformel I'gg die 2-Tet-5-R'²⁻1,3-dioxane und die 2-R'²⁻5-Tet-1,3-dioxane, die Teilformel l'ii die 2-Tet-5-R'²⁻pyrimidine und die 2-R'²⁻5-Tet-pyrimidine.

Die Verbindungen der Formel I' können nach an sich bekannten Methoden hergestellt werden, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I' umsetzt.

So können die Verbindungen der Formel I' hergestellt werden, indem man eine Verbindung, die sonst der Formel I' entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I', können aber an Stelle des Tetralinrings einen Naphthalin- oder Dihydronaphthalinring oder einen Dihydronaphthalinonring oder an Stelle eines Cyclohexanrings einen Cyclohexenring oder Cyclohexanonring und/oder an Stelle einer -CH₂CH₂-Gruppe eine -CH = CH-Gruppe oder eine -CH₂-CO-Gruppe enthalten.

Die Reduktion erfolgt unter Bedingungen, bei denen die CN-Gruppe intakt bleibt, zweckmäßig durch katalytische Hydrierung bei Temperaturen zwischen etwa 0 und etwa 100_{°} sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. Pt0₂, PdO), auf einem Träger (z.B. Pd auf Kohle, Calciumcarbonat oder Strontiumcarbonat) oder in feinverteilter Form eingesetzt werden können.

Verbindungen der Formel I' sind weiterhin erhältlich, indem man an ein entsprechendes Dihydronaphthalinderivat (das sonst der Formel I' entspricht, jedoch im Tetralinrest keine CN-Gruppe, dafür aber eine zusätzliche Doppelbindung enthält) Cyanwasserstoff anlagert.

Diese Anlagerung gelingt z.B. in Gegenwart eines inerten Lösungsmittels, z.B. eines halogenierten Kohlenwasserstoffs wie CH₂CI₂ oder CHCl₃, eines Nitrils wie Acetonitril oder eines Amids wie Dimethylformamid (DMF) bei Temperaturen zwischen etwa -10 und + 150 _{°} und Drucken zwischen etwa 1 und 100 bar. Ein Zusatz von Katalysatoren kann günstig sein, z.B. kann eine HCN-Anlagerung durch Zusatz von Palladium-bis-[2,3-0-isopropyliden-2,3-dihydroxy-1,4-bis-(diphenylphosphino)-butan] katalysiert werden.

Ester der Formel I' (Z = -CO-O- oder -O-CO- oder R'² = -O-CO-Alkyl oder -COO-Alkyl) können auch durch Veresterung entsprechender Carbonsäuren der Formeln Tet-COOH, R'²⁻A_{°}-COOH oder Tet-Z-A_{°}-COOH (oder ihrer reaktionsfähigen Derivate) mit Alkoholen bzw. Phenolen der Formeln R'²⁻A°-OH, Tet-OH oder Tet-Z-A°-OH (oder ihren reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, z.B. auch gemischte Anhydride der Formeln Tet-CO-O-CO-CH₃, R'²⁻A°-CO-O-CO-CH₃ und Tet-Z-A°-CO-O-CO-CH_{3,} Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate der Formeln R'²⁻A-OM, Tet-OM und Tet-Z-A_{°}-OM in Betracht, worin M ein Äquivalent eines Metalls, vorzugsweise eines Alkalimetalls wie Na oder K, bedeutet.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen -50 und + 250 _{°} , vorzugsweise zwischen -20 und + 80 ° . Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25 ° und + 20 ° .

Dioxanderivate der Formel I (worin die Gruppe A eine 1,3-Dioxan-2,5-diyl-Gruppe bedeutet) werden zweckmäßig durch Reaktion eines entsprechenden Aldehyds der Formel Tet-Z-CHO bzw. R'²⁻CHO (oder eines seiner reaktionsfähigen Derivate) mit einem entsprechenden 1,3-Diol der Formel R'²⁻CH(CH₂0H)₂ bzw. Tet-Z-CH(CH₂0H)₂ (oder einem seiner reaktionsfähigen Derivate) hergestellt, vorzugsweise in Gegenwart eines inerten Lösungsmittels wie Benzol oder Toluol und/oder eines Katalysators, z.B. einer starken Säure wie Schwefelsäure, Benzol- oder p-Toluolsulfonsäure, bei Temperaturen zwischen etwa 20° und etwa 150°, vorzugsweise zwischen 80 und 120°. Als reaktionsfähige Derivate der Ausgangsstoffe eignen sich in erster Linie Acetale z.B. der Formeln Tet-Z-CH(OR⁶)_{2,}R'²⁻CH(OR⁶)_{2,} R'²⁻CH(CH₂O)₂-CHR' bzw. Tet-Z-CH(CH₂0)₂-CHR⁷, worin R⁶ Alkyl mit 1-4 C-Atomen, zwei Reste R⁶ zusammen auch Alkylen mit 2 oder 3 C-Atomen und R⁷ H, Alkyl mit 1-4 C-Atomen oder Phenyl bedeuten.

Die genannten Aldehyde und 1,3-Diole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester erhältlich.

Zur Herstellung der Nitrile der Formel I' können entsprechende Säureamide, in denen an Stelle der CN-Gruppe eine CONH₂-Gruppe steht, dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie SOCI₂, PCl_{3,} PCl₅, POCI₃, SO₂Cl₂, COCI₂, ferner P₂0s, AlCl₃ (z.B. als Doppelverbindung mit NaCI), aromatische Sulfonsäuren und Sulfonsäurehalogenide. Man kann dabei in Gegenwart oder Abwesenheit eines inerten Lösungsmittels bei Temperaturen zwischen etwa 0° und 150° arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der Nitrile der Formel I' kann man auch entsprechende Säurehalogenide, vorzugsweise die Chloride, mit Sulfamid umsetzen, zweckmäßig in einem inerten Lösungsmittel wie Tetramethylensulfon bei Temperaturen zwischen etwa 80 und 150°, vorzugsweise bei 120°. Nach üblicher Aufarbeitung kann man direkt die Nitrile isolieren.

Zur Herstellung der Nitrile der Formel I' kann auch eine entsprechende Chlor- oder Bromverbindung mit einem Cyanid umgesetzt werden, zweckmäßig mit einem Metallcyanid wie NaCN, KCN oder Cu₂(CN)₂, z.B. in Gegenwart von Pyridin in einem inerten Lösungsmittel wie DMF oder N-Methylpyrrolidon bei Temperaturen zwischen 20 ° und 200 °.

Die bevorzugten Nitrile der Formel I', worin die Nitrilgruppe in 2-Stellung steht, sind bevorzugt auch durch Reaktion von Nitrilen der Formel II' mit Verbindungen der Formel III' erhältlich. Die Nitrile der Formel II' sind beispielsweise aus entsprechenden Halogeniden (entsprechend Formel II', aber CI oder Br an Stelle von CN) und Metallcyaniden oder aus den entsprechenden Ketonen (Tetralonen) nach J. Jiricny, D.M. Orere, C.B. Reese, J.Chem.Soc.Perk. I, 1487 (1980) erhältlich, die Verbindungen der Formel III' - soweit sie nicht bekannt sind - durch Reduktion entsprechender Carbonsäureester zu den entsprechenden Hydroxyverbindungen (III', X' = OH) sowie gegebenenfalls Umsetzung derselben mit anorganischen Halogeniden wie SOCI₂, HBr oder HJ; unter den Verbindungen der Formel III' sind diejenigen bevorzugt, in denen Q Alkyl, R'²⁻Cy- oder R'²-A°-CH₂CH₂- bedeutet. Das Nitril II' wird zweckmäßig zunächst mit einer starken Base wie NaH, NaNH₂, Lithiumdiisopropylamid, -piperidid oder -2,5-diisopropylpiperidid oder K-tert.-Butylat in das entsprechende Carbanion übergeführt, vorzugsweise in einem inerten Lösungsmittel, beispielsweise einem Kohlenwasserstoff wie Toluol, einem Ether wie THF oder Dioxan, einem Amid wie DMF, einem Sulfoxid wie Dimethylsulfoxid oder einem Gemisch derartiger Lösungsmittel. Nach Zugabe von III' (worin X' von OH verschieden ist) hält man zweckmäßig 0,5 bis 16 Stunden bei Temperaturen zwischen -30 und 100°. Eine Umsetzung von II' mit III' (X' = OH) gelingt dagegen zweckmäßig in Gegenwart von Azodicarbonsäureestern/Triphenylphosphin in THF bei Temperaturen zwischen etwa -30 und + 30 _{°} .

In ganz analoger Weise sind Nitrile der Formel I' durch "intramolekulare Alkylierung" erhältlich, indem man ein Nitril, das der Formel I' entspricht, aber an Stelle einer C-C-Bindung zwischen dem die CN-Gruppe tragenden C-Atom und einem diesem benachbarten C-Atom ein zusätzliches H-Atom (an dem die CN-Gruppe tragenden C-Atom) und eine zusätzliche Gruppe X' (an dem diesem benachbarten C-Atom) enthält, unter Abspaltung von HX' cyclisiert.

Ether der Formel I' (worin R'¹ und/oder R'² Alkylketten bedeuten, worin bis zu zwei CH₂-Gruppen durch O-Atome ersetzt sind und/oder worin Z eine -OCH₂- oder eine -CH₂0-Gruppe ist) sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaOH, KOH, Na₂C0₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie Aceton, DMF oder Dimethylsulfoxid oder auch einem Überschuß an wäßriger oder wäßrig-alkoholischer NaOH oder KOH bei Temperaturen zwischen etwa 20 und 100°.

Folgende Synthesewege sind für die Herstellung von Verbindungen der Formel I' besonders bevorzugt.

### Syntheseweg 1:

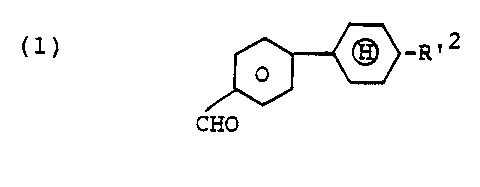

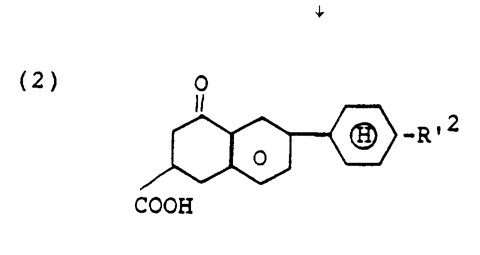

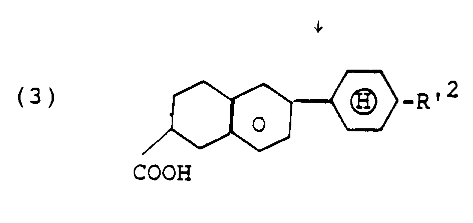

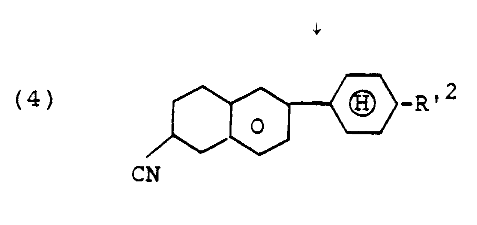

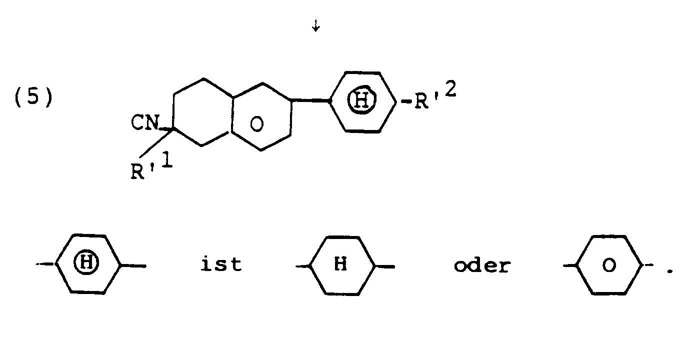

Man erhält Verbindungen (2) aus (1) durch Stobbe-Kondensation mit anschließender Reduktion und durch Ringschluß nach W.S. Johnson et al., Org. Reactions 6, 2 (1954).

Nach Reduktion der Ketogruppe (3) und Überführung der Carbonsäuregruppe in die Nitrilgruppe (4) wird alkyliert und man erhält das Endprodukt (5).

### Syntheseweg 2:

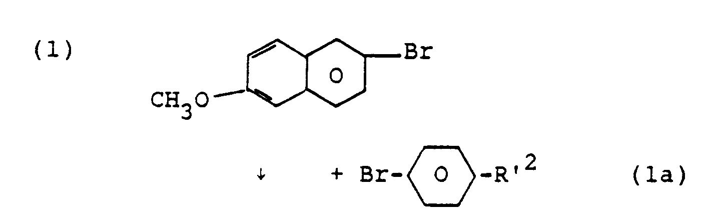

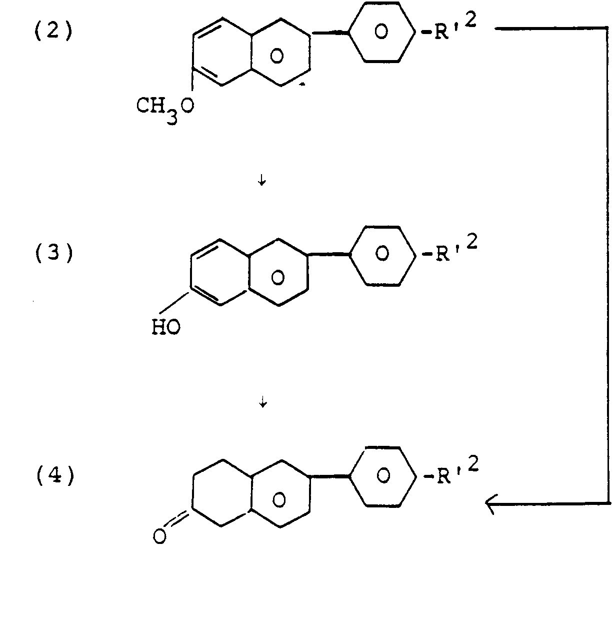

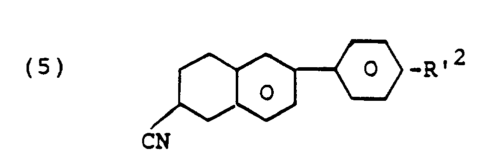

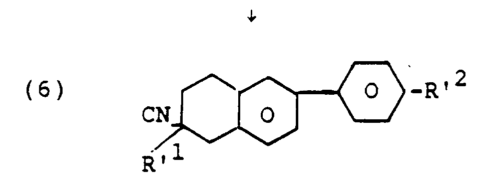

Verbindung (2), erhältlich durch die Umsetzung von (1) mit (1a) nach Negishi et al., J.Org.Chem. 42, 1821 (1977), wird über die Hydroxyverbindung (3) oder analog der Vorschrift in Org.Synth.Coll. Vol. IV 903 direkt zu Verbindung (4) umgesetzt.

Das Keton (4) wird nach D.M. Orere, C.B. Reese, JCS.Chem.Comm. 280, 1977 mit Kaliumcyanid und 2,4,6-Triisopropylbenzolsulfonsäurehydrazid zu (5) umgesetzt. Das Endprodukt (6) wird durch Alkylierung erhalten.
Syntheseweg 3: n = 0,1 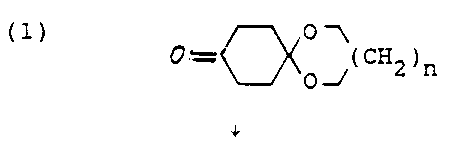 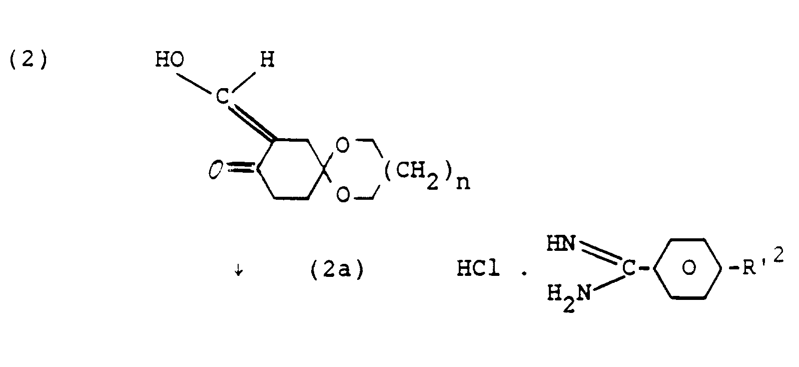 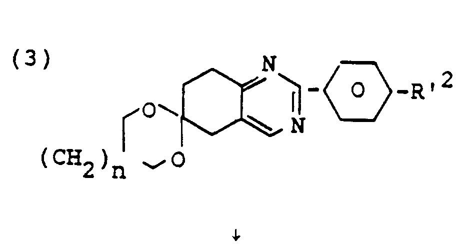 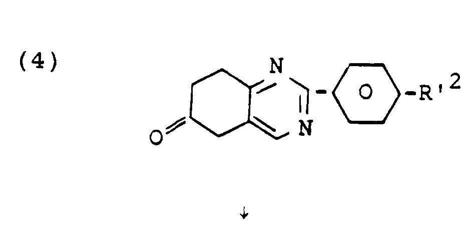 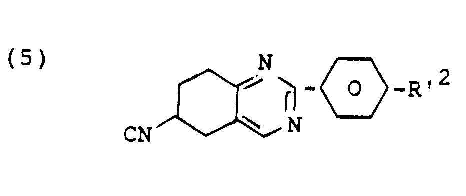 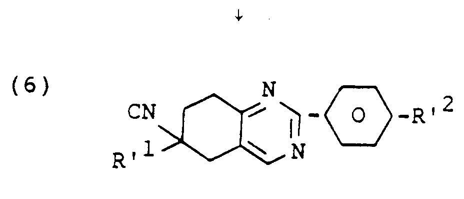 1,4-Cyclohexandionmonoethylenketal oder 1,4-Cyclohexandionmonopropylenketal wird in Gegenwart einer Base mit Ameisensäureethylester zu (2) umgesetzt. Durch Kondensation mit (2a) erhält man daraus Verbindungen (3), anschließend wird durch Spaltung der Spiroverbindung das Keton (4) erhalten. Das Endprodukt (6) ist analog Syntheseweg 2 [vgl. Verbindungen (4)-(6)] zugänglich.

Die erfindungsgemäßen Dielektrika bestehen aus 2 bis 15, vorzugsweise 3 bis 12 Komponenten, darunter mindestens einer Verbindung der Formel I'. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'Bis-cyclohexylbiphenyle, Phenyl- oder Cyclohexylpyrimidine, Phenyl- oder Cyclohexyldioxane, gegebenenfalls halogenisierten Stilbene, Benzylphenylether, Tolane und substituierten Zimtsäuren.

Die erfindungsgemäßen Phasen enthalten vorzugsweise mindestens zwei insbesondere mindestens drei Verbindungen der Formel I. Besonders bevorzugt sind erfindungsgemäße chiral getiltete smektische flüssigkristalline Phasen, deren achirale Basismischung neben Verbindungen der Formel I mindestens eine andere Komponente mit negativer oder betragsmäßig kleiner positiver dielektrischer Anisotropie enthält. Diese weiteren Komponente(n) der chiralen Basismischung können 1 bis 50 %, vorzugsweise 10 bis 25 %, der Basismischung ausmachen. Als weitere Komponenten mit betragsmäßig kleiner positiver oder negativer dielektrischer Anisotropie eignen sich Verbindungen der Teilformeln Va bis Vp: 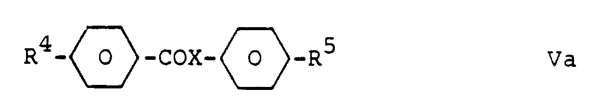 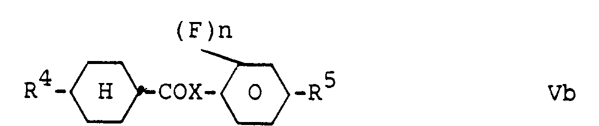 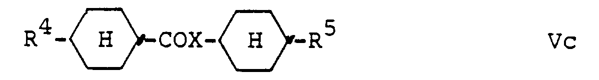 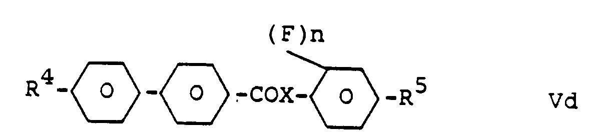 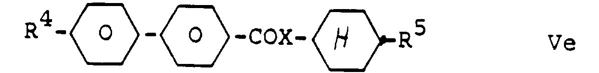 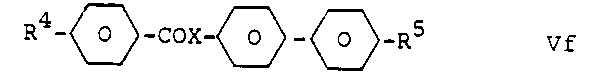 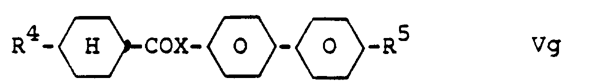 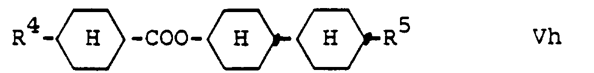 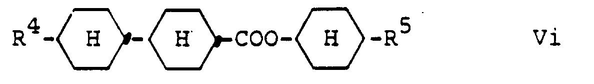 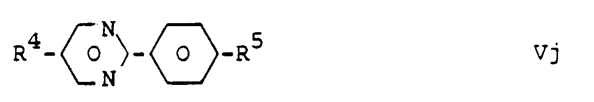 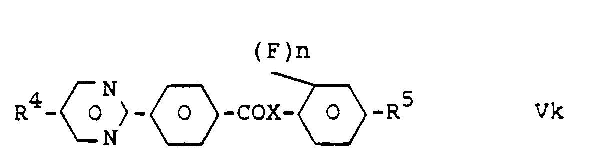 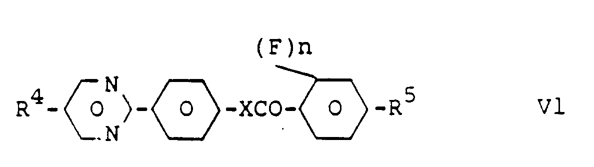 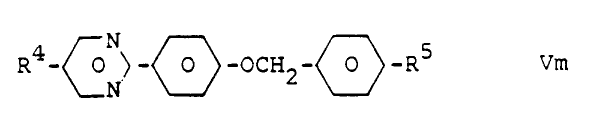 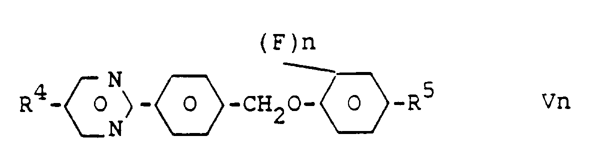 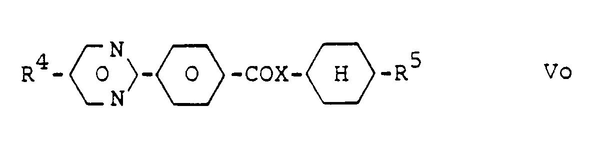 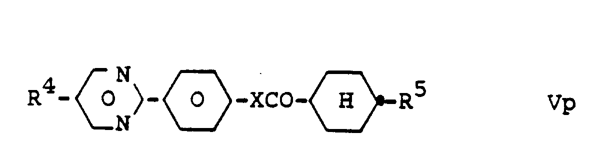 R⁴ und R⁵ sind jeweils vorzugsweise geradkettiges Alkyl, Alkoxy, Alkanoyloxy oder Alkoxycarbonyl mit jeweils 3 bis 12 C-Atomen. X ist vorzugsweise O. n ist 0 oder 1.

Besonders bevorzugt sind die Verbindungen der Teilformeln Va, Vb, Vd und Vf, worin R⁴ und R⁵ jeweils geradkettiges Alkyl oder Alkoxy mit jeweils 5 bis 10 C-Atomen bedeutet.

Die Verbindungen der Teilformeln Vc, Vh und Vi eignen sich als Zusätze zur Schmelzpunkterniedrigung und werden normalerweise den Basismischungen mit nicht mehr als 5 %, vorzugsweise 1 bis 3 %, zugesetzt. R⁴ und R⁵ bedeuten in den Verbindungen der Teilformeln Vc, Vh und Vi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist diejenige der Formel 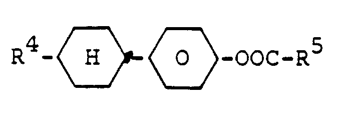 worin R⁴ und R⁵ die für Vc, Vh und Vi angegebene bevorzugte Bedeutung haben.

Als weitere Komponenten mit negativer dielektrischer Anisotropie eignen sich weiterhin Verbindungen enthaltend das Strukturelement B oder C. 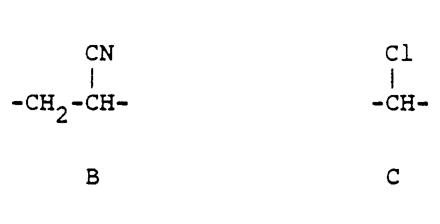 Bevorzugte Verbindungen dieser Art entsprechen den Formeln Vlb und Vlc: 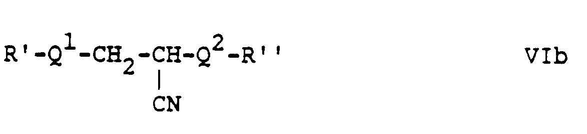
R¹-Q³-Q⁴-R"' Vlc

R' und R" bedeuten jeweils vorzugsweise geradkettige Alkyl- oder Alkoxy-Gruppen mit jeweils 2 bis 10 C-Atomen. Q¹ und Q² bedeuten jeweils 1,4-Phenylen, trans-1,4-Cyclohexylen, 4,4'-Biphenylyl, 4-(trans-4-Cyclohexyl)-phenyl, trans,trans-4,4'-Bicyclohexyl oder eine der Gruppen Q¹ und Q² auch eine Einfachbindung.

Q³ und Q⁴ bedeuten jeweils 1,4-Phenylen, 4,4'-Biphenylyl oder trans-1,4-Cyclohexylen. Eine der Gruppen Q³ und Q⁴ kann auch 1,4-Phenylen bedeuten, worin mindestens eine CH-Gruppe durch N ersetzt ist. R'" ist ein optisch aktiver Rest mit einem asymmetrischen Kohlenstoffatom der Struktur 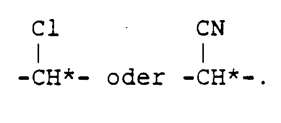 Besonders bevorzugte Verbindungen der Formel Vlc sind diejenigen der Formel Vlc': 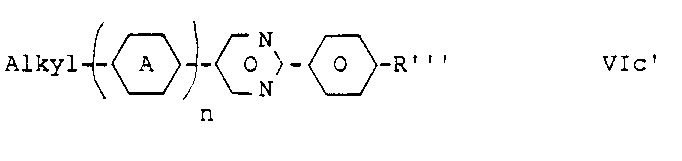 worin A 1,4-Phenylen oder trans-1,4-Cyclohexylen und n 0 oder 1 bedeutet.

Die Herstellung der erfindungsgemäßen Phasen erfolgt in an sich üblicher Weise. In der Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Die folgenden Beispiele sollen die Erfindung erläutern. Vor- und nachstehend bedeuten Prozentangaben Gewichtsprozent; alle Temperaturen sind in Grad Celsius angegeben. Es bedeuten ferner: K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

### Beispiel 1

Eine flüssigkristalline Phase bestehend aus
25 % 4-(5-Heptylpyrimidin-2-yl)-phenyl-(p-pentylbenzyl)-ether,
33 % 4-(5-Heptylpyrimidin-2-yl)-phenyl-(p-hexylbenzyl)-ether,
12 % 4-(5-Nonylpyrimidin-2-yl)-phenyl-(p-pentylbenzyl)-ether,
3 % 4-(5-Nonylpyrimidin-2-yl)-phenyl-(p-hexylbenzyl)-ether,
21 % 2-p-Nonyloxyphenyl-5-nonylpyrimidin,
3 % 2,3-Dicyan-1,4-bis-(trans-4-propylcyclohexylcarboxy)-benzol und
3 % 2,3-Dicyan-1,4-bis-(trans-4-pentylcyclohexylcarboxy)-benzol

hat K/Sc 7°, Sc/N 64°, N/I 95,5 und eine dielektrische Anisotropie Δ_{∈} von -0,5.

### Beispiel 2

Eine flüssigkristalline Phase bestehend aus
20 % 4-(5-Heptylpyrimidin-2-yl)-phenyl-(p-pentylbenzyl)-ether,
27 % 4-(5-Heptylpyrimidin-2-yl)-phenyl-(p-hexylbenzyl)-ether,
8 % 4-(5-Nonylpyrimidin-2-yl)-phenyl-(p-pentylbenzyl)-ether,
4 % 4-(5-Nonylpyrimidin-2-yl)-phenyl-(p-propylbenzyl)-ether,
24 % 2-p-Nonyloxyphenyl-5-nonylpyrimidin,
4 % 2,3-Dicyan-1,4-bis-(trans-4-propylcyclohexylcarboxy)-benzol
7 % 2,3-Dicyan-1,4-bis-(trans-4-propoxycyclohexylcarboxy)-benzol und
6 % 2,3-Dicyan-1-(trans-4-butoxycyclohexylcarboxy)-4-(trans-4-hexoxycyclohexylcarboxy)-benzol

hat K/Sc 12°, Sc/N 73 °, N/I 104 ° und △_{∈} von -1,0.

### Beispiel 3

Eine flüssigkristalline Phase bestehend aus
20 % 4-(5-Heptylpyrimidin-2-yl)-phenyl-(p-pentylbenzyl)-ether,
20 % 4-(5-Heptylpyrimidin-2-yl)-phenyl-(p-hexylbenzyl)-ether,
4 % 4-(5-Nonylpyrimidin-2-yl)-phenyl-(p-pentylbenzyl)-ether,
4 % 4-(5-Nonylpyrimidin-2-yl)-phenyl-(p-propylbenzyl)-ether,
15 % 4-(5-Octylpyrimidin-2-yl)-phenyl-(p-pentylbenzyl)-ether,
6 % 4-(5-Octylpyrimidin-2-yl)-phenyl-(p-heptylbenzyl)-ether,
18 % 2-p-Nonyloxyphenyl-5-nonylpyrimidin,
4 % 2,3-Dicyan-1,4-bis-(trans-4-butoxycyclohexylcarboxy)-benzol
6 % 2,3-Dicyan-1,4-bis-(trans-4-hexoxycyclohexylcarboxy)-benzol und
3 % 2,3-Dicyan-1,4-bis-(2-trans-4-pentylcyclohexylethyl)-benzol

hat K/Sc 10°, Sc/N 66 °, N/I 91,5 ° und △_{∈} von -1,0.

### Beispiel 4

Eine flüssigkristalline Phase bestehend aus
25 % 4-(5-Nonylpyrimidin-2-yl)-phenyl-(p-hexylbenzyl)-ether,
30 % 4-(5-Nonylpyrimidin-2-yl)-phenyl-(p-propylbenzyl)-ether,
10 % 4-(5-Hexylpyrimidin-2-yl)-phenyl-(p-octylbenzyl)-ether,
10 % 4-(5-Octylpyrimidin-2-yl)-phenyl-(p-pentylbenzyl)-ether,
10 % 2-p-Nonyloxyphenyl-5-nonylpyrimidin,
5 % 2-p-Hexyloxyphenyl-5-nonylpyrimidin,
8 % 2,3-Dicyan-1,4-bis-(2-trans-4-hexylcyclohexylethyl)-benzol und
2 % 2,3-Dicyan-1-(2-trans-4-butylcyclohexylethyl)-4-(trans-4-pentylcyclohexylmethoxy)-benzol

hat K/Sc 11°, Sc/N 70 °, N/I 98 ° und △_{∈} von -0,5.

### Beispiel 5

Eine flüssigkristalline Phase bestehend aus
18 % 4-(5-Nonylpyrimidin-2-yl)-phenyl-(p-heptylbenzyl)-ether,
10 % 4-(5-Nonylpyrimidin-2-yl)-phenyl-(p-propylbenzyl)-ether,
30 % 4-(5-Heptylpyrimidin-2-yl)-phenyl-(p-pentylbenzyl)-ether,
22 % 4-(5-Hexylpyrimidin-2-yl)-phenyl-(p-pentylbenzyl)-ether,
10 % 2-p-Nonyloxyphenyl-5-nonylpyrimidin,
8 % 2,3-Dicyan-1-(trans-4-pentylcyclohexylcarboxy)-4-(2-trans-4-pentylcyclohexylethyl)-benzol und
2 % 2,3-Dicyan-1-(trans-4-heptylcyclohexylcarboxy)-4-(2-trans-4-heptylcyclohexylethyl)-benzol

hat K/Sc 6°, Sc/N 90° , N/I 97 ° und △_{∈} von -1,0.

### Beispiel 6

Eine flüssigkristalline Phase bestehend aus
12 % 4-(5-Nonylpyrimidin-2-yl)-phenyl-(p-heptylbenzyl)-ether,
12 % 4-(5-Nonylpyrimidin-2-yl)-phenyl-(p-propylbenzyl)-ether,
9 % 4-(5-Nonylpyrimidin-2-yl)-phenyl-(p-pentylbenzyl)-ether,
18 % 4-(5-Heptylpyrimidin-2-yl)-phenyl-(p-pentylbenzyl)-ether,
11 % 2-p-Nonyloxyphenyl-5-nonylpyrimidin,
8 % trans-4-Octylcyclohexancarbonsäure-(p-nonyloxyphenylester),
8 % trans-4-Heptylcyclohexancarbonsäure-(p-hexyloxy phenylester),
10 % p-Octyloxybenzoesäure-(p-heptylphenylester),
4 % p-Pentylbenzoesäure-(4'-hexylbiphenyl-4-yl-ester),
4 % 2-Cyan-1,4-bis-(trans-4-pentylcyclohexylcarboxy)-benzol und
4 % 2-Cyan-1,4-bis-(trans-4-heptylcyclohexylcarboxy)-benzol

hat K/Sc 0 °, Sc/N 66,5 °, N/I 79 ° und △_{∈} von -2,3.

### Beispiel 7

Eine flüssigkristalline Phase bestehend aus
18 % 4-(5-Nonylpyrimidin-2-yl)-phenyl-(p-heptylbenzyl)-ether,
23 % 4-(5-Nonylpyrimidin-2-yl)-phenyl-(p-pentylbenzyl)-ether,
15 % 4-(5-Heptylpyrimidin-2-yl)-phenyl-(p-heptylbenzyl)-ether,
14 % 4-(5-Heptylpyrimidin-2-yl)-phenyl-(p-pentylbenzyl)-ether,
10 % 2-p-Nonyloxyphenyl-5-nonylpyrimidin,
5 % p-Octyloxybenzoesäure-(p-heptylphenylester),
10 % 2,3-Dicyan-1-(trans-4-butylcyclohexyl)-4-trans-4-hexylcyclohexyl)-benzol und
5 % p-Pentylbenzoesäure-(4'-hexylbiphenyl-4-yl-ester)

hat K/Sc 6°, Sc/N 68,5 °, N/I 81 _{°} und △_{∈} von -1,3.

### Beispiel 8

Eine flüssigkristalline Phase bestehend aus
17 % 4-(5-Heptylpyrimidin-2-yl)-phenyl-(p-pentylbenzyl)-ether,
26 % 4-(5-Heptylpyrimidin-2-yl)-phenyl-(p-hexylbenzyl)-ether,
13 % 4-(5-Nonylpyrimidin-2-yl)-phenyl-(p-pentylbenzyl)-ether,
4 % 4-(5-Nonylpyrimidin-2-yl)-phenyl-(p-hexylbenzyl)-ether,
30 % r-1-Cyan-1-octyl-cis-4-(4'-hexylbiphenyl-4-yl)-cyclohexan,
5 % r-1-Cyan-1-pentyl-cis-4-(4'-pentylbiphenyl-4-yl)-cyclohexanund
5 % r-1-Cyan-1-heptyl-cis-4-(4'-pentylbiphenyl-4-yl)-cyclohexan,

hat K/Sc 13 °, Sc/N 70 °, N/I 88,5 ° und △_{∈} von -2,6.

### Beispiel 9

Eine flüssigkristalline Phase bestehend aus
19 % r-1-Cyan-1-pentyl-cis-4-(4'-butylbiphenyl-4-yl)-cyclohexan,
28 % r-1-Cyan-1-heptyl-cis-4-(4'-hexylbiphenyl-4-yl)-cyclohexan,
15 % r-1-Cyan-1-butyl-cis-4-(4'-octylbiphenyl-4-yl)-cyclohexan,
10 % p-Pentylbenzoesäure-(4'-hexylbiphenyl-4-yl-ester),
8 % trans-4-Octylcyclohexancarbonsäure-(p-nonyloxyphenylester),
14 % trans-4-Heptylcyclohexancarbonsäure-(p-hexyloxyphenylester) und
6 % trans-4-Heptylcyclohexancarbonsäure-(p-octyloxyphenylester)

hat K/Sc 13°, Sc/N 74°, N/I 90 ° und △_{∈} von -4,7.

### Beispiel 10

Eine flüssigkristalline Phase bestehend aus
23 % r-1-Cyan-1-pentyl-cis-4-(4'-pentylbiphenyl-4-yl)-cyclohexan,
20 % r-1-Cyan-1-heptyl-cis-4-(4'-hexylbiphenyl-4-yl)-cyclohexan,
18 % r-1-Cyan-1-butyl-cis-4-(4'-octylbiphenyl-4-yl)-cyclohexan,
13 % r-1-Cyan-1-pentyl-cis-4-(4'-butylbiphenyl-4-yl)-cyclohexan,
10 % trans-4-Octylcyclohexancarbonsäure-(p-nonyloxyphenylester),
10 % trans-4-Heptylcyclohexancarbonsäure-(p-hexyloxyphenylester),
3 % 2,3-Dicyan-1,4-bis-(trans-4-pentylcyclohexylcarboxy)-benzol und
3 % 2,3-Dicyan-1,4-bis-(trans-4-propylcyclohexylcarboxy)-benzol

hat K/Sc 15 °, Sc/N 71 °, N/I 94,5 ° und △_{∈} von -5,3.

### Beispiel 11

Man stellt eine flüssigkristalline Phase her, bestehend aus
4 % 2-p-Heptyloxyphenyl-5-heptylpyrimidin,
30 % 2-p-Undecyloxyphenyl-5-hexylpyrimidin,
30 % 2-p-Nonyloxyphenyl-5-nonylpyrimidin,
12 % r-1-Cyan-cis-4-[p-(5-heptylpyrimidinyl-2)-phenyl]-cyclohexan-1-carbonsäurepentylester,
14 % r-1-Cyan-cis-4-[p-(5-octylpyrimidinyl-2)-phenyl]-cyclohexan-1-carbonsäurebutylester und
10 % p-(5-Hexylpyrimidinyl-2)-phenyl-2-chlorpropionat (optisch aktiv).

### Beispiel 12

Man stellt eine flüssigkristalline Phase her, bestehend aus
20 % 4-(5-Hexylpyrimidin-2-yl)-phenyl-(p-pentylbenzyl)-ether,
20 % 4-(5-Heptylpyrimidin-2-yl)-phenyl-(p-hexylbenzyl)-ether,
20 % 4-(5-Nonylpyrimidin-2-yl)-phenyl-(p-propylbenzyl)-ether,
5 % 4-(5-Nonylpyrimidin-2-yl)-phenyl-(p-cyanbenzyl)-ether,
10 % r-1-Cyan-cis-4-[p-(5-heptylpyrimidinyl-2)-phenyl]-cyclohexan-1-carbonsäurebutylester,
15 % r-1-Cyan-cis-4-[p-(5-octylpyrimidinyl-2)-phenyl]-cyclohexan-1-carbonsäurepentylester und
10 % p-(5-Hexylpyrimidinyl-2)-phenyl-2-chlorpropionat (optisch aktiv).

### Beispiel 13

Eine flüssigkristalline Phase bestehend aus
95 % r-1-Cyan-1-butoxy-cis-4-(4'-octylbiphenyl-4-yl)-cyclohexanund
5 % r-1-Cyan-1-(1-hydroxy-3-methylcyclohexyl)-trans-4-pentylcyclohexyl)-cyclohexan (optisch aktiv)

hat I 140° Ch 102° S; 93 ° Sc* 44° K und eine spontane Polarisation P von 2,5 nC/cm².

### Beispiel 14

Eine flüssigkristalline Phase bestehend aus
50 % r-1-Cyan-1-butoxy-cis-4-(4'-octylbiphenyl-4-yl)-cyclohexanund
50 % p-2-Methylheptoxy-benzoesäure-(4'-octyloxybiphenyl-4-yl-ester) (optisch aktiv)

hat 1 140 ° Ch 112 ° Sc* 55° K und P = 9,8 nC/cm².

### Beispiel 15

Eine flüssigkristalline Phase bestehend aus
42 % r-1-Cyan-1-butoxy-cis-4-(4'-octylbiphenyl-4-yl)-cyclohexan,
43 % r-1-Cyan-1-pentyl-cis-4-(4'-octylbiphenyl-4-yl)-cyclohexan,
10 % r-1-Cyan-1-pentyl-cis-4-(4'-butylbiphenyl-4-yl)-cyclohexanund
5 % p-(5-Hexylpyrimidinyl-2)-phenyl-2-chlorpropionat (optisch aktiv)

hat I 125 ° Ch 92 ° S_{A}* 70° Sc* < RT K und P = 3,8 nC/cm².

### Beispiel 16

Eine flüssigkristalline Phase bestehend aus
30 % r-1-Cyan-1-octyl-cis-4-(4'-pentylbiphenyl-4-yl)-cyclohexan,
34 % r-1-Cyan-1-pentyl-cis-4-(4'-octylbiphenyl-4-yl)-cyclohexan,
18 % r-1-Cyan-1-butoxy-cis-4-(4'-octylbiphenyl-4-yl)-cyclohexan,
8 % r-1-Cyan-1-pentyl-cis-4-(4'-butylbiphenyl-4-yl)-cyclohexan und
10 % p-(5-Hexylpyrimidinyl-2)-phenyl-2-chlorpropionat (optisch aktiv)

hat I 109 ° Ch 89° SA* 53° Sc* < RT K und P = 1,8 nC/cm².

### Beispiel 17

Eine flüssigkristalline Phase bestehend aus
13 % 4-(5-Heptylpyrimidin-2-yl)-phenyl-(p-pentylbenzyl)-ether,
16 % 4-(5-Heptylpyrimidin-2-yl)-phenyl-(p-hexylbenzyl)-ether,
5 % 4-(5-Nonylpyrimidin-2-yl)-phenyl-(p-pentylbenzyl)-ether,
12 % 2-p-Nonyloxyphenyl-5-nonylpyrimidin,
4 % r-1-Cyan-1-pentyl-cis-4-(4'-butylbiphenyl-4-yl)-cyclohexan,
15 % r-1-Cyan-1-octyl-cis-4-(4'-pentylbiphenyl-4-yl)-cyclohexan,
16 % r-1-Cyan-1-pentyl-cis-4-(4'-octylbiphenyl-4-yl)-cyclohexan,
9 % r-1-Cyan-1-butoxy-cis-4-(4'-octylbiphenyl-4-yl)-cyclohexan und
10 % p-(5-Hexylpyrimidinyl-2)-phenyl-2-chlorpropionat (optisch aktiv)

hat I 99° Ch 83 ° Sc* 35 ° K und P = 5,5 nC/cm².

### Beispiel 18

Ein ferroelektrisches smektisches Material bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
28 % r-1-Cyan-cis-4-(4'-butyloxybiphenyl-4-yl)-1-octylcyclohexan,
14 % r-1-Cyan-cis-4-(4'-hexyl-biphenyl)-4-yl)-1-heptylcyclohexan,
6 % r-1-Cyan-1-(trans-4-pentylcyclohexyl)-cis-4-(trans-4-pentylcyclohexyl)-cyclohexan und
10 % p-(5-Hexylpyrimidin-2-yl)phenyl-2-chlorpropionat (optisch aktiv)

hat einen Klärpunkt von 92 ° und eine dielektrische Anisotropie △_{∈}-2,6.

### Beispiel 19

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
8 % 2-p-Hexyloxyphenyl-5-nonylpyrimidin,
25 % 2-p-Nonyloxyphenyl-5-nonylpyrimidin,
30 % r-1-Cyan-cis-4-(4'-octyloxybiphenyl-4-yl)-1-octylcyclohexan,
15 % r-1-Cyan-cis-4-(4'-nonanoyloxybiphenyl-4-yl)-1-butylcyclohexan und
10 % r-1-Cyan-cis-4-[p-(5-nonylpyrimidin-2-yl)-phenyl]-1-(2-methylbutyl)-cyclohexan (optisch aktiv)

hat K -8 ° S^{X}_{C}65 _{°} SÄ und P = 8 nC/cm² bei 20 °.

### Beispiel 20

Man stellt eine flüssigkristalline Phase her bestehend aus
3 % 2-p-Hexyloxyphenly-5-heptylpyrimidin,
3 % 2-p-Heptyloxyphenly-5-heptylpyrimidin,
3 % 2-p-Octyloxyphenly-5-heptylpyrimidin,
3 % 2-p-Nonyloxyphenly-5-heptylpyrimidin,
8 % 2-p-Hexyloxyphenly-5-nonylpyrimidin,
25 % 2-p-Nonyloxyphenly-5-nonylpyrimidin,
30 % r-1-Cyan-cis-4-(4'-nonanoyloxybiphenyl-4-yl)-1-octylcyclohexan,
10 % r-1-Cyan-cis-4-(p-octylphenyl)-1-octylcyclohexan,
5 % r-1-Cyan-cis-4-(p-oxtylphenyl)-1-(2-methylbutyl)-cyclohexan (optisch aktiv) und
10 % r-1-Cyan-cis-4-[p-(p-heptylphenoxycarbonyl)-phenyl]-1-(2-methylbutyl)-cyclohexan (optisch aktiv).

### Beispiel 21

Man stellt eine flüssigkristalline Phase her bestehend aus
3 % 2-p-Hexyloxyphenly-5-heptylpyrimidin,
3 % 2-p-Heptyloxyphenly-5-heptylpyrimidin,
3 % 2-p-Octyloxyphenly-5-heptylpyrimidin,
3 % 2-p-Nonyloxyphenly-5-heptylpyrimidin,
8 % 2-p-Hexyloxyphenly-5-nonylpyrimidin,
25 % 2-p-Nonyloxyphenly-5-nonylpyrimidin,
30 % r-1-Cyan-cis-4-(4'-nonanoyloxybiphenyl-4-yl)-1-octylcyclohexan,
10 % r-1-Cyan-cis-4-(p-octyloxyphenyl)-1-octylcyclohexan,
5 % r-1-Cyan-cis-4-(p-octyloxyphenyl)-1-(2-methylbutyl)-cyclohexan (optisch aktiv) und
10 % r-1-Cyan-cis-4-[p-(p-heptylphenoxycarbonyl)-phenyl]-1-(2-methylbutyl)-cyclohexan (optisch aktiv).

### Beispiel 22

Man stellt eine flüssigkristalline Phase her bestehend aus
3 % 2-p-Hexyloxyphenly-5-heptylpyrimidin,
3 % 2-p-Heptyloxyphenly-5-heptylpyrimidin,
3 % 2-p-Octyloxyphenly-5-heptylpyrimidin,
3 % 2-p-Nonyloxyphenly-5-heptylpyrimidin,
8 % 2-p-Hexyloxyphenly-5-nonylpyrimidin,
25 % 2-p-Nonyloxyphenly-5-nonylpyrimidin,
30 % r-1-Cyan-cis-4-(4'-nonanoyloxybiphenyl-4-yl)-1-octylcyclohexan,
10 % r-1-Cyan-cis-4-(p-octanoyloxyphenyl)-1-octylcyclohexan,
5 % r-1-Cyan-cis-4-(p-octanoyloxyphenyl)-1-(2-methylbutyl)-cyclohexan (optisch aktiv) und
10 % r-1-Cyan-cis-4-[p-(p-heptylphenoxycarbonyl)-phenyl]-1-(2-methylbutyl)-cyclohexan (optisch aktiv).

### Beispiel 23

Man stellt eine flüssigkristalline Phase her bestehend aus
3 % 2-p-Hexyloxyphenly-5-heptylpyrimidin,
3 % 2-p-Heptyloxyphenly-5-heptylpyrimidin,
3 % 2-p-Octyloxyphenly-5-heptylpyrimidin,
3 % 2-p-Nonyloxyphenly-5-heptylpyrimidin,
8 % 2-p-Hexyloxyphenly-5-nonylpyrimidin,
25 % 2-p-Nonyloxyphenly-5-nonylpyrimidin,
30 % r-1-Cyan-cis-4-(4'-nonanoyloxybiphenyl-4-yl)-1-octylcyclohexan,
10 % r-1-Cyan-cis-4-(p-(2-methylbutoxy)-phenyl)-1-octylcyclohexan,
5 % r-1-Cyan-cis-4-(p-(2-methylbutoxy)-phenyl)-1-(2-methylbutyl)-cyclohexan (optisch aktiv) und
10 % r-1-Cyan-cis-4-[p-(p-heptylphenoxycarbonyl)-phenyl]-1-(2-methylbutyl)-cyclohexan (optisch aktiv).

### Beispiel 24

Man stellt eine flüssigkristalline Phase her bestehend aus
3 % 2-p-Hexyloxyphenly-5-heptylpyrimidin,
3 % 2-p-Heptyloxyphenly-5-heptylpyrimidin,
3 % 2-p-Octyloxyphenly-5-heptylpyrimidin,
3 % 2-p-Nonyloxyphenly-5-heptylpyrimidin,
8 % 2-p-Hexyloxyphenly-5-nonylpyrimidin,
25 % 2-p-Nonyloxyphenly-5-nonylpyrimidin,
20 % r-1-Cyan-1-(trans-4-octylcyclohexyl-ethyl)-cis-4-(p-octyloxyphenyl)-cyclohexan,
10 % r-1-Cyan-1-(trans-4-octylcyclohexyl-ethyl)-cis-4-(p-heptyloxyphenyl)-cyclohexan,
10 % r-1-Cyan-1-(trans-4-octylcyclohexyl-ethyl)-cis-4-(p-octylphenyl)-cyclohexan,
5 % r-1-Cyan-1-(trans-4-octylcyclohexyl-ethyl)-cis-4-(p-heptylphenyl)-cyclohexan,
10 % r-1-Cyan-cis-4-[p-(5-octylpyrimidin-2-yl)-phenyl]-1-(2-methylbutyl)-cyclohexan (optisch aktiv).

### Beispiel 25

Eine flüssigkristalline Phase bestehend aus
3 % 2-p-Hexyloxyphenly-5-heptylpyrimidin,
3 % 2-p-Heptyloxyphenly-5-heptylpyrimidin,
3 % 2-p-Octyloxyphenly-5-heptylpyrimidin,
3 % 2-p-Nonyloxyphenly-5-heptylpyrimidin,
8 % 2-p-Hexyloxyphenly-5-nonylpyrimidin,
25 % 2-p-Nonyloxyphenly-5-nonylpyrimidin,
30 % r-1-Cyan-cis-4-[p-(p-octyloxybenzoyloxy)-phenyl]-1-octylcyclohexan,
15 % r-1-Cyan-cis-4-[p-(p-octyloxybenzoyloxy)-phenyl]-1-pentylcyclohexan,
10 % r-1-Cyan-cis-4-[p-(5-nonyloxypyrimidin-2-yl)-phenyl]-1-(2-methylbutyl)-cyclohexan (optisch aktiv).

hat K-10_{°} Sc 61 ° S^{X}_{A}und P = 10 nC/cm² bei 20°.

### Beispiel 26

Man stellt eine flüssigkristalline Phase her bestehend aus
3 % 2-p-Hexyloxyphenly-5-heptylpyrimidin,
3 % 2-p-Heptyloxyphenly-5-heptylpyrimidin,
3 % 2-p-Octyloxyphenly-5-heptylpyrimidin,
3 % 2-p-Nonyloxyphenly-5-heptylpyrimidin,
8 % 2-p-Hexyloxyphenly-5-nonylpyrimidin,
25 % 2-p-Nonyloxyphenly-5-nonylpyrimidin,
15 % 1-Cyan-1-trans-4-(p-heptyloxyphenyl)-cyclohexyl-2-(trans-4-octylcyclohexyl)-ethan,
15 % 1-Cyan-1-trans-4-(p-octyloxyphenyl)-cyclohexyl-2-(trans-4-octylcyclohexyl)-ethan,
10 % 1-Cyan-1-trans-4-(p-octylphenyl)-cyclohexyl-2-(trans-4-butylcyclohexyl)-ethan,
5 % 1-Cyan-1-trans-4-(p-heptylphenyl)-cyclohexyl-2-(trans-4-butylcyclohexyl)-ethan und
10 % r-1-Cyan-cis-4-[p-(5-octylpyrimidin-2-yl)-phenyl]-1-(2-methylbutyl)-cyclohexan.

### Beispiel 27

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
8 % 2-p-Hexyloxyphenyl-5-nonylpyrimidin,
25 % 2-p-Nonyloxyphenyl-5-nonylpyrimidin,
25 % r-1-Cyan-cis-4-(4'-heptyloxy-3'-fluorbiphenyl-4-yl)-1-nonylcyclohexan,
10 % r-1-Cyan-cis-4-(4'-octyloxy-3'-fluorbiphenyl-4-yl)-1-butylcyclohexan,
5 % r-1-Cyan-cis-4-[trans-4-(p-octylphenyl)-cyclohexyl]-1-(2-methylbutyl)-cyclohexan (optisch aktiv)
5 % r-1-Cyan-cis-4-[trans-4-(p-octyloxyphenyl)-cyclohexyl]-1-(2-methylbutyl)-cyclohexan (optisch aktiv) und
10 % r-1-Cyan-cis-4-[p-(p-octyloxyphenoxycarbonyl)-phenyl]-1-heptylcyclohexan.

### Beispiel 29

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
8 % 2-p-Hexyloxyphenyl-5-nonylpyrimidin,
25 % 2-p-Nonyloxyphenyl-5-nonylpyrimidin,
10 % r-1-Cyan-cis-4-(p-octyloxyphenyl)-1-octylcyclohexan,
10 % r-1-Cyan-cis-4-(4'-octyloxycarbonylbiphenyl-4-yl)-1-octylcyclohexan,
5 % r-1-Cyan-cis-4-(p-heptyloxybenzoyloxy)-1-nonylcyclohexan,
10 % r-1-Cyan-cis-4-[p-(p-octylphenyl)-benzoyloxy]-1-butylcyclohexan,
5 % r-1-Cyan-cis-4-[p-(p-octyloxyphenyl)-benzoyloxy]-1-butylcyclohexan und
15 % r-1-Cyan-cis-4-[p-(p-nonylphenyl)-benzoyloxy]-1-(2-methylbutyl)-cyclohexan (optisch aktiv).

### Beispiel 30

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
8 % 2-p-Hexyloxyphenyl-5-nonylpyrimidin,
25 % 2-p-Nonyloxyphenyl-5-nonylpyrimidin,
10 % r-1-Cyan-cis-4-(p-octyloxycarbonyloxyphenyl)-1-octylcyclohexan,
10 % r-1-Cyan-cis-4-(4'-octyloxycarbonylbiphenyl-4-yl)-1-octylcyclohexan,
5 % r-1-Cyan-cis-4-(p-heptyloxybenzoyloxy)-1-nonylcyclohexan,
10 % r-1-Cyan-cis-4-[p-(p-octylphenyl)-benzoyloxy]-1-butylcyclohexan,
5 % r-1-Cyan-cis-4-[p-(p-octyloxyphenyl)-benzoyloxy]-1-butylcyclohexan und
15 % r-1-Cyan-cis-4-[p-(p-nonylphenyl)-benzoyloxy]-1-(2-methylbutyl)-cyclohexan (optisch aktiv).

### Beispiel 31

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
8 % 2-p-Hexyloxyphenyl-5-nonylpyrimidin,
25 % 2-p-Nonyloxyphenyl-5-nonylpyrimidin,
10 % r-1-Cyan-cis-4-(p-octyloxyphenyl)-1-octylcyclohexan,
10 % r-1-Cyan-cis-4-(4'-octyloxycarbonylbiphenyl-4-yl)-1-octylcyclohexan,
5 % r-1-Cyan-cis-4-(p-heptyloxybenzoyloxy)-1-nonylcyclohexan,
10 % r-1-Cyan-cis-4-[p-(p-octylphenyl)-benzoyloxy]-1-butylcyclohexan,
5 % r-1-Cyan-cis-4-[p-(p-octyloxyphenyl)-benzoyloxy]-1-butylcyclohexan und
15 % r-1-Cyan-cis-4-[p-(5-nonylpyrimidin-2-yl)-phenoxycarbonyl]-1-(2-methylbutyl)-cyclohexan (optisch aktiv).

### Beispiel 32

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
8 % 2-p-Hexyloxyphenyl-5-nonylpyrimidin,
25 % 2-p-Nonyloxyphenyl-5-nonylpyrimidin,
20 % r-1-Cyan-cis-4-octyl-1-[trans-4-(p-octyloxyphenyl)-cyclohexyl-ethyl]-cyclohexan,
10 % r-1-Cyan-cis-4-butyl-1-[trans-4-(p-octyloxyphenyl)-cyclohexyl-ethyl]-cyclohexan,
10 % r-1-Cyan-cis-4-nonyl-1-[trans-4-(p-heptylphenyl)-cyclohexyl-ethyl]-cyclohexan,
5 % r-1-Cyan-cis-4-(2-methylbutyl)-1-[trans-4-(p-octylphenyl)-cyclohexyl-ethyl]-cyclohexan (optisch aktiv),
5 % r-1-Cyan-cis-4-(p-octylphenoxycarbonyl)-1-nonylcyclohexan und
5 % r-1-Cyan-cis-4-[trans-4-(p-octylphenyl)-cyclohexyl]-1-(2-methylbutyl)-cyclohexan (optisch aktiv).

### Beispiel 33

Eine flüssigkristalline Phase bestehend aus
2 % 2-p-Hexyloxyphenyl-5-heptylpyrimidin,
2 % 2-p-Heptyloxyphenyl-5-heptylpyrimidin,
2 % 2-p-Octyloxyphenyl-5-heptylpyrimidin,
2 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
30 % r-1-Cyan-cis-4-(4'-octyloxybiphenyl-4-yl)-1-octylcyclohexan,
8 % r-1-Cyan-cis-4-(4'-butylbiphenyl-4-yl)-1-octylcyclohexan,
14 % r-1-Cyan-cis-4-(4'-heptyloxybiphenyl-4-yl)-1-(2-methylbutyl)-cyclohexan, (optisch aktiv) und
10 % 1-Cyan-3-methyl-1-(4'-pentylbiphenyl-4-yl-ethyl)-cyclohexan,

zeigt K/S^{*} -29°, S^{*}_{C} /S^{*}_{A} 66°, SÄ /Ch 72°, Ch/I 89°, Pₛ = 30,2 nC/cm² bei 20°, Pitch in der cholesterischen Phase: -21 um, Tiltwinkel 28 bei 20 °C und eine Schaltzeit von 315 µs bei 2 um und 12 Volt.

### Beispiel 34

Zu einem auf 0 °C gekühlten Gemisch aus 67 g 4-Hydroxy-4'-heptylbiphenyl, 55,75 g 4-Cyan-4-pentyl- cyclohexancarbonsäure (erhältlich aus 4-Cyan-cyclohexan-carbonsäure durch Alkylierung mit Pentylbromid in Gegenwart von 2 Äquivalenten Lithiumdiisopropylamid), 3 g DMAP und 400 ml CH₂CCI₂ gibt man unter Eiskühlung 56 g DCC in 50 ml CH₂CCI₂. Dann wird das Eisbad entfernt und das Reaktionsgemisch 3 Std. bei Raumtemperatur gerührt. Der ausgefallene N,N'-Dicyclohexylharnstoff wird durch Filtration entfernt und die CH₂C1₂-Phase nacheinander mit H₂0, InHCI und NaHCO₃-Lösung gewaschen und über MgS0₄ getrocknet. Nach dem Abziehen des Lösungsmittels wird der Rückstand durch Kristallisation und Säulenchromatographie gereinigt. Man erhält r-1-Cyan-1-pentyl-cis-4-[p-(p-heptylphenyl)-phenoxycarbonyl]-cyclohexan.

Analog erhält man r-1-Cyan-1-pentyl-cis-4-(p-heptylphenoxycarbonyl)-cyclohexan sowie die homologen 1-Alkyl-1-cyancyclohexylverbindungen, die homologen p-Alkylphenyl-, p-Alkoxyphenyl, p-Alkanoyloxyphenyl-Verbindungen.

### Beispiel 35

Zu einem Gemisch aus 30 g p-Octyloxyphenol, 22 g p(4-Cyan-4-pentylcyclohexyl)-benzoesäure (erhältlich aus 4-Phenylcyclohexancarbonsäure durch Überführung in 4-Phenylcyclohexancarbonitril und anschließende Alkylierung des Nitrils mit Pentylbromid in Gegenwart von LDA als Base und Friedel-Crafts-Acylierung des Aromaten mit Acetylchlorid in Gegenwart von 2 Äquivalenten AlCl₃ und Haloformabbau), 1,5 g DMAP und 200 ml CH₂Cl₂ gibt man bei 0° bis 5° 22,7 g DCC in 25 ml CH₂ C1₂. Dann wird 3 Stunden bei Raumtemperatur nachgerührt und wie bereits beschrieben aufgearbeitet. Man erhält r-1-Cyan-1-pentyl-cis-4-[p-(p-octyloxyphenoxycarbonyl)-phenyl]-cyclohexan.
Analog erhält man die homologen 1-Cyan-1-alkyl-Verbindungen sowie die homologen p-Alkyl-, p-Alkoxy-, p-Alkanoyloxy- sowie p-Alkoxycarbonyloxy-phenoxycarbonyl-Verbindungen.

### Beispiel 36

Zu einem Gemisch aus 25 g p-Octyloxybenzoesäure, 27 g p(4-Cyan-4-pentylcyclohexyl)phenol (erhältlich aus p-Methoxyphenylcyclohexancarbonsäure durch Überführung in p-Methoxyphenylcyclohexancarbonitril und anschließende Alkylierung des Carbonitrils mit Pentylbromid in Gegenwart von LDA und anschließende Etherspaltung mit Jodtrimethylsilan), 1,5 g DMAP und 200 ml CH₂CCI₂ gibt man wie oben beschrieben 22,7 g DCC in 25 ml CH₂CCI₂. Es wird wie üblich aufgearbeitet. Man erhält r-1-Cyan-1-pentyl- cis-4-[p-(p-octyloxybenzoyloxy)-phenyl]-cyclohexan.
Analog erhält man die homologen 1-Cyan-1-alkyl-Verbindungen sowie die homologen p-Alkyl-, p-Alkoxy-, p-Alkanoyloxy-, p-Alkoxycarbonyl- sowie p-Alkoxycarbonyloxy-benzoyloxy-Verbindungen.

### Beispiel 37

Zu einem Gemisch aus 25 g p-Octyloxybenzoesäure, 28,5 g 2-Cyan-2-octyl-6-hydroxy-1,2,3,4-tetrahydronaphthalin (erhältlich durch Friedel-Crafts Reaktion von p-Methoxyphenylessigsäurechlorid mit Ethen in Gegenwart einer Lewis-Säure und anschließende Überführung des so erhaltenen Ketons in das Nitril nach C.W. Reese et al., JCS.Chem.Comm. 280, 1977, Alkylierung des Nitrils mit Octylbromid in Gegenwart von LDA als Base und Etherspaltung mit Jodtrimethylsilan), 1,5 g DMAP und 200 ml CH₂CI₂ gibt man wie üblich 22,7 g DCC in 25 ml CH₂CI₂ zu und arbeitet dann wie üblich auf. Man erhält 2-Cyan-2-octyl-6-(p-octyloxybenzoyloxy)-1,2,3,4-tetrahydronaphthalin.
Analog erhält man die homologen 2-Cyan-2-alkyl-Verbindungen sowie die homologen p-Alkoxy-, p-Alkyl-oder p-Alkanoyloxy-benzoyloxy-Verbindungen.

### Beispiel 38

Zu einer Lösung von 1,4 ml Diisopropylamin in 10 ml THF tropft man bei -20_{°} 6,1 ml einer 15 %igen Lösung von Butyllithium in Hexan. Anschließend kühlt man auf -60° , tropft eine Lösung von 3,74 g 1-Cyan-4-(4-n-octyl-biphenyl-4'-yl)-cyclohexan in 10 ml THF zu und rührt noch 15 Minuten nach. Man fügt portionsweise 7,6 g Perfluoroctyl-phenyliodonium-trifluormethansulfonat zu, rührt noch 30 Minuten bei -60°, entfernt das Kühlbad und läßt über Nacht bei Raumtemperatur nachrühren. Man versetzt mit 20 ml 2n Salzsäure und extrahiert mit Dichlormethan. Nach Aufarbeitung der organischen Phase erhält man r-1-Cyan-1 -heptadekafluoroctyl-cis-4-(4-n-octylbiphenyl-4'-yl)-cylohexan.

### Beispiel 39

Man löst 2,9 g r-1-Cyan-1-hexyl-cis-4-(4-hydroxyphenyl)-cyclohexanund 0,8 g Pyridin in 25 ml Toluol, tropft unter Rühren bei 100 5,1 g 4-Perfluorheptylbenzoyl-chlorid, gelöst in 10 ml Toluol, zu und läßt 3 Stunden nachreagieren. Nach dem Abkühlen wird vom Pyridinhydrochlorid abgesaugt, das Lösungsmittel verdampft und der Rückstand durch Kristallisation gereinigt. Man erhält r-1-Cyan-1-hexyl-cis-4[4-(4-perfluor- heptylbenzoyloxy)-phenyl]-cyclohexan.

### Beispiel 40

Ein Gemisch von 5,3 g p-[p-(4-Cyan-4-octyl-cyclohexyl)-phenyl]-phenol(erhältlich durch alkalische Etherspaltung aus r-1-Cyan-cis-4-(4'-propyloxybiphenyl-4-yl)-1-octylcyclohexan mit Kalium-tert.-butylat in NMP bei 180°), 1,9 g optisch aktiver 2-Chlor-3-methyl-buttersäure und 170 g DMAP werden in 40 ml CH₂CI₂ suspendiert. Dann werden bei 0° 3,1 g DCC in 5 ml CH₂CI₂ zugetropft und 12 Stunden bei Raumtemperatur gerührt. Nach Abtrennung des Dicyclohexylharnstoffes und üblicher Aufarbeitung erhält man optisch aktiven 2-Chlor-3-methyl-buttersäure-4'-(4-cyan-4-octylcyclohexyl)-biphenyl-4-yl-ester, K 81 S_{A} 138 I.

### Beispiel 42

Zu einem Gemisch von 6,24 g 4-Heptyloxy-4'-biphenylcarbonsäure, 4,46 g 4-Cyan-4-heptylcyclohexan- ol [erhältlich aus trans-4-Cyancyclohexanol durch Schützen der Hydroxylgruppe als THP-Ether, Umsetzung mit Heptyljodid und Lithiumdiisopropylamid und anschließende Abspaltung der Schutzgruppe] und 0,2 g 4-N,N'-Dimethylaminopyridin in 45 ml Dichlormethan werden unter Eiskühlung 4,54 g Dicyclohexylcarbodiimid in 10 ml Dichlormethan getropft. Nach 25 Stunden wird das ausgefallene Harnstoffderivat abgesaugt, das Filtrat mit Methylenchlorid verdünnt und nacheinander mehrmals mit verdünnter Salzsäure und mit Wasser gewaschen. Nach dem Trocknen, Entfernen des Lösungsmittels und Reinigung durch Chromatographie und Kristallisation erhält man 4-Heptyloxy-4'-biphenylcarbonsäure-(4-cyan-4-heptylcyclohexylester).

Analog werden hergestellt:
4-Octyloxy-4'-biphenylcarbonsäure-(4-cyan-4-heptylcyclohexylester)
4-Nonyloxy-4'-biphenylcarbonsäure-(4-cyan-4-heptylcyclohexylester)
4-Decyloxy-4'-biphenylcarbonsäure-(4-cyan-4-heptylcyclohexylester)
4-Hexyloxy-4'-biphenylcarbonsäure-(4-cyan-4-heptylcyclohexylester)
4-Pentyloxy-4'-biphenylcarbonsäure-(4-cyan-4-heptylcyclohexylester)
4-Butyloxy-4'-biphenylcarbonsäure-(4-cyan-4-heptylcyclohexylester)
4-Heptyl-4'-biphenylcarbonsäure-(4-cyan-4-heptylcyclohexylester)
4-Octyl-4'-biphenylcarbonsäure-(4-cyan-4-heptylcyclohexylester)
4-Nonyl-4'-biphenylcarbonsäure-(4-cyan-4-heptylcyclohexylester)
4-Decyl-4'-biphenylcarbonsäure-(4-cyan-4-heptylcyclohexylester)
4-Butyloxy-2'-fluor-4'-biphenylcarbonsäure-(4-cyan-4-heptylcyclohexylester)
4-Pentyloxy-2'-fluor-4'-biphenylcarbonsäure-(4-cyan-4-heptylcyclohexylester)
4-Hexyloxy-2'-fluor-4'-biphenylcarbonsäure-(4-cyan-4-heptylcyclohexylester)
4-Heptyloxy-2'-fluor-4'-biphenylcarbonsäure-(4-cyan-4-heptylcyclohexylester)
4-Octyloxy-2'-fluor-4'-biphenylcarbonsäure-(4-cyan-4-heptylcyclohexylester)
4-Nonyloxy-2'-fluor-4'-biphenylcarbonsäure-(4-cyan-4-heptylcyclohexylester)
4-Decyloxy-2'-fluor-4'-biphenylcarbonsäure-(4-cyan-4-heptylcyclohexylester)
4-Heptyl-2'-fluor-4'-biphenylcarbonsäure-(4-cyan-4-heptylcyclohexylester)
4-Octyl-2'-fluor-4'-biphenylcarbonsäure-(4-cyan-4-heptylcyclohexylester)
4-Nonyl-2'-fluor-4'-biphenylcarbonsäure-(4-cyan-4-heptylcyclohexylester)
4-Decyl-2'-fluor-4'-biphenylcarbonsäure-(4-cyan-4-heptylcyclohexylester)
4-Butyloxy-3'-fluor-4'-biphenylcarbonsäure-(4-cyan-4-heptylcyclohexylester)
4-Pentyloxy-3'-fluor-4'-biphenylcarbonsäure-(4-cyan-4-heptylcyclohexylester)
4-Hexyloxy-3'-fluor-4'-biphenylcarbonsäure-(4-cyan-4-heptylcyclohexylester)
4-Heptyloxy-3'-fluor-4'-biphenylcarbonsäure-(4-cyan-4-heptylcyclohexylester)
4-0cty!oxy-3'-f!uor-4'-bipheny!carbonsäure-(4-cyan-4-hepty!cyc!ohexy!ester)
4-Nonyloxy-3'-fluor-4'-biphenylcarbonsäure-(4-cyan-4-heptylcyclohexylester)
4-Decyloxy-3'-fluor-4'-biphenylcarbonsäure-(4-cyan-4-heptylcyclohexylester)
4-Heptyl-3'-fluor-4'-biphenylcarbonsäure-(4-cyan-4-heptylcyclohexylester)
4-Octyl-3'-fluor-4'-biphenylcarbonsäure-(4-cyan-4-heptylcyclohexylester)
4-Nonyl-3'-fluor-4'-biphenylcarbonsäure-(4-cyan-4-heptylcyclohexylester)
4-Decyloxy-3'-fluor-4'-biphenylcarbonsäure-(4-cyan-4-heptylcyclohexylester)
4-Butyloxy-3-fluor-4'-biphenylcarbonsäure-(4-cyan-4-heptylcyclohexylester)
4-Pentyloxy-3-fluor-4'-biphenylcarbonsäure-(4-cyan-4-heptylcyclohexylester)
4-Hexyloxy-3-fluor-4'-biphenylcarbonsäure-(4-cyan-4-heptylcyclohexylester)
4-Heptyloxy-3-fluor-4'-biphenylcarbonsäure-(4-cyan-4-heptylcyclohexylester)
4-Octyloxy-3-fluor-4'-biphenylcarbonsäure-(4-cyan-4-heptylcyclohexylester)
4-Nonyloxy-3-fluor-4'-biphenylcarbonsäure-(4-cyan-4-heptylcyclohexylester)
4-Decyloxy-3-fluor-4'-biphenylcarbonsäure-(4-cyan-4-heptylcyclohexylester)
4-Heptyl-3-fluor-4'-biphenylcarbonsäure-(4-cyan-4-heptylcyclohexylester)
4-Octyl-3-fluor-4'-biphenylcarbonsäure-(4-cyan-4-heptylcyclohexylester)
4-Nonyl-3-fluor-4'-biphenylcarbonsäure-(4-cyan-4-heptylcyclohexylester)
4-Decyloxy-3-fluor-4'-biphenylcarbonsäure-(4-cyan-4-heptylcyclohexylester)
4-Butyloxy-2-fluor-4'-biphenylcarbonsäure-(4-cyan-4-heptylcyclohexylester)
4-Pentyloxy-2-fluor-4'-biphenylcarbonsäure-(4-cyan-4-heptylcyclohexylester)
4-Hexyloxy-2-fluor-4'-biphenylcarbonsäure-(4-cyan-4-heptylcyclohexylester)
4-Heptyloxy-2-fluor-4'-biphenylcarbonsäure-(4-cyan-4-heptylcyclohexylester)
4-Octyloxy-2-fluor-4'-biphenylcarbonsäure-(4-cyan-4-heptylcyclohexylester)
4-Nonyloxy-2-fluor-4'-biphenylcarbonsäure-(4-cyan-4-heptylcyclohexylester)
4-Decyloxy-2-fluor-4'-biphenylcarbonsäure-(4-cyan-4-heptylcyclohexylester)
4-Hepty1-2-fluor-4'-biphenylcarbonsäure-(4-cyan-4-heptylcyclohexylester)
4-Octyl-2-fluor-4'-biphenylcarbonsäure-(4-cyan-4-heptylcyclohexylester)
4-Nonyl-2-fluor-4'-biphenylcarbonsäure-(4-cyan-4-heptylcyclohexylester)
4-Decyloxy-2-fluor-4'-biphenylcarbonsäure-(4-cyan-4-heptylcyclohexylester)
p-(trans-4-Propylcyclohexyl)-benzoesäure-(4-cyan-4-heptylcyclohexylester)
p-(trans-4-Butylcyclohexyl)-benzoesäure-(4-cyan-4-heptylcyclohexylester)
p-(trans-4-Pentylcyclohexyl)-benzoesäure-(4-cyan-4-heptylcyclohexylester)
p-(trans-4-Hexylcyclohexyl)-benzoesäure-(4-cyan-4-heptylcyclohexylester)
p-(trans-4-Heptylcyclohexyl)-benzoesäure-(4-cyan-4-heptylcyclohexylester)
p-(trans-4-Octylcyclohexyl)-benzoesäure-(4-cyan-4-heptylcyclohexylester)
p-(trans-4-Nonylcyclohexyl)-benzoesäure-(4-cyan-4-heptylcyclohexylester)
p-(trans-4-Decylcyclohexyl)-benzoesäure-(4-cyan-4-heptylcyclohexylester)
trans-4-(trans-4-Propylcyclohexyl)-cyclohexancarbonsäure-(4-cyan-4-heptylcyclohexylester)
trans-4-(trans-4-Butylcyclohexyl)-cyclohexancarbonsäure-(4-cyan-4-heptylcyclohexylester)
trans-4-(trans-4-Pentylcyclohexyl)-cyclohexancarbonsäure-(4-cyan-4-heptylcyclohexylester)
trans-4-(trans-4-Hexylcyclohexyl)-cyclohexancarbonsäure-(4-cyan-4-heptylcyclohexylester)
trans-4-(trans-4-Heptylcyclohexyl)-cyclohexancarbonsäure-(4-cyan-4-heptylcyclohexylester)
trans-4-(trans-4-Octylcyclohexyl)-cyclohexancarbonsäure-(4-cyan-4-heptylcyclohexylester)
trans-4-(trans-4-Nonylcyclohexyl)-cyclohexancarbonsäure-(4-cyan-4-heptylcyclohexylester)
trans-4-(trans-4-Decylcyclohexyl)-cyclohexancarbonsäure-(4-cyan-4-heptylcyclohexylester)
p-(5-Propylpyrimidin-2-yl)-benzoesäure-(4-cyan-4-heptylcyclohexylester)
p-(5-Butylpyrimidin-2-yl)-benzoesäure-(4-cyan-4-heptylcyclohexylester)
p-(5-Pentylpyrimidin-2-yl)-benzoesäure-(4-cyan-4-heptylcyclohexylester)
p-(5-Hexylpyrimidin-2-yl)-benzoesäure-(4-cyan-4-heptylcyclohexylester)
p-(5-Heptylpyrimidin-2-yl)-benzoesäure-(4-cyan-4-heptylcyclohexylester)
p-(5-Octylpyrimidin-2-yl)-benzoesäure-(4-cyan-4-heptylcyclohexylester)
p-(5-Nonylpyrimidin-2-yl)-benzoesäure-(4-cyan-4-heptylcyclohexylester)
p-(5-Decylpyrimidin-2-yl)-benzoesäure-(4-cyan-4-heptylcyclohexylester)
p-(5-Propylpyridin-2-yl)-benzoesäure-(4-cyan-4-heptylcyclohexylester)
p-(5-Butylpyridin-2-yl)-benzoesäure-(4-cyan-4-heptylcyclohexylester)
p-(5-Pentylpyridin-2-yl)-benzoesäure-(4-cyan-4-heptylcyclohexylester)
p-(5-Hexylpyridin-2-yl)-benzoesäure-(4-cyan-4-heptylcyclohexylester)
p-(5-Heptylpyridin-2-yl)-benzoesäure-(4-cyan-4-heptylcyclohexylester)
p-(5-Octylpyridin-2-yl)-benzoesäure-(4-cyan-4-heptylcyclohexylester)
p-(5-Nonylpyridin-2-yl)-benzoesäure-(4-cyan-4-heptylcyclohexylester)
p-(5-Decylpyridin-2-yl)-benzoesäure-(4-cyan-4-heptylcyclohexylester)

### Beispiel 43

Zu einem Gemisch von 6,3 4-Heptyl-4-cyancyclohexancarbonsäure [F. 880, erhältlich aus 4-Cyancyclohexancarbonsäure durch Alkylierung mit Heptylbromid und Lithiumdiisopropylamid], 7,0 g 4-Octyl-4'-hydroxybiphenyl und 100 mg 4-N,N'-Dimethylaminopyridin in 150 ml Dichlormethan tropft man bei 0 °5,6 g Dicyclohexylcarbodiimid in 50 ml Dichlormethan.

Unter Erwärmung auf Raumtemperatur läßt man das Reaktionsgemisch zwei Stunden rühren, saugt den ausgefallenen Harnstoff ab und arbeitet das Filtrat wie üblich auf. Man erhält r-1-Cyan-1-heptylcyclohexan- cis-4-carbonsäure-(4-octylbiphenyl-4'-yl-ester), K 71 S_{B} 100 S_{c} 118 S_{A} 161 N 165 I.

Analog erhält man die entsprechenden Ester durch Umsetzung mit folgenden Hydroxyverbindungen oder homologen Verbindungen:

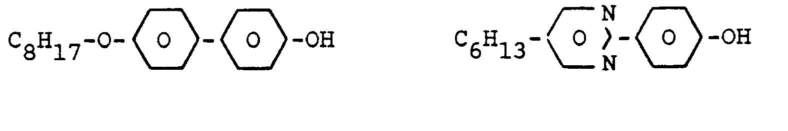

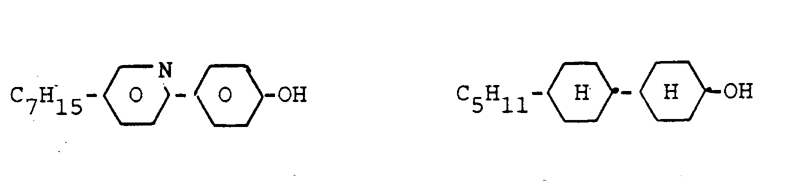

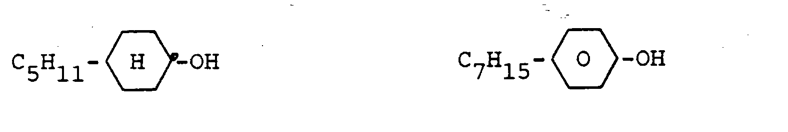

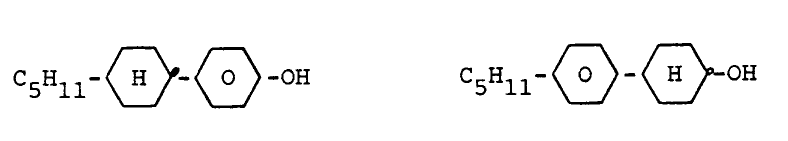

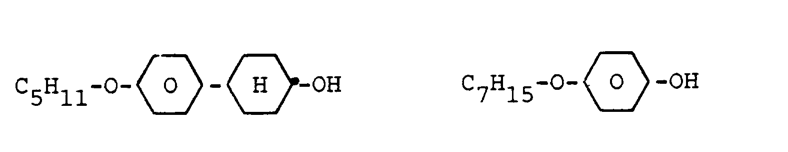

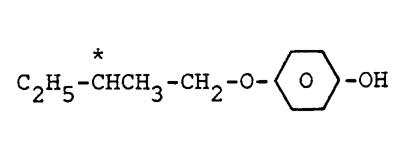

### Beispiel 44

Durch Umsetzung von 4-Cyan-4-heptylcyclohexylmethyljodid [erhältlich durch Reduktion von 4-Cyan-4- heptylcyclohexancarbonsäureethylester mit LiBH₃CN zum entsprechenden Methylalkohol, Überführung in das Mesylat und Umsetzung mit NaJ in Aceton] mit trans-4-Pentyl-cyclohexanol in Gegenwart von Natriumhydrid/Diglyme erhält man trans-4-Pentylcyclohexyl-(4-cyan-4-heptylcyclohexyl)-methylether.

Analog erhält man die entsprechenden Ether aus den Cyclohexanolen aus Beispiel 43.

### Beispiel 45

Eine flüssigkristalline Phase bestehend aus
2 % 2-p-Hexyloxyphenyl-5-heptylpyrimidin,
2 % 2-p-Heptyloxyphenyl-5-heptylpyrimidin,
2 % 2-p-Octyloxyphenyl-5-heptylpyrimidin,
2 % 2-p-Nonyloxyphenyl-5-heptylpyrimidin,
7 % 2-p-Hexyloxyphenyl-5-nonylpyrimidin,
25 % 2-p-Nonyloxyphenyl-5-nonylpyrimidin,
30 % r-1-Cyan-cis-4-(4'-octyloxybiphenyl-4-yl)-1-octylcyclohexan,
5 % r-1-Cyan-cis-4-(4'-hexylbiphenyl-4-yl)-1-heptylcyclohexan,
14 % r-1-Cyan-cis-4-(4'-heptyloxybiphenyl-4-yl)-1-(2-methylbutyl)-cyclohexan (optisch aktiv) und
11 % 1-Cyan-3-methyl-1-(4'-pentylbiphenyl-4-yl-ethyl)-cyclohexan (optisch aktiv)

zeigt K/S^{*}_{C} < -30°, S^{*}_{C} /S_{A} 65°, S_{A}/Ch 71 °, Ch/I 87°, Pₛ = 32,7 nC/cm² bei 20 und eine Schaltzeit von 90 µs bei 20° und 15 V/um.

### Beispiel 46

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
6 % 2-p-Hexyloxyphenyl-5-nonylpyrimidin,
25 % 2-p-Nonyloxyphenyl-5-nonylpyrimidin,
26 % r-1-Cyan-cis-4-(4'-butyloxybiphenyl-4-yl)-1-octylcyclohexan,
11 % r-1-Cyan-cis-4-(4'-hexylbiphenyl-4-yl)-1-heptylcyclohexan,
10 % r-1-Cyan-cis-4-(4'-octylbiphenyl-4-yl)-1-perfluoroctylcyclohexan und
10 % 2-[p-(5-nonylpyrimidin-2-yl)-phenoxy]-propionsäureethylester (optisch aktiv)

zeigt S^{*}_{C}/S_{A} 59°, S_{A}/Ch 75°, Ch/I 88 ° und Pₛ = 8 nC/cm².

### Beispiel 47

Eine flüssigkristalline Phase bestehend aus
3 % 2-p-Hexyloxyphenyl-5-octylpyrimidin,
3 % 2-p-Heptyloxyphenyl-5-octylpyrimidin,
3 % 2-p-Octyloxyphenyl-5-octylpyrimidin,
5 % 2-p-Hexyloxyphenyl-5-nonylpyrimidin,
5 % 2-p-Heptyloxyphenyl-5-nonylpyrimidin,
27 % 2-p-Nonyloxyphenyl-5-nonylpyrimidin,
25 % r-1-Cyan-cis-4-(4'-octyloxybiphenyl-4-yl)-1-octylcyclohexan,
10 % r-1-Cyan-cis-4-(4'-hexylbiphenyl-4-yl)-1-heptylcyclohexan,
10 % p-Perfluorheptylbenzoesäure-p-(4-cyan-4-hexylcyclohexyl)-phenylester und
9 % p-(5-Hexylpyrimidin-2-yl)-phenyl-2-chlorpropionat (optisch aktiv)

zeigt S^{*}_{C}/S_{A} 64°, S_{A}/Ch 74°, Ch/I 87 ° und Pₛ = 11 nC/cm².

### Beispiel 49

Man stellt ein ferroelektrisches smektisches Material her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
28 % r-1-Cyan-cis-4-(4'-butyloxybiphenyl-4-yl)-1-octylcyclohexan,
14 % r-1-Cyan-cis-4-(4'-hexyl-biphenyl-4-yl)-1-heptylcyclohexan,
6 % r-1-Cyan-1-(trans-4-pentylcyclohexyl)-cis-4-(trans-4-pentylcyclohexyl)-cyclohexan und
10 % r-1-Cyan-cis-4-[4'-(3,7-dimethyloctyloxy)-biphenyl-4-yl]-1-octylcyclohexan (optisch aktiv).

### Beispiel 51

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
6 % 2-p-Hexyloxyphenyl-5-nonylpyrimidin,
23 % 2-p-Nonyloxyphenyl-5-nonylpyrimidin,
15 % r-1-Cyan-cis-4-(4'-octyloxybiphenyl-4-yl)-1-heptylcyclohexan,
15 % r-1-Cyan-cis-4-(4'-octyloxybiphenyl-4-yl)-1-octyl cyclohexan,
5 % 2-Cyan-2-Octyl-6-(p-heptyloxyphenyl)-trans-decalin,
5 % r-1-Cyan-cis-4-pentylcyclohexancarbonsäure-(4'-hexylbiphenyl-4-yl-ester,
10 % 1-Cyan-3-methyl-1-(4'-pentylbiphenyl-4-yl-ethyl)-cyclohexan (optisch aktiv) und
9 % r-1-Cyan-cis-4-(4'-heptyloxybiphenyl-4-yl)-1-(2-methylbutyl)-cyclohexan (optisch aktiv)

zeigt S^{*}_{C}/S_{A} 58°, S_{A}/Ch 65°, Ch/I 82 ° und Pₛ = 24 nC/cm².

### Beispiel 53

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
3 % 2-p-Hexyloxyphenyl-5-nonylpyridin,
3 % 2-p-Octyloxyphenyl-5-nonylpyridin,
23 % 2-p-Nonyloxyphenyl-5-nonylpyrimidin,
15 % r-1-Cyan-cis-4-(4'-octyloxybiphenyl-4-yl)-1-octylcyclohexan,
15 % r-1-Cyan-cis-4-(4'-octyloxybiphenyl-4-yl)-1-nonylcyclohexan,
15 % r-1-Cyan-cis-4-(4'-hexylbiphenyl-4-yl)-1-heptylcyclohexan,
4 % r-1-Cyan-cis-4-(4'-heptylmercaptobiphenyl-4-yl)-1-octylcyclohexan und
10 % 1-Cyan-3-methyl-1-(4'-pentylbiphenyl-4-yl-ethyl)-cyclohexan (optisch aktiv).

### Beispiel 54

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
6 % 2-p-Hexyloxyphenyl-5-nonylpyridin,
25 % 2-p-Nonyloxyphenyl-5-nonylpyrimidin,
15 % r-1-Cyan-cis-4-(4'-octyloxybiphenyl-4-yl)-1-octylcyclohexan,
15 % r-1-Cyan-cis-4-(4'-octyloxybiphenyl-4-yl)-1-nonylcyclohexan,
5 % p-(4-Cyan-4-heptylcyclohexyl)-benzoesäure-p-heptylphenylester,
5 % 1-Cyan-4-pentylcyclohexancarbonsäure-p-(trans-4-propylcyclohexyl)-phenylester,
5 % r-1-Cyan-cis-4-(4'-hexylbiphenyl-4-yl)-1-heptylcyclohexan und
12 % 2-Chlor-3-methylbuttersäure-p-(5-nonylpyrimidin-2-yl)-phenylester (optisch aktiv).

### Beispiel 55

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
6 % 2-p-Hexyloxyphenyl-5-nonylpyridin,
25 % 2-p-Nonyloxyphenyl-5-nonylpyrimidin,
15 % r-1-Cyan-cis-4-(4'-butyloxybiphenyl-4-yl)-1-octylcyclohexan,
15 % r-1-Cyan-cis-4-(4'-octyloxybiphenyl-4-yl)-1-octylcyclohexan,
3 % r-1-Cyan-cis-4-(4'-heptyloxybiphenyl-4-yl)-1-[2-(trans-4-pentylcyclohexyl)-ethyl]-cyclohexan,
5 % r-1-Cyan-cis-4-[p-(5-heptyldioxan-2-yl)-phenyl]-1-hexylcyclohexan,
5 % r-1-Cyan-cis-4-(4'-butylbiphenyl-4-yl)-1-octyl-cyclohexan und
14 % 2-Chlor-3-methylbuttersäure-p-(5-hexylpyrimidin-2-yl)-phenylester (optisch aktiv)

zeigt S_{C}^{*}/S_{A} 60°, S_{A}/Ch 63°, Ch/I 83 und Pₛ = 17 nC/cm².

### Beispiel 56

Man stellt ein ferroelektrisches smektisches Material her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-Heptylpyrimidin,
7 % 2-p-Hexyloxyphenyl-5-nonylpyridin,
23 % 2-p-Nonyloxyphenyl-5-nonylpyrimidin,
28 % r-1-Cyan-cis-4-(4'-butyloxybiphenyl-4-yöl)-1-octylcyclohexan,
14 % r-1-cyan-cis-4-(4'-hexyl-biphenyl-4-yl)-1-heptylcyclohexan,
6 % r-1-Cyan-1-(trans-4-pentylcyclohexyl)-cis-4-(trans-4-pentylcyclohexyl)-cyclohexan und
10 % 2-[4'-(4-Cyan-4-octylcyclohexyl)-biphenyl-4-yloxy]-propionsäureethylester (optisch aktiv).

### Beispiel 57

Zu einem Gemisch von 19,4 g 4-(4'-Hydroxybiphenyl-4-yl)-1-cyan-1-octylcyclohexan, 6,5 g Ethyllactat und 13,1 g Triphenylphosphin in 300 ml Tetrahydrofuran gibt man 9,6 g Diethylazodicarboxylat gelöst in Tetrahydrofuran, rührt eine Stunde bei 500, läßt über Nacht stehen, entfernt das Lösungsmittel und nimmt den Rückstand in 100 ml heißem Toluol auf. Nach dem Abkühlen der Toluollösung auf 0 wird filtriert und aus dem Filtrat das Produkt wie üblich durch Chromatographie isoliert. Man erhält optisch aktiven 2-[4'-(4-Cyan-4-octylcyclohexyl)-biphenyl-4-yloxy]-propionsäureethylester.

Durch entsprechende Umsetzung mit Alkyllactaten mit Phenolen der Formeln 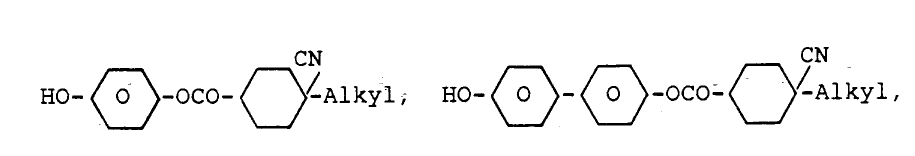 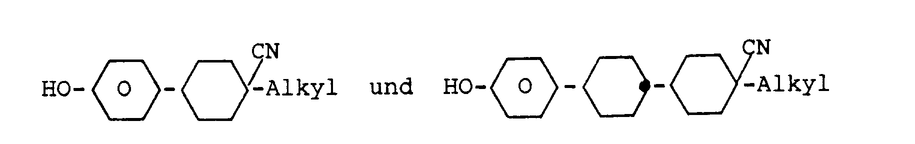 erhält man analoge Verbindungen.

### Beispiel 58

Zu einem Gemisch von 20,6 g p-Hydroxybenzoesäure-(4-cyan-4-heptylcyclohexyl)-ester [erhältlich durch Veresterung von 4-Cyan-4-heptylcyclohexanol mit p- Benzyloxybenzoesäure und Abspaltung der Benzylgruppe durch katalytische Hydrierung], 11,5 g optisch aktivem Hexyllactat und 15,7 g Triphenylphosphin in 150 ml Tetrahydrofuran gibt man eine Lösung von 11,5 g Diethylazodicarboxylat in Tetrahydrofuran, rührt eine Stunde bei 50 und entfernt nach 12 Stunden das Lösungsmittel. Der Rückstand wird in 100 ml heißem Toluol gelöst, abgekühlt, filtriert und das Filtrat wie üblich durch Chromatographie und Kristallisation gereinigt. Man erhält optisch aktiven p-(4-Cyan-4-heptylcyclohexyloxycarbonyl)-phenoxypropionsäurehex- ylester.

Durch entsprechende Umsetzung von Alkyllactaten mit Phenolen der Formel 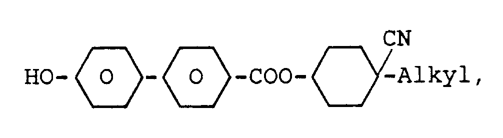 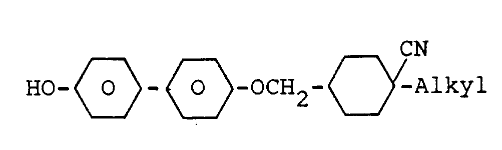 und 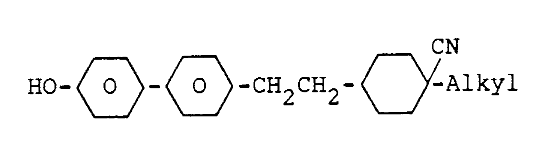 erhält man analoge Verbindungen.

## Patentansprüche

1. Verwendung von Verbindungen der Formel I
R¹⁻Q¹⁻A-(Q²)_{q}-R² I
worin
R¹ und R² jeweils unabhängig voneinander eine Alkylgruppe oder eine Polyfluoralkylgruppe mit jeweils 1 bis 15 C-Atomen, worin auch eine oder mehrere CH₂-Gruppen bzw. CF₂-Gruppen durch eine Gruppierung ausgewählt aus der Gruppe -O-, -S-, -CO-, -CH-Halogen-, -O-CO-, -O-COO-, -CO-O- und -CH = CH- oder auch durch eine Kombination von zwei geeigneten Gruppierungen ersetzt sein können, wobei zwei Heteroatome nicht direkt miteinander verknüpft sind, einer der Reste R¹ und R² auch Halogen,
A 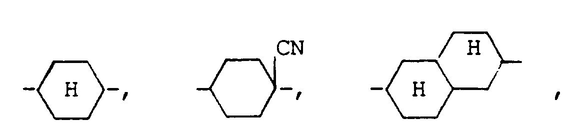 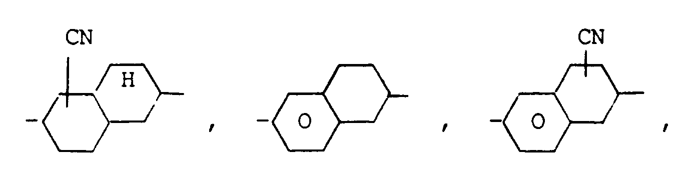 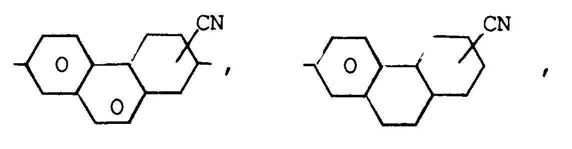 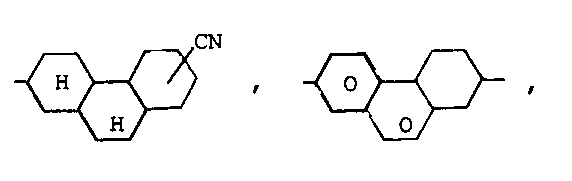 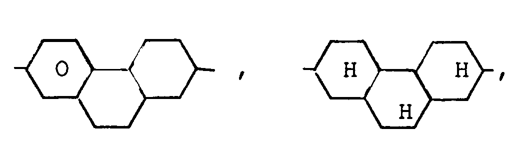 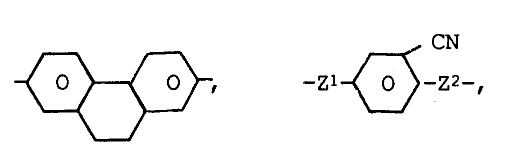 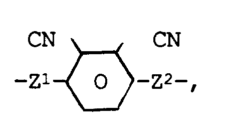 oder eine dieser Gruppen, worin eine oder mehrere nicht benachbarte CH₂-Gruppen durch O und/oder S oder aliphatische und/oder aromatische CH-Gruppen durch N ersetzt sind,
q 0 oder 1,
Q¹ und Q² jeweils unabhängig voneinander -(A°-Z°)ₚ-, wobei
A unsubstituiertes oder ein- oder mehrfach durch Halogenatome, CH₃- und/oder Nitril-Gruppen substituiertes 1,4-Cyclohexylen, worin auch eine oder zwei nicht benachbarte CH₂-Gruppen durch -O- und/oder -S- und/oder eine 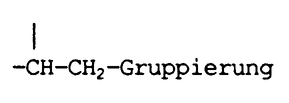 durch 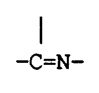 ersetzt sein können (Cy), oder unsubstituiertes oder ein- oder mehrfach durch Halogenatome, CH₃- und/oder Nitril-Gruppen substituiertes 1,4-Phenylen, worin auch eine oder mehrere CH-Gruppen durch N ersetzt sein können (Ph), bedeutet, einer der Reste A auch 2,6-Naphthylen (Na) oder Tetrahydro-2,6-naphthylen (4H-Na), gegebenenfalls durch Halogen oder CN substituiert,
Z^{°}, Z¹ und Z² jeweils unabhängig voneinander -CO-O-, -O-CO-, -CH₂0-, -OCH₂-, -CH₂CH₂-, -CHCN-CH₂-, -CH₂-CHCN- oder eine Einfachbindung, und
p 1, 2 oder 3, oder im Falle A = Tetra- oder Octahydrophenanthren auch 0 bedeutet, wobei: im Falle A = 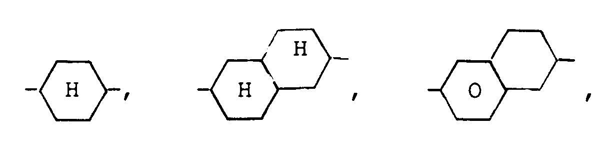 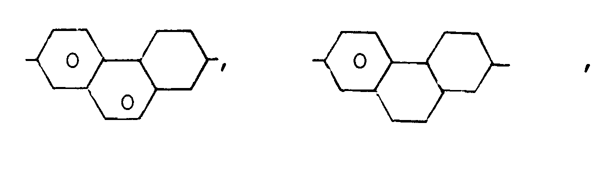 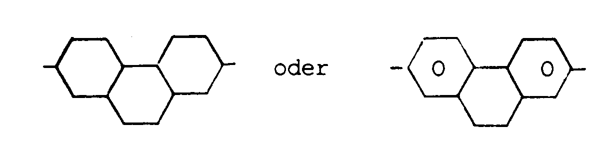 mindestens eine Gruppe Z ° -CHCNCH₂- oder -CH₂CHCN- bedeutet,
als Komponenten chiraler getilteter smektischer Phasen mit negativen Werten der dielektrischen Anisotropie, wobei
r-1-Cyan-1-(trans-4-pentylcyclohexyl)-trans-4-(trans-4-heptylcyclohexyl)-cyclohexan
1-Cyan-1-(trans-4-pentylcyclohexyl)-2-(trans-4-propylcyclohexyl)-ethan
1-Cyan-1-(trans-4-pentylcyclohexyl)-2-[trans-4-(trans-4-pentylcyclohexyl)-cyclohexyl]-ethan
r-1-Cyan-cis-4-(trans-4-butylcydohexyl(-1-butylcydohexan
sowie die Verbindungen der Formeln 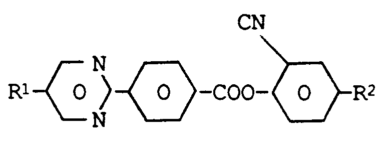 und 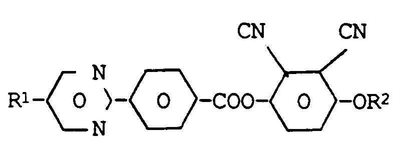 und worin
R¹ und R² unverzweigtes C₃₋₁₀-Alkyl bedeuten,
ausgenommen sind.

2. Chirale getiltete smektisch flüssigkristalline Phase mit negativen Werten der dielektrischen Anisotropie mit mindestens zwei flüssigkristallinen Komponenten, dadurch gekennzeichnet, daß sie mindestens eine Verbindung der Formel 1 nach Anspruch 1 enthält.

3. Phase nach Anspruch 2, worin mindestens eine der Verbindungen der Formel la
R¹⁻Q¹⁻A-R² la
entspricht.

4. Phase nach Anspruch 2, worin A eine Gruppe der Formel (A) 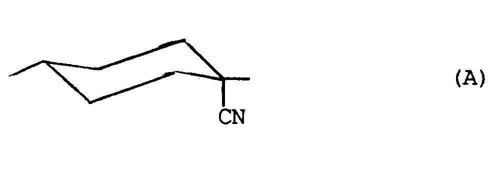 oder deren Spiegelbild bedeutet.

5. Phase nach Anspruch 2, worin R¹ und R², jeweils unabhängig voneinander R-, R-O-, R-O-CO-, R-O-COO- oder R-COO-, worin R eine geradkettige Alkylgruppe mit 5 bis 12 C-Atomen ist, bedeuten.

6. Phase nach Anspruch 2, worin R¹ und R², jeweils unabhängig voneinander R_{F}, R_{F}CH₂, R_{F}CH₂CH₂, R_{F}CH₂0 und R_{F}COO bedeuten, worin R_{F} eine geradkettige Perfluoralkylgruppe mit 2-12 C-Atomen bedeutet, worin auch eine oder mehrere Fluoratome durch H ersetzt sein können.

7. Phase nach Anspruch 2, worin mindestens einer der Reste R¹ und R² R_{F} bedeutet, wobei R_{F} eine geradkettige Perfluoralkylgruppe mit 2 bis 12 C-Atomen ist, worin auch ein oder mehrere Fluoratome durch H ersetzt sein können.

8. Phase nach Anspruch 2, dadurch gekennzeichnet, daß sie mindestens eine optische aktive Verbindung enthält.

9. Phase nach Anspruch 2, dadurch gekennzeichnet, daß die Verbindungen der Formel I ausgewählt sind aus den Verbindungen der Teilformeln la, Ib, Ic und II, 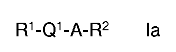 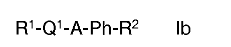 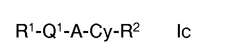 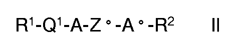 worin R¹, Q¹, A, A°, Z°, Cy, Ph und R² die in Anspruch 1 gegebene Bedeutung besitzen.

10. Phase nach Anspruch 9, dadurch gekennzeichnet, daß die Verbindungen der Teilformel la ausgewählt ist aus den Verbindungen der Teilformeln lah, lal, lam und lan, 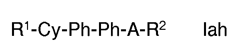 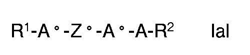 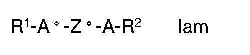 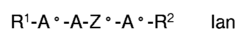 worin Cy, Ph und R² die in Anspruch 1 angegebene Bedeutung besitzen und R¹ Alkyl, Alkoxy, Alkanoyloxy, Alkoxycarbonyl oder Alkoxycarbonyloxy bedeutet,
A trans-1,4-Cyclohexylen oder gegebenenfalls durch 1-Fluoratom substituiertes 1,4-Phenylen, und
Z 0 -CO-O-, -O-CO- oder -CH₂CH₂ oder, falls A trans-1,4-Cyclohexylen ist, auch -CH₂-CHCN-oder -CHCN-CH₂- bedeuten.

11. Phase nach Anspruch 10, dadurch gekennzeichnet, daß die Verbindungen der Teilformel lal ausgewählt sind aus den Teilformeln lala bis lalc, 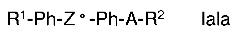 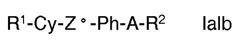 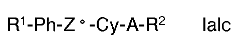 worin R¹, R², Ph, A, Z und R² die für Teilformel lal angegebene Bedeutung besitzen.

12. Elektrooptisches Anzeigeelement, dadurch gekennzeichnet, daß es als Dielektrikum eine Phase nach Anspruch 2 oder 3 enthält.

## Claims

1. Use of compounds of the formula I 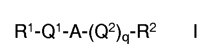 in which
R¹ and R² are each, independently of one another, an alkyl group or a polyfluoroalkyl group, in each case having 1 to 15 carbon atoms, in which, in addition, one or more CH₂ groups or CF₂ groups may be replaced by a group selected from the group consisting of -O-, -S-, -CO-, -CH-halogen-, -O-CO-, -O-COO-, -CO-O- and -CH = CH- or alternatively by a combination of two suitable groups, where two hetero-atoms are not linked directly to one another, or one of the radicals R¹ and R² is alternatively halogen,
A is 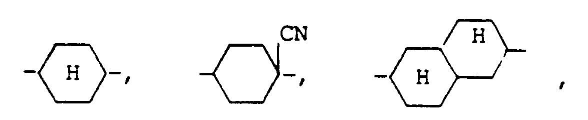 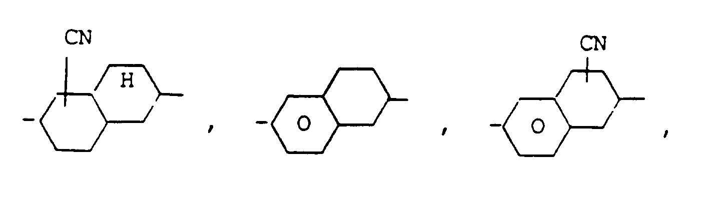 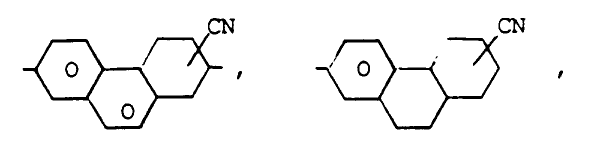 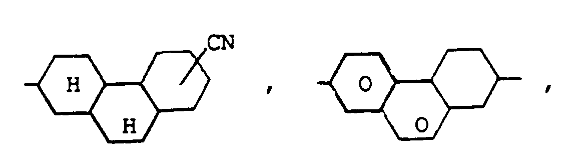 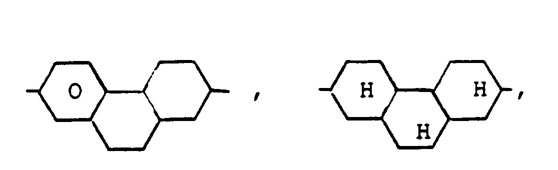 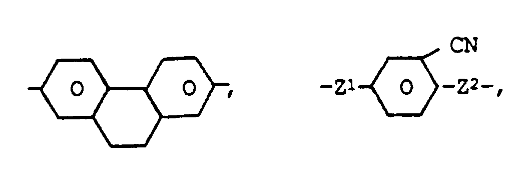 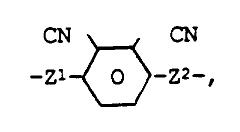 or one of these groups in which one or more non-adjacent CH₂ groups have been replaced by O and/or S or aliphatic and/or aromatic CH groups have been replaced by N,
q is 0 or 1,
Q¹ and Q² are each, independently of one another, -(A°-Z°)ₚ-, where
A is 1,4-cyclohexylene which is unsubstituted or monosubstituted or polysubstituted by halogen atoms, CH₃ and/or nitrile groups, in which, in addition, one or two non-adjacent CH₂ groups may be replaced by -O- and/or -S- and/or one 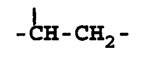 group may be replaced by 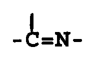 (Cy), or 1,4-phenylene which is unsubstituted or monosubstituted or polysubstituted by halogen atoms, CH₃ and/or nitrile groups, in which, in addition, one or more CH groups may be replaced by N (Ph), or one of the radicals A is alternatively 2,6-naphthylene (Na) or tetrahydro-2,6-naphthylene (4H-Na), optionally substituted by halogen or CN,
Z°, Z¹ and Z² are each, independently of one another, -CO-O-, -O-CO-, -CH₂-O-, -OCH₂-, -CH₂CH₂-, -CHCN-CH₂-, -CH₂-CHCN- or a single bond, and
p is 1, 2 or 3, or in the case of A = tetra- or octahydrophenanthrene, is alternatively 0, where: in the case where A = 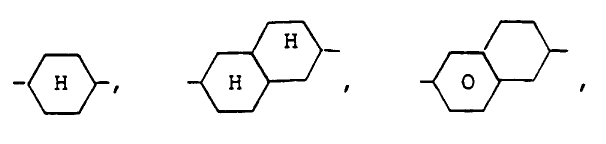 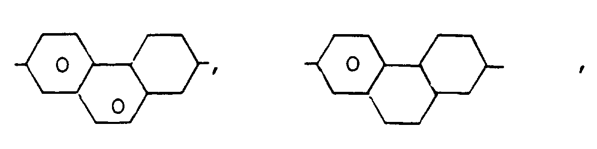 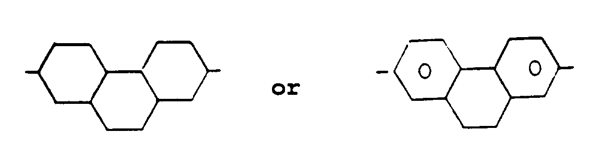 at least one group Z ° is -CHCNCH₂- or -CH₂CHCN-,
as components of chiral, tilted, smectic phases having negative dielectric anisotropy values, with the exclusion of
r-1-cyan-1-(trans-4-pentylcyclohexyl)-trans-4-(trans-4-heptylcyclohexyl)cyclohexane
1-cyan-1-(trans-4-pentylcyclohexyl)-2-(trans-4-propylcyclohexyl)ethane
1-cyan-1-(trans-4-pentylcyclohexyl)-2-[trans-4-(trans-4-pentylcyclohexyl)cyclohexyl]ethane
r-1-cyan-cis-4-(trans-4-butylcyclohexyl)-1-butylcyclohexane
and the compounds of the formulae 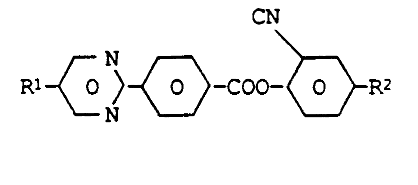 and 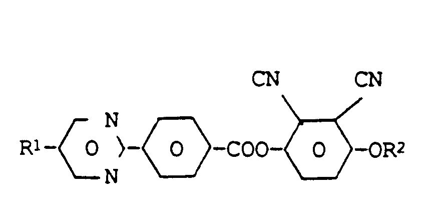 and in which
R¹ and R² are unbranched C₃₋₁₀-alkyl.

2. Chiral, tilted, smectic, liquid-crystalline phase having negative dielectric anisotropy values, containing at least two liquid-crystalline components, characterized in that it contains at least one compound of the formula I according to Claim 1.

3. Phase according to Claim 2, in which at least one of the compounds conforms to the formula la

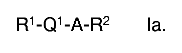

4. Phase according to Claim 2, in which A is a group of the formula (A) 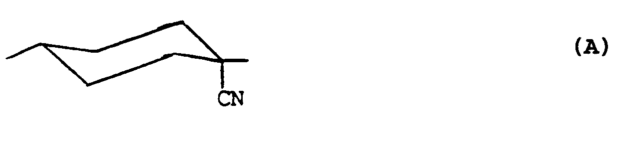 or the mirror image thereof.

5. Phase according to Claim 2, in which R¹ and R², in each case independently of one another, are R-, R-O-, R-O-CO-, R-O-COO- or R-COO-, in which R is a straight-chain alkyl group having 5 to 12 carbon atoms.

6. Phase according to Claim 2, in which R¹ and R², in each case independently of one another, are R_{F}, R_{F}CH₂, R_{F}CH₂CH₂, R_{F}CH₂0 or R_{F}COO, in which R_{F} is a straight-chain perfluoroalkyl group having 2 - 12 carbon atoms, and in which one or more fluorine atoms may be replaced by H.

7. Phase according to Claim 2, in which at least one of the radicals R¹ and R² is R_{F}, where R_{F} is a straight-chain perfluoroalkyl group having 2 to 12 carbon atoms, and in which one or more fluorine atoms may be replaced by H.

8. Phase according to Claim 2, characterized in that it contains at least one optically active compound.

9. Phase according to Claim 2, characterized in that the compounds of the formula I are selected from the compounds of the subformulae la, Ib, Ic and II, 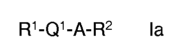 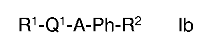 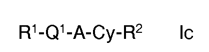 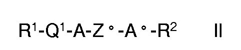 in which R¹, Q¹, A, A°, Z°, Cy, Ph and R² are as defined in Claim 1.

10. Phase according to Claim 9, characterized in that the compounds of the subformula la are selected from the compounds of the subformulae lah, lal, lam and Ian, 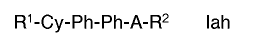 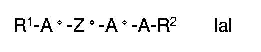 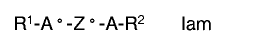 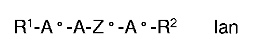 in which Cy, Ph and R² are as defined in Claim 1, and R¹ is alkyl, alkoxy, alkanoyloxy, alkoxycarbonyl or alkoxycarbonyloxy,
A is trans-1,4-cyclohexylene or 1,4-phenylene which is optionally substituted by one fluorine atom, and
Z ° is -CO-O-, -O-CO- or -CH₂CH₂ or, if A is trans-1,4-cyclohexylene, is alternatively -CH₂-CHCN- or -CHCN-CH₂.

11. Phase according to Claim 10, characterized in that the compounds of the subformula lal are selected from the subformulae lala to lalc, 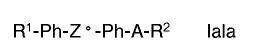 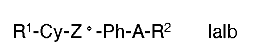 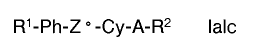 in which R¹, R², Ph, A, Z ° and R² are as defined for subformula lal.

12. Electro-optical display element, characterized in that it contains, as dielectric, a phase according to Claim 2 or 3.

## Revendications

1. Application de composés de formule I : 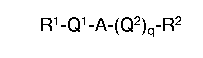 dans laquelle :
R¹ et R² représentent chacun indépendamment l'un de l'autre un groupe alkyle ou un groupe polyfluoroalkyle avec à chaque fois de 1 à 15 atomes de carbone, où encore un ou plusieurs groupes CH₂ ou groupes CF₂ peuvent être remplacés par un groupement choisi dans le groupe -O-, -S-, -CO-, -CH-halogène-, -O-CO-, -O-COO-, -CO-O- et -CH=CH- ou encore par une combinaison de deux groupements appropriés, où deux hétéroatomes ne sont pas directement reliés l'un à l'autre, l'un des radicaux R¹ et R² représente également un halogène,
A représente 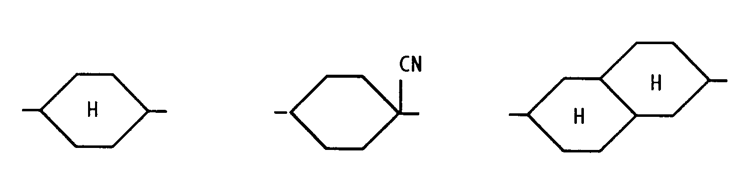 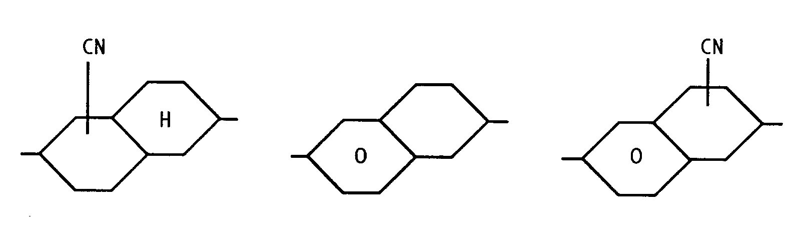 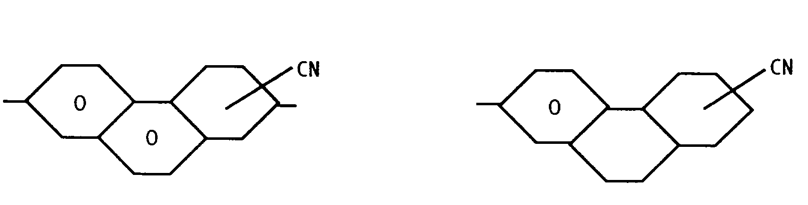 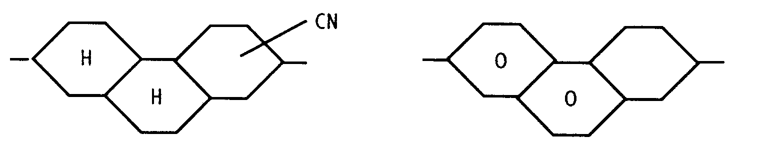 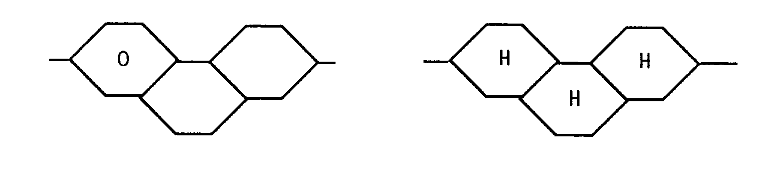 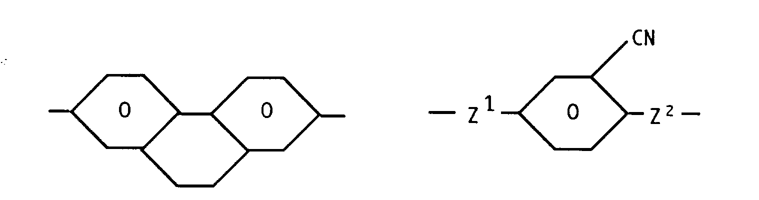 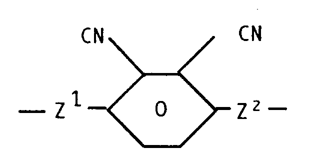 ou un de ces groupes, où un ou plusieurs groupes CH₂ non voisins sont remplacés par O et/ou S, ou des groupes CH aliphatiques et/ou aromatiques par de l'azote,
q vaut 0 ou 1,
Q¹ et Q² représentent chacun indépendamment l'un de l'autre -(A °-Z °)ₚ, où
A représente un 1,4-cyclohexylène non substitué, mono- ou poly-substitué par des atomes d'halogène, des groupes CH₃ et/ou nitrile, où également un ou deux groupes CH₂ non voisins peuvent être remplacés par -O- et/ou -S- et/ou un groupement 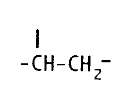 par 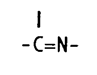 (Cy), ou un 1,4-phénylène non substitué, mono ou polysubstitué par des atomes d'halogène, des groupes CH₃ et/ou nitrile, où également un ou plusieurs groupes CH peuvent être remplacés par N (Ph), l'un des radicaux A représente également le 2,6-naphtylène (Na) ou le tétrahydro-2,6-naphtylène (4H-Na), éventuellement substitué par un halogène ou CN,
Z°, Z¹ et Z² représentent chacun indépendamment l'un de l'autre -CO-O-, -O-CO-, -CH₂O-, OCH₂-, -CH₂CH₂-, -CHCN-CH₂-, -CH₂CHCN- ou une liaison simple, et
p vaut 1, 2 ou 3, ou encore 0, dans le cas où A = tétra- ou octa-hydrophénanthrène, et ;
dans le cas où A = 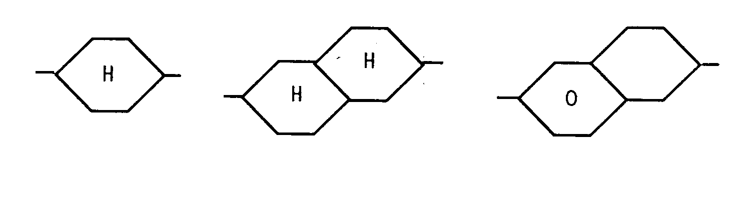 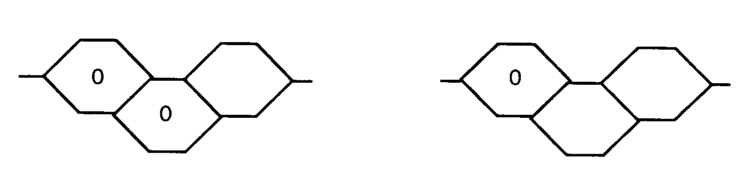 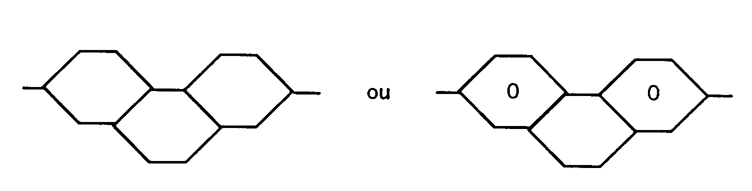 au moins un groupe Z ° représente -CHCNCH₂- ou -CH₂CHCN-,
comme composants de phases smectiques inclinées chirales à valeurs négatives de l'anisotropie diélectrique, où sont exclus les composés
r-1-cyano-1-(trans-4-pentylcyclohexyl)-trans-4-(trans-4-heptylcyclo-hexyl)-cyclohexane,
1-cyano-1-(trans-4-pentylcyclohexyl)-2-(trans-4-propylcyclohexyl)-éthane,
1-cyano-1-(trans-4-pentylcyclohexyl)-2-[trans-4-(trans-4-pentyl-cyclohexyl)-cyclohexyl]-éthane,
r-1-cyano-cis-4-(trans-4-butylcyclohexyl)-1-butylcyclohexane,
ainsi que les composés de formules : 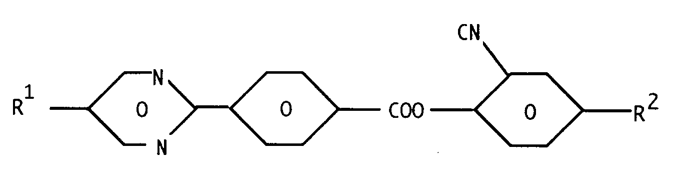 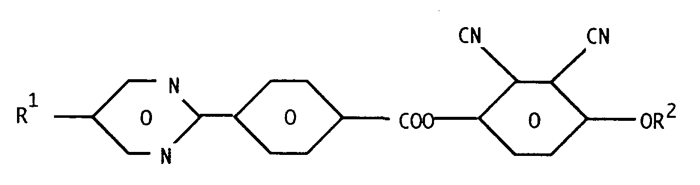 dans lesquelles
R¹ et R² représentent un alkyle en C₃ à C₁₀ non ramifié.

2. Phase cristalline liquide smectique inclinée chirale avec valeurs négatives de l'anisotropie diélectrique avec au moins deux composants à cristaux liquides, caractérisée en ce qu'elle contient au moins un composé de formule I selon la revendication 1.

3. Phase selon la revendication 2, où au moins un des composés correspond à la formule la :

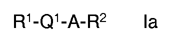

4. Phase selon la revendication 2, où A représente un groupe de formule (A) : 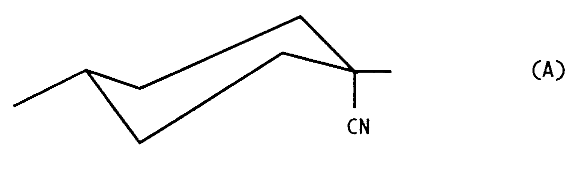 ou son image spéculaire.

5. Phase selon la revendication 2, où R¹ et R² représentent chacun indépendamment l'un de l'autre R-, R-O-, R-O-CO-, R-O-COO- ou R-COO-, où R est un groupe alkyle à chaîne droite en C₅ à C₁₂.

6. Phase selon la revendication 2, où R¹ et R² représentent chacun indépendamment l'un de l'autre R_{F}, R_{F}CH₂, R_{F}CH₂CH₂, R_{F}CH₂0 et R_{F}COO, où R_{F} représente un groupe perfluoroalkyle en C₂ à C₁₂, où également un ou plusieurs atomes de fluor peuvent être remplacés par de l'hydrogène.

7. Phase selon la revendication 2, où au moins un des radicaux R¹ et R² représente R_{F}, où R_{F} est un groupe perfluoroalkyle à chaîne droite en C₂ à C₁₂, où également un ou plusieurs atomes de fluor peuvent être remplacés par de l'hydrogène.

8. Phase selon la revendication 2, caractérisée en ce qu'elle contient au moins un composé optiquement actif.

9. Phase selon la revendication 2, caractérisée en ce que les composés de formule I sont choisis parmi les composés de formules partielles la, Ib, Ic et Il : 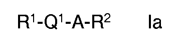 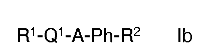 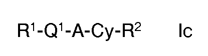 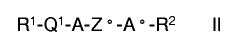 où R¹, Q¹, A, A°, Z°, Cy, Ph et R² ont la signification donnée dans la revendication 1.

10. Phase selon la revendication 9, caractérisée en ce que les composés de formule partielle la sont choisis parmi les composés de formules partielles lah, lal, lam et lan, 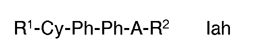 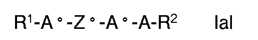 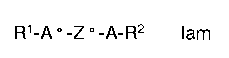 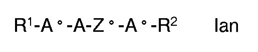 où Cy, Ph et R² ont la signification donnée dans la revendication 1 et R¹ représente un alkyle, alcoxy, alcanoyloxy, alcoxycarbonyle ou alcoxycarbonyloxy,
A représente un trans-1,4-cyclohexylène ou un 1,4-phénylène éventuellement substitué par un atome de chlore, et
Z° représente -CO-O-, -O-CO- ou -CH₂CH₂, ou encore, si A est un trans-1,4-cyclohexylène, -CH₂-CHCN- ou -CHCN-CH₂-.

11. Phase selon la revendication 10, caractérisée en ce que les composés de formule partielle lal sont choisis parmi les formules partielle lala à lalc, 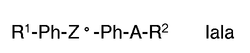 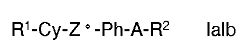 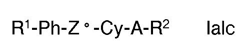 où R¹, R², Ph, A, Z ° et R² ont la signification indiquée pour la formule partielle IaL.

12. Elément d'affichage électro-optique, caractérisé en ce qu'il contient comme diélectrique une phase selon la revendication 2 ou la revendication 3.
